(19) **Europäisches Patentamt / European Patent Office / Office européen des brevets**

(11) **EP 2 970 434 B1**

(12) **EUROPEAN PATENT SPECIFICATION**

(45) Date of publication and mention
of the grant of the patent:
**05.12.2018 Bulletin 2018/49**

(21) Application number: **14763190.7**

(22) Date of filing: **14.03.2014**

(51) Int Cl.:
*C07K 14/81* [(2006.01)]    *C07K 19/00* [(2006.01)]
*C12N 15/63* [(2006.01)]

(86) International application number:
**PCT/US2014/029159**

(87) International publication number:
**WO 2014/144658 (18.09.2014 Gazette 2014/38)**

(54) **DUAL REACTIVITY POTENT KUNITZ INHIBITOR OF FIBRINOLYSIS**

POTENTER KUNITZ-INHIBITOR DER FIBRINOLYSE MIT DUALER REAKTIVITÄT

INHIBITEUR DE FIBRINOLYSE DU TYPE KUNITZ, PUISSANT ET À DOUBLE RÉACTIVITÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.03.2013 US 201361793131 P
30.12.2013 US 201361922026 P**

(43) Date of publication of application:
**20.01.2016 Bulletin 2016/03**

(73) Proprietor: **The Regents of the University of
California
Oakland, CA 94607 (US)**

(72) Inventor: **BAJAJ, Paul, S.
Los Angeles, California 90024 (US)**

(74) Representative: **Salisbury, Frances et al
Mewburn Ellis LLP
City Tower
40 Basinghall Street
London EC2V 5DE (GB)**

(56) References cited:
WO-A2-2007/076537      WO-A2-2007/076537
US-A- 5 728 674        US-A1- 2008 026 998
US-A1- 2009 036 365

• M. S. BAJAJ ET AL: "Engineering Kunitz Domain 1 (KD1) of Human Tissue Factor Pathway Inhibitor-2 to Selectively Inhibit Fibrinolysis: PROPERTIES OF KD1-L17R VARIANT", JOURNAL OF BIOLOGICAL CHEMISTRY, vol. 286, no. 6, 11 February 2011 (2011-02-11), pages 4329-4340, XP055283872, US ISSN: 0021-9258, DOI: 10.1074/jbc.M110.191163
• BAJAJ ET AL.: 'Engineering kunitz domain 1 (KD1) of human tissue factor pathway inhibitor-2 to selectively inhibit fibrinolysis properties of KD1-L17R variant' JOURNAL OF BIOLOGICAL CHEMISTRY vol. 286, no. 6, 2011, pages 4329 - 4340, XP055283872
• DATABASE NCBI 13 December 2010 Database accession no. AAB23819.1

EP 2 970 434 B1

**Description**

BACKGROUND

Technical Field

Embodiments of the invention disclosed herein relate generally to compositions and methods for inhibiting proteolysis, and in particular, for inhibiting fibrinolysis that can lead to detrimental excessive bleeding and for inhibiting proteolytic degradation that can contribute to tumor growth and progression, including cancer metastasis. More specifically, the present embodiments relate to novel Kunitz-type inhibitor polypeptides that are potent plasmin inhibitors and that exhibit advantageously low anti-coagulation activity and low immunogenicity.

Description of the Related Art

The proteolytic enzyme plasmin (E.C. 3.4.4.14) is a 90 kDa broad spectrum serine protease that is found in human plasma following activation from its zymogen, plasminogen, by tissue plasminogen activator (tPA) or urokinase (uPA). Plasmin plays a central role in the fibrinolytic process and in regulating the turnover of extracellular matrix (ECM) components. While tPA is believed to be primarily involved in plasmin activation leading to thrombolysis in the vasculature, uPA is thought to be mainly responsible for activation of plasmin that results in ECM degradation. Cell surface uPA that is bound to membrane uPA receptors (uPAR) enhances plasminogen activation and consequent plasmin-dependent proteolysis. While bound to the cell surface, plasmin is protected from inhibition by circulating alpha$_2$-antiplasmin, thereby confining plasmin-mediated proteolysis locally to sites at or near the cell surface.

Plasmin proteolytic activity is a major contributor to pathologic processes such as inflammation, tumor cell growth and tumor metastasis. For example, invasive properties of tumor cells may depend at least in part on direct plasmin-mediated proteolysis of ECM components, and indirectly on plasmin-mediated activation of other proteases involved in ECM degradative processes, such as pro-matrix metalloproteinases (proMMPs) and collagenases. Tumorigenic effects may be accelerated by activation of MMP-3 and MMP-9 by plasmin via the uPA/uPAR complex at the cell surface. Directly and indirectly plasmin-induced proteolytic events thus increase blood vessel permeability to facilitate vascular entry by, and systemic dissemination of, tumor cells.

Plasmin and MMPs may also participate in angiogenesis by promoting the release from ECM of specific matrix-derived growth factors such as basic fibroblast growth factor (bFGF) and vascular endothelial growth factor (VEGF), and by activating latent forms of transforming growth factor-beta (TGF-β), insulin-like growth factor-1 (IGF-1), and insulin-like growth factor binding protein (IGFBP). Moreover, endothelial cells at the leading edge of a newly formed blood vessel express components of both the plasmin and MMP pathways under the regulation of common growth factors and cytokines.

Inhibition of plasmin has been widely desired in reducing perioperative bleeding and in minimizing the need for transfusions during surgery, particularly during organ transplantation, orthopaedic and cardiac surgery. Excessive bleeding can require blood transfusion, which is associated with increased risk of one or more deleterious sequelae such as transmission of an infection, allergic reaction, and mismatched transfusion (e.g., inadvertent or undetected mismatch between transfusion donor and recipient at one or more of blood group antigens, major or minor histocompatibility antigens, or other markers).

Previously, the plasmin inhibitor aprotinin (Trasylol®) found widespread use for reducing perioperative bleeding, but was withdrawn from use in 2007 after a large multi-center study (Blood Conservation using Antifibrinolytics in a Randomised Trial, BART) found higher mortality rates associated with this drug than with alternative treatments during high-risk cardiac surgery (Furgusson et al., 2008 N Engl J Med. 358:2319). Aprotinin is a Kunitz-type inhibitor (Laskowski and Kato, 1980 Annu. Rev. Biochem. 49:593) that was originally isolated from bovine lung tissue and that inhibits virtually all serine proteases. Although aprotinin was the most effective antifibrinolytic agent known prior to its withdrawal, its use was discontinued after aprotinin was linked to increased incidence of myocardial infarction, vein graft hypercoagulation, renal failure, and mortality. Additionally, second and subsequent uses of aprotinin, a bovine protein, in a given human patient were linked to an increased likelihood of anaphylactic shock due to immunogenicity of the xenogeneic protein.

Other fibrinolysis inhibitors that are clinically available are commonly referred to as lysine analogues and include epsilon-aminocaproic acid (ε-ACA or EACA, Amicor) and tranexamic acid (TXA). These inhibitors bind to the lysine binding sites that are present in the first and fourth kringle domains of plasminogen, thereby preventing plasminogen binding to fibrin, which precludes efficient plasminogen activation by tPA or uPA. Clinical studies have found the lysine analogues to be highly variable in their ability to reduce perioperative bleeding and they have not been extensively characterized with respect to potential risks associated with their use. Higher doses of lysine analogues are required to reduce bleeding than had been used for aprotinin, and recent reports have suggested that lysine analogues may cause seizures. As candidate therapeutic plasminogen inhibitors, the lysine analogues are thus regarded as unreliable.

Despite such advances in the recognition at the molecular level of proteolytic mechanisms that contribute to tumorigenesis and metastasis, there remains a need for safe and effective therapies to block cancer progression and/or prevent metastasis. Similarly, the need remains unmet for safe, effective and reliable antifibrinolytic agents to decrease harmful perioperative bleeding and/or otherwise inhibit plasmin activity, despite general understanding in the art of the processes of fibrinogen activation and plasmin-mediated proteolysis. Certain of the presently disclosed embodiments address these needs and offer other related advantages.

US2008/0026998 describes a human Kunitz-type inhibitory polypeptide with enhanced antifibrinolytic activity, methods of making, and methods of use. The polypeptide is structurally similar to the KD1 domain of human tissue factor pathway inbitior-2 (TFPI-2).

BRIEF SUMMARY

According to certain embodiments of the invention disclosed herein, there is provided a plasmin-inhibiting polypeptide, comprising a polypeptide of general formula (I): N-Y-J-Z-C (I) wherein: (a) N is an amino terminus of the plasmin-inhibiting polypeptide and consists of 0, 1, 2, 3, 4, 5, 6, 7, 8, 9, 10, 11, 12, 13, 14, 15, 16, 17, 18, 19, 20, 21, 22, 23, 24, 25-30, 31-40, 41-50, or 51-60 amino acids that are independently selected from natural and non-natural amino acids, (b) Y is either nothing or methionine or N-formylmethionine, (c) J is a Kunitz-type proteinase first inhibitor domain (KD1) polypeptide of 57 amino acids having the amino acid sequence set forth in SEQ ID NO:1, (d) Z is a KD1 carboxy-region polypeptide that is selected from: (i) a KD1 carboxy-region polypeptide of general formula (II): I-X3-K-V-X4-K (II)

[SEQ ID NO:2] in which X3 and X4 are amino acids independently selected from natural and non-natural amino acids and are not C, F, R or W, (ii) a KD1 carboxy-region polypeptide of general formula (III): I-X3-K (III) in which X3 is an amino acid selected from natural and non-natural amino acids and is not C, F, R or W, (iii) a KD1 carboxy-region polypeptide of general formula (IV): I-X3-K-V-X5 (IV) [SEQ ID NO:20] in which X3 and X5 are independent, X3 is an amino acid selected from natural and non-natural amino acids and is not C, F, R or W, and X5 is an amino acid selected from natural and non-natural amino acids and is not C, F, R or W, (iv) a KD1 carboxy-region polypeptide of general formula (V): I-X3-K-V (V) [SEQ ID NO:21] in which X3 is an amino acid selected from natural and non-natural amino acids and is not C, F, R or W, (v) a KD1 carboxy-region polypeptide of general formula (VI): I-X3-K-X6 (VI) [SEQ ID NO:30] in which X3 and X6 are amino acids independently selected from natural and non-natural amino acids, X3 is not C, F, R or W, and X6 is any natural or non-natural amino acid, and (vi) a KD1 carboxy-region polypeptide of general formula (VII): I-X3-K-X6-X7 (VII) [SEQ ID NO:31] in which X3, X6 and X7 are amino acids independently selected from natural and non-natural amino acids, X3 is not C, F, R or W, X6 is any natural or non-natural amino acid, and X7 is any natural or non-natural amino acid, (e) C is a carboxy terminus of the plasmin-inhibiting polypeptide and consists of 0, 1, 2, 3, 4, 5, 6, 7, 8, 9, 10, 11, 12, 13, 14, 15, 16 17, 18, 19, 20, 21, 22, 23, 24, 25-30, 31-40, 41-50, or 51-60 amino acids that are independently selected from natural and non-natural amino acids, and (f) the plasmin-inhibiting polypeptide inhibits plasmin activity.

In certain further embodiments the plasmin-inhibiting polypeptide is selected from: (a) the plasmin-inhibiting polypeptide in which N, Y and C are each nothing, J is the KD1 polypeptide having the amino acid sequence set forth in SEQ ID NO:1, and Z is the KD1 carboxy-region polypeptide of general formula (II): I-X3-K-V-X4-K (II) (SEQ ID NO: 2) in which X3 and X4 are amino acids independently selected from natural and non-natural amino acids and are not C, F, R or W, (b) the plasmin-inhibiting polypeptide in which N, Y and C are each nothing, J is the KD1 polypeptide having the amino acid sequence set forth in SEQ ID NO:1, and Z is the KD1 carboxy-region polypeptide of general formula (III): I-X3-K (III) in which X3 is an amino acid selected from natural and non-natural amino acids and is not C, F, R or W, (c) the plasmin-inhibiting polypeptide in which N, Y and C are each nothing, J is the KD1 polypeptide having the amino acid sequence set forth in SEQ ID NO:1, and Z is the KD1 carboxy-region polypeptide of general formula (IV): I-X3-K-V-X5 (IV)

[SEQ ID NO:20] in which X3 and X5 are independent, X3 is an amino acid selected from natural and non-natural amino acids and is not C, F, R or W, and X5 is an amino acid selected from natural and non-natural amino acids and is not C, F, R or W, (d) the plasmin-inhibiting polypeptide in which N, Y and C are each nothing, J is the KD1 polypeptide having the amino acid sequence set forth in SEQ ID NO:1, and Z is the KD1 carboxy-region polypeptide of general formula (V): I-X3-K-V (V) [SEQ ID NO:21] in which X3 is an amino acid selected from natural and non-natural amino acids and is not C, F, R or W, (e) the plasmin-inhibiting polypeptide in which N and C are each nothing, Y is methionine or N-formylmethionine, J is the KD1 polypeptide having the amino acid sequence set forth in SEQ ID NO:1, and Z is the KD1 carboxy-region polypeptide of general formula (II): I-X3-K-V-X4-K (II) (SEQ ID NO: 2) in which X3 and X4 are amino acids independently selected from natural and non-natural amino acids and are not C, F, R or W, (f) the plasmin-inhibiting polypeptide in which N and C are each nothing, Y is methionine or N-formylmethionine, J is the KD1 polypeptide having the amino acid sequence set forth in SEQ ID NO:1, and Z is the KD1 carboxy-region polypeptide of general formula (III): I-X3-K (III) in which X3 is an amino acid selected from natural and non-natural amino acids and is not C, F, R or W, (g) the plasmin-inhibiting polypeptide in which N and C are each nothing, Y is methionine or N-formylmethionine, J is the KD1 polypeptide having the amino acid sequence set forth in SEQ ID NO:1, and Z is the KD1 carboxy-region polypeptide of general formula (IV): I-X3-K-V-X5 (IV) [SEQ ID NO:20] in which X3 and X5 are independent, X3 is an amino acid selected from natural and non-natural amino acids and is not C, F, R or W, and X5 is an amino acid selected from natural and non-natural amino acids and is not C, F, R or W, and (h) the plasmin-inhibiting polypeptide in which N and C are each nothing, Y is methionine or N-formylmethionine, J is the KD1 polypeptide having the amino acid sequence set forth in SEQ ID NO:1, and Z is the KD1 carboxy-region polypeptide of general formula (V): I-X3-K-V (V) [SEQ ID NO:21] in which X3 is an amino acid selected from natural and non-natural amino acids and is not C, F, R or W.

In certain other embodiments the plasmin-inhibiting polypeptide is selected from: (a) the plasmin-inhibiting polypeptide in which N, Y and C are each nothing, J is the KD1 polypeptide having the amino acid sequence set forth in SEQ ID NO:1, and Z is the KD1 carboxy-region polypeptide of formula (II) having the sequence IEKVPK [SEQ ID NO:2 in which X3 is E and X4 is P, SEQ ID NO: 39], (b) the plasmin-inhibiting polypeptide in which N, Y and C are each nothing, J is the KD1 polypeptide having the amino acid sequence set forth in SEQ ID NO:1, and Z is the KD1 carboxy-region polypeptide of formula (III) having the sequence IEK, (c) the plasmin-inhibiting polypeptide in which N, Y and C are each nothing, J is the KD1 polypeptide having the amino acid sequence set forth in SEQ ID NO:1, and Z is the KD1 carboxy-region polypeptide of formula (IV) having the sequence IEKVP [SEQ ID NO: 40; SEQ ID NO:20 in which X3 is E and X5 is P], (d) the plasmin-inhibiting polypeptide in which N, Y and C are each nothing, J is the KD1 polypeptide having the amino acid sequence set forth in SEQ ID NO:1, and Z is the KD1 carboxy-region polypeptide of formula (V) having the sequence IEKV [SEQ ID NO: 41; SEQ ID NO:21 in which X3 is E], (e) the plasmin-inhibiting polypeptide in which N and C are each nothing, Y is methionine or N-formylmethionine, J is the KD1 polypeptide having the amino acid sequence set forth in SEQ ID NO:1, and Z is the KD1 carboxy-region polypeptide of formula (II) having the sequence IEKVPK [SEQ ID NO:2 in which X3 is E and X4 is P, SEQ ID NO: 39], (f) the plasmin-inhibiting polypeptide in which N and C are each nothing, Y is methionine or N-formylmethionine, J is the KD1 polypeptide having the amino acid sequence set forth in SEQ ID NO:1, and Z is the KD1 carboxy-region polypeptide of formula (III) having the sequence IEK, (g) the plasmin-inhibiting polypeptide in which N and C are each nothing, Y is methionine or N-formylmethionine, J is the KD1 polypeptide having the amino acid sequence set forth in SEQ ID NO:1, and Z is the KD1 carboxy-region polypeptide of formula (IV) having the sequence IEKVP [SEQ ID NO: 40; SEQ ID NO:20 in which X3 is E and X5 is P], and (h) the plasmin-inhibiting polypeptide in which N and C are each nothing, Y is methionine or N-formylmethionine, J is the KD1 polypeptide having the amino acid sequence set forth in SEQ ID NO:1, and Z is the KD1 carboxy-region polypeptide of formula (V) having the sequence IEKV [SEQ ID NO: 41; SEQ ID NO:21 in which X3 is E].

According to certain embodiments there is provided a plasmin-inhibiting polypeptide of no more than 200, 190, 180, 170, 160, 150, 140, 130, 120, 110, 100, 90, 89, 88, 87, 86, 85, 84, 83, 82, 81, 80, 79, 78, 77, 76, 75, 74, 73, 72, 71, 70, 69, 68, 67, 66, 65, 64 or 63 amino acids, comprising the amino acid sequence set forth in any one of SEQ ID NOS:3-18, 22-29 and 32-35, wherein the plasmin-inhibiting polypeptide inhibits plasmin activity.

According to certain embodiments there is provided a plasmin-inhibiting polypeptide of 60 or 61 amino acids that has an amino acid sequence that is at least 96, 97, 98 or 99% identical to the amino acid sequence of a plasmin-inhibiting polypeptide of general formula (I): N-Y-J-Z-C (I) in which: (a) N is an amino terminus of the plasmin-inhibiting polypeptide, (b) Y is either nothing or methionine or N-formylmethionine, (c) J is a Kunitz-type proteinase first inhibitor domain (KD1) polypeptide of 57 amino acids having the amino acid sequence set forth in SEQ ID NO:1, in which amino acid X1 at position 11 in SEQ ID NO:1 is D, N or S, amino acid X2 at position 17 in SEQ ID NO:1 is R, and a cysteine residue is present at each of amino acid positions 14 and 38 in SEQ ID NO:1, (d) Z is a KD1 carboxy-region polypeptide of general formula (III): I-X3-K (III) in which X3 is an amino acid selected from natural and non-natural amino acids and is not C, F, R or W, (e) C is a carboxy terminus of the plasmin-inhibiting polypeptide, and (f) the plasmin-inhibiting polypeptide inhibits plasmin activity.

In certain embodiments there is provided a plasmin-inhibiting polypeptide of 63 or 64 amino acids that has an amino acid sequence that is at least 94, 95, 96, 97, 98 or 99% identical to the amino acid sequence of a plasmin-inhibiting polypeptide of general formula (I): N-Y-J-Z-C (I) in which: (a) N is an amino terminus of the plasmin-inhibiting polypeptide, (b) Y is either nothing or methionine or N-formylmethionine, (c) J is a Kunitz-type proteinase first inhibitor domain (KD1) polypeptide of 57 amino acids having the amino acid sequence set forth in SEQ ID NO:1, in which amino acid X1 at position 11 in SEQ ID NO:1 is D, N or S, amino acid X2 at position 17 in SEQ ID NO:1 is R, and a cysteine residue is present at each of amino acid positions 14 and 38 in SEQ ID NO:1, (d) Z is a KD1 carboxy-region polypeptide of general formula (II): I-X3-K-V-X4-K (II) [SEQ ID NO:2] in which X3 and X4 are amino acids independently selected from natural and non-natural amino acids and are not C, F, R or W, (e) C is a carboxy terminus of the plasmin-inhibiting polypeptide, and (f) the plasmin-inhibiting polypeptide inhibits plasmin activity.

In certain embodiments there is provided a plasmin-inhibiting polypeptide of 61, 62, 63 or 64 amino acids that has an amino acid sequence that is at least 94, 95, 96, 97, 98 or 99% identical to the amino acid sequence of a plasmin-inhibiting polypeptide of general formula (I): N-Y-J-Z-C (I) in which: (a) N is an amino terminus of the plasmin-inhibiting polypeptide, (b) Y is either nothing or methionine or N-formylmethionine, (c) J is a Kunitz-type proteinase first inhibitor domain (KD1) polypeptide of 57 amino acids having the amino acid sequence set forth in SEQ ID NO:1, in which amino acid X1 at position 11 in SEQ ID NO:1 is D, N or S, amino acid X2 at position 17 in SEQ ID NO:1 is R, and a cysteine residue is present at each of amino acid positions 14 and 38 in SEQ ID NO:1, (d) Z is a KD1 carboxy-region polypeptide that is selected from: (i) a KD1 carboxy-region polypeptide of general formula (II): I-X3-K-V-X4-K

(II) [SEQ ID NO:2] in which X3 and X4 are amino acids independently selected from natural and non-natural amino acids and are not C, F, R or W, (ii) a KD1 carboxy-region polypeptide of general formula (III): I-X3-K (III) in which X3 is an amino acid selected from natural and non-natural amino acids and is not C, F, R or W, (iii) a KD1 carboxy-region polypeptide of general formula (IV): I-X3-K-V-X5 (IV) [SEQ ID NO:20] in which X3 and X5 are independent, X3 is an amino acid selected from natural and non-natural amino acids and is not C, F, R or W, and X5 is an amino acid selected from natural and non-natural amino acids and is not C, F, R or W, (iv) a KD1 carboxy-region polypeptide of general formula (V): I-X3-K-V (V) [SEQ ID NO:21] in which X3 is an amino acid selected from natural and non-natural amino acids and is not C, F, R or W, (v) a KD1 carboxy-region polypeptide of general formula (VI): I-X3-K-X6 (VI) [SEQ ID NO:30] in which X3 and X6 are amino acids independently selected from natural and non-natural amino acids, X3 is not C, F, R or W, and X6 is any natural or non-natural amino acid, and (vi) a KD1 carboxy-region polypeptide of general formula (VII): I-X3-K-X6-X7 (VII) [SEQ ID NO:31] in which X3, X6 and X7 are amino acids independently selected from natural and non-natural amino acids, X3 is not C, F, R or W, X6 is any natural or non-natural amino acid, and X7 is any natural or non-natural amino acid, (e) C is a carboxy terminus of the plasmin-inhibiting polypeptide, and (f) the plasmin-inhibiting polypeptide inhibits plasmin activity.

According to certain further embodiments, the above described plasmin-inhibiting polypeptide has decreased anti-coagulation activity compared to a wild type human tissue factor pathway inhibitor-2 (TFPI-2) polypeptide first Kunitz-type proteinase inhibitor domain (KD1) having the amino acid sequence set forth in SEQ ID NO:19. In certain embodiments there is provided a fusion protein comprising any one of the above described plasmin-inhibiting polypeptides fused to a fusion polypeptide domain. In certain embodiments there is provided a pharmaceutical composition comprising such a fusion protein; and a physiologically acceptable carrier. In certain embodiments there is provided any of the above described plasmin-inhibiting polypeptides to which a polyakylene glycol is attached to form a PAGylated polypeptide. In certain further embodiments polyethylene glycol is the polyakylene glycol and the PAGylated polypeptide is a PEGylated polypeptide.

Turning to another embodiment there is provided an isolated polynucleotide comprising a nucleic acid sequence that encodes the above described plasmin-inhibiting polypeptide. In another embodiment there is provided an expression vector comprising such a polynucleotide. In another embodiment there is provided a host cell transformed or transfected with such an expression vector. In another embodiment there is provided a method of producing the above described plasmin-inhibiting polypeptide, said method comprising the steps of: a) culturing the above described host cell under conditions and for a time sufficient to permit expression of the plasmin-inhibiting polypeptide; and b) isolating the plasmin-inhibiting polypeptide from the cultured host cell. In certain embodiments the plasmin-inhibiting polypeptide that is expressed by the host cell comprises N-terminal methionine or N-formylmethionine and the host cell expresses a methionine aminopeptidase (MAP) under conditions and for a time sufficient for the MAP to remove the N-terminal methionine or N-formylmethionine from the plasmin-inhibiting polypeptide.

In another embodiment there is provided a pharmaceutical composition, comprising: a) any of the above described plasmin-inhibiting polypeptides; and b) a physiologically acceptable carrier. In another embodiment there is provided a method for inhibiting plasmin activity, comprising contacting (i) plasmin with (ii) the above described plasmin-inhibiting polypeptide, under conditions and for a time sufficient for the plasmin-inhibiting polypeptide to bind to the plasmin, and thereby inhibiting plasmin activity. In certain embodiments the step of contacting takes place *in vitro.*

In another embodiment there is provided a method for treating a subject in need of inhibition of a plasmin activity, comprising administering to the subject an effective amount of the above described plasmin-inhibiting polypeptide or the above described isolated polynucleotide. In certain embodiments the subject has or is suspected of being at risk for having cancer, hemophilia, rheumatoid arthritis or systemic inflammatory response syndrome (SIRS), or the subject is in need of or has undergone angiogenesis, bone remodeling or coronary artery bypass grafting (CABG). In certain other embodiments the subject is undergoing surgery or has recently undergone surgery, which in certain further embodiments is cardiovascular surgery, oncological surgery, genitourinary surgery, orthopedic surgery, thoracic surgery, plastic surgery, trauma surgery, abdominal surgery, transplant surgery, neurologic surgery or otolaryngological surgery.

In another embodiment there is provided a method for isolating a plasmin-inhibiting polypeptide that comprises a Kunitz-type proteinase first inhibitor domain (KD1), comprising: (a) contacting (i) a solid phase on which is immobilized plasmin that has been inactivated, with (ii) a sample that comprises a plasmin-inhibiting polypeptide which comprises a Kunitz-type proteinase first inhibitor domain (KD1) and impurities, under conditions and for a time sufficient for binding of the KD1 to the inactivated plasmin; (b) washing the solid phase under conditions that do not disrupt KD1-plasmin binding, to remove impurities; and (c) eluting the KD1 from the solid phase, and thereby isolating the polypeptide that comprises the KD1. In certain embodiments the sample contains one or more of the above described plasmin-inhibiting polypeptides.

In another embodiment there is provided a method for protecting an isolated protein or a protein in an isolated biological sample from proteolytic degradation, comprising: contacting the isolated protein or the isolated biological sample with at least one of the above described plasmin-inhibiting polypeptides.

These and other aspects of the invention will be evident upon reference to the following detailed description and attached drawings. All of the U.S. patents, U.S. patent application publications, U.S. patent applications, foreign patents, foreign patent applications and non-patent publications referred to in this specification and/or listed in the Application Data Sheet as if each was incorporated individually. Aspects of the invention can be modified, if necessary, to employ concepts of the various patents, applications and publications to provide yet further embodiments of the invention.

BRIEF DESCRIPTION OF THE SEVERAL VIEWS OF THE DRAWINGS

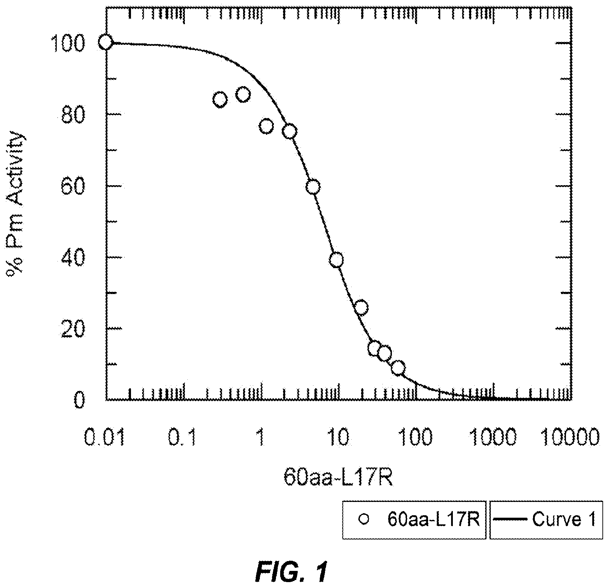

Figure 1 shows inhibition of plasmin activity by a plasmin-inhibiting polypeptide comprising the amino acid sequence set forth in SEQ ID NO:5. The calculated inhibition constant ($K_i$) was 2.36 nM.

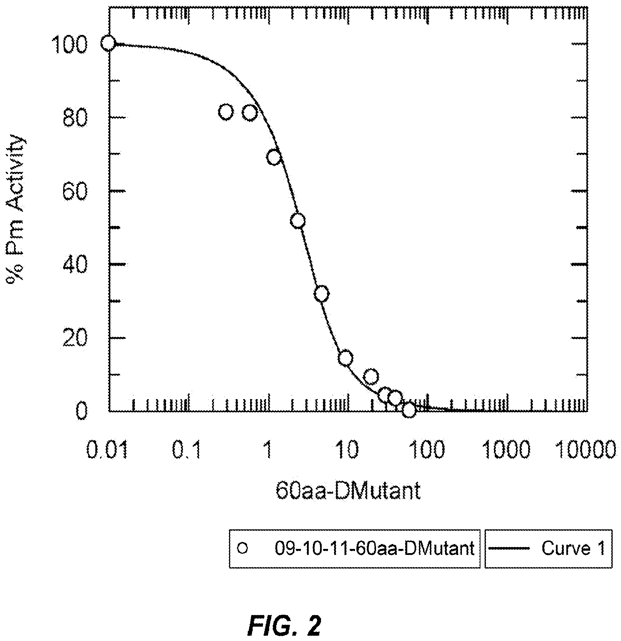

Figure 2 shows inhibition of plasmin activity by a plasmin-inhibiting polypeptide comprising the amino acid sequence set forth in SEQ ID NO:3. The calculated inhibition constant ($K_i$) was 0.49 nM.

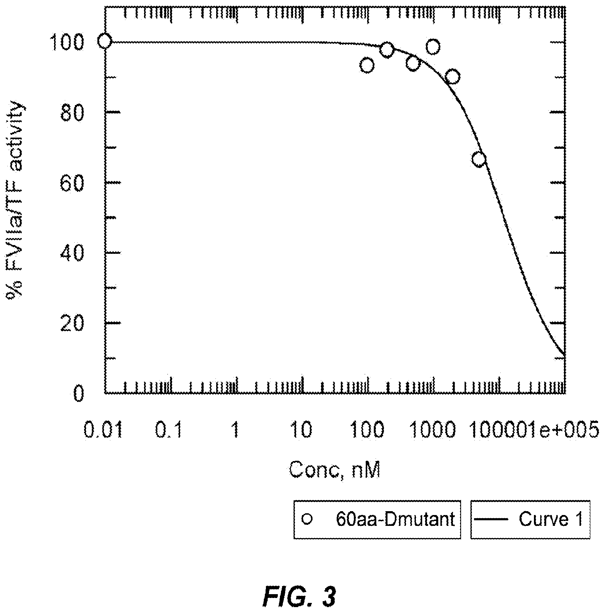

Figure 3 shows the effect on an assay for Factor Vila-Tissue Factor amidolytic activity of a plasmin-inhibiting polypeptide comprising the amino acid sequence set forth in SEQ ID NO:3. The calculated inhibition constant ($K_i$) was 5869.5 nM.

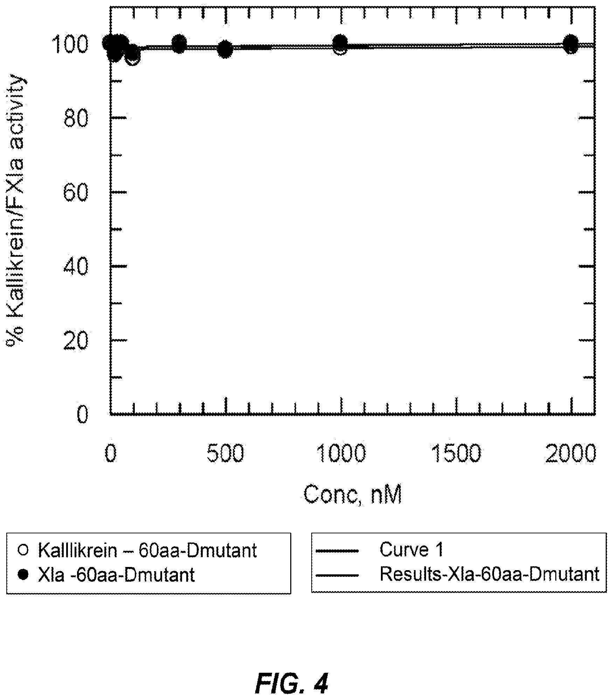

Figure 4 shows the effects of a plasmin-inhibiting polypepide comprising the amino acid sequence set forth in SEQ ID NO:3 on assays for inhibition of Factor XIa amidolytic activity (closed circles) and kallikrein amidolytic activity (open circles).

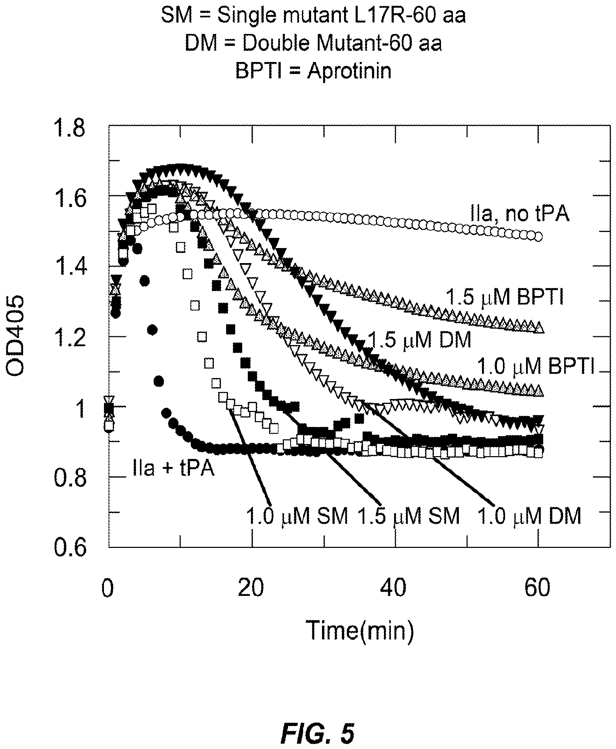

Figure 5 shows the effects of a plasmin-inhibiting polypeptide comprising the amino acid sequence set forth in SEQ ID NO:5 (SM), and of a plasmin-inhibiting polypeptide comprising the amino acid sequence set forth in SEQ ID NO:3 (DM), and of aprotinin (BPTI), on clotting time in a plasma clot fibrinolysis assay.

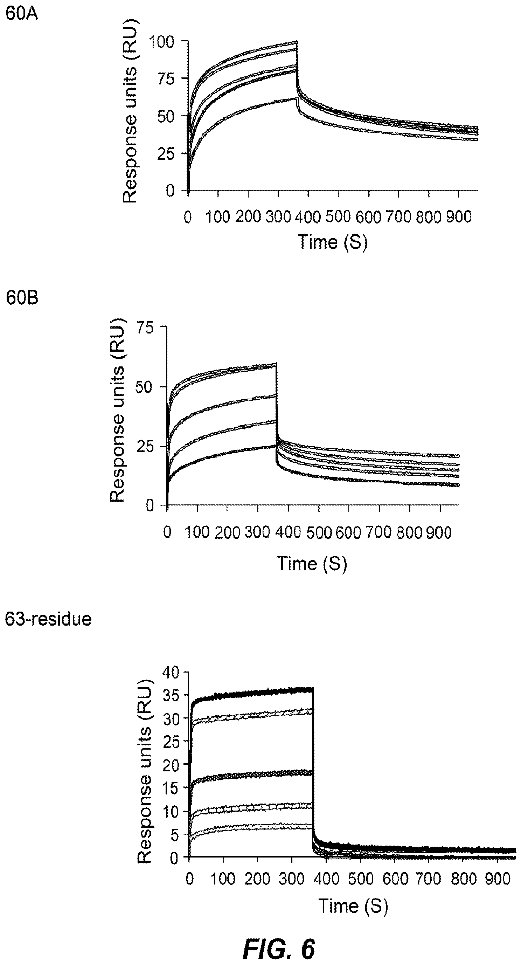

Figure 6 shows binding by plasmin-inhibiting polypeptides at varying concentrations to immobilized active site-blocked plasmin in a surface plasmon resonance assay. Top panel (60A), plasmin-inhibiting polypeptide comprising the amino acid sequence set forth in SEQ ID NO:5; middle panel (60B), plasmin-inhibiting polypeptide comprising the amino acid sequence set forth in SEQ ID NO:3; bottom panel (63-residue), plasmin-inhibiting polypeptide comprising the amino acid sequence set forth in SEQ ID NO:6. Aprotinin did not bind to active site-blocked plasmin in the surface plasmon resonance assay.

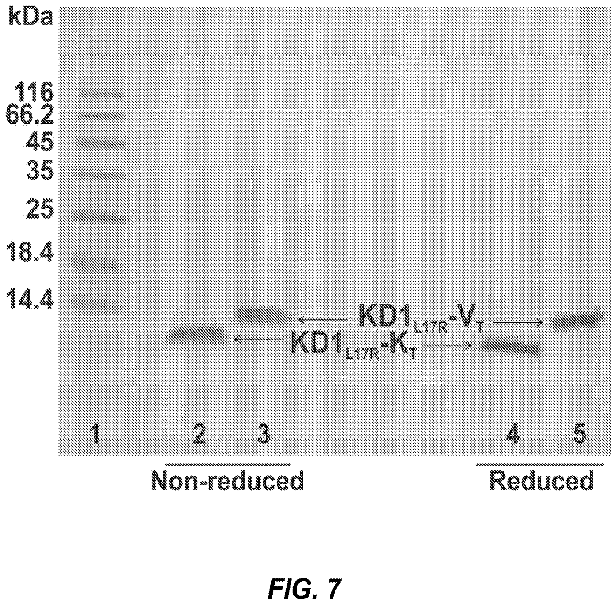

Figure 7 shows SDS-PAGE of $KD1_{L17R}$-$V_T$ and $KD1_{L17R}$-$K_T$. Lane 1, mixture of reduced EZ-Run™ protein Markers (Fisher BioReagents); lane 2, 5 $\mu$g of unreduced $KD1_{L17R}$-$K_T$; lane 3, 5 $\mu$g of unreduced $KD1_{L17R}$-$V_T$; lane 4, 5 $\mu$g of reduced $KD1_{L17R}$-$K_T$; and lane 5, 5 $\mu$g of reduced $KD1_{L17R}$-$V_T$. Concentration of acrylamide used was 15%.

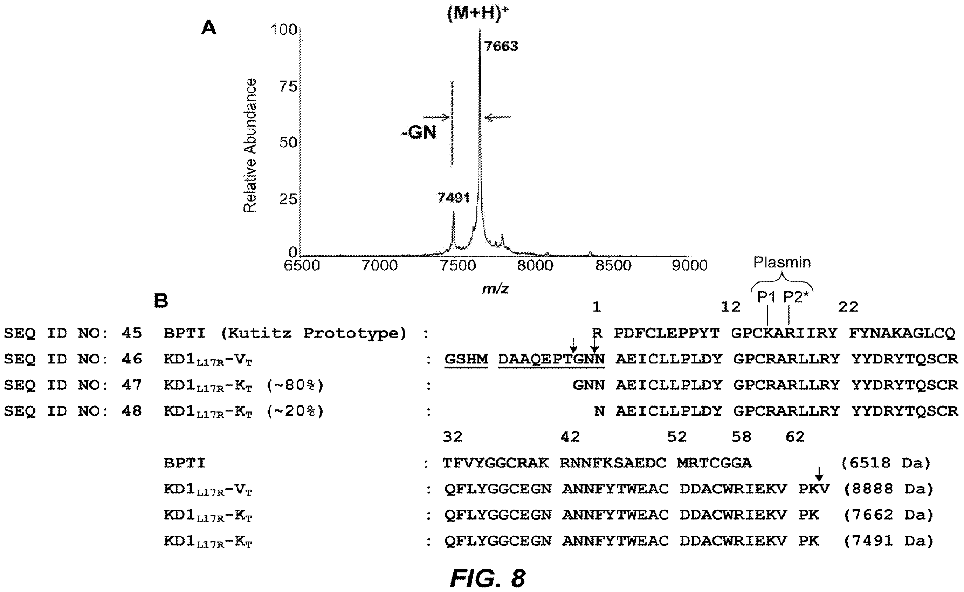

Figure 8. MALDI-TOF-ESI mass spectrometry data and amino acid sequence alignment of $KD1_{L17R}$-$K_T$ with the starting molecule ($KD1_{L17R}$-$V_T$) and BPTI. (A) Mass spectrometry data of $KD1_{L17R}$-$K_T$ obtained using Applied Biosystems Voyager™ DE-STR. The spectra were calibrated so that the masses should be within 0.1% of the theoretical values. (B) Amino acid sequences of $KD1_{L17R}$-$V_T$ and the two $KD1_{L17R}$-$K_T$ species-one (SEQ ID NO:47) with N-terminus sequence Gly-Asn-Asn-Ala-Glu-Ile (~80%, SEQ ID NO: 49), and the second (SEQ ID NO:48) with N-terminus sequence Asn-Ala-Glu-Ile (~20%, SEQ ID NO: 50). The minor sequence is internal to the major sequence starting at cycle 3 (asparagine) and the calculated difference in MW between the two species was 171 Da, is consistent with the mass spectrometry data shown in Fig. 8(A). It is inferred according to non-limiting theory that IIa or a contaminating protease cleaved the peptide bonds (shown by↓) between amino acids Thr-Gly and Asn-Asn on the N-terminal side, and between Lys-Val on the C-terminal side of $KD1_{L17R}$-$V_T$. The numbering system used is that of BPTI (Prototype Kunitz inhibitor). The N-terminal Gly-Ser-His-Met sequence in $KD1_{L17R}$-$V_T$ was derived from the IIa cleavage site introduced during cloning. The P1 and P2' subsites recognized by plasmin are marked.

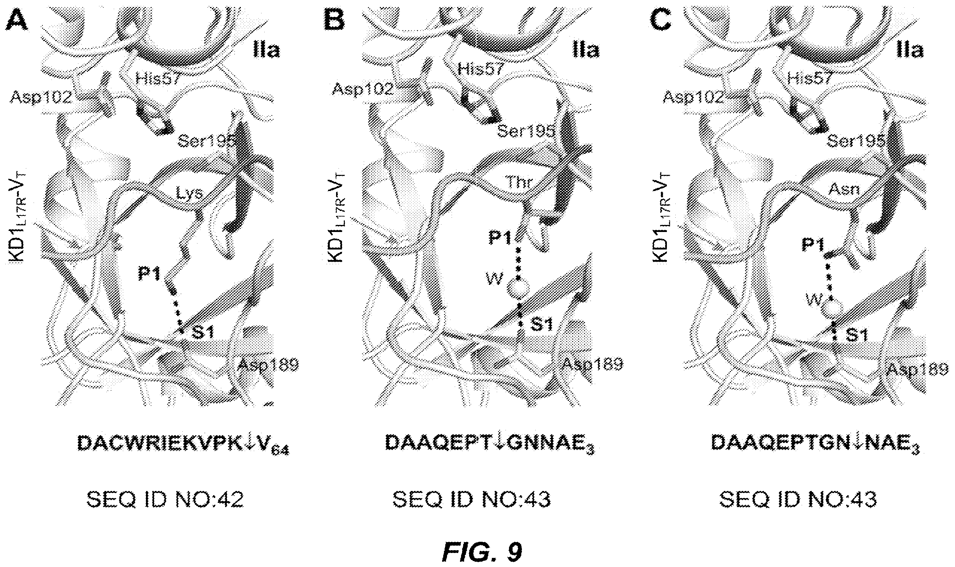

Figure 9. Schematic representations of the molecular interactions between the S1 pocket residue Asp189 of IIa and the P1 residue (recognized by IIa) of KD1L17R-VT at the three observed cleavage sites. The modeled complexes of IIa (lightly shaded ribbon diagram) and of KD1L17R-VT (dark narrow strand labeled with arrow) are shown in cartoon representations. Asp189 of IIa could make a direct hydrogen bond with P1 residue Lys63 (A) of KD1L17R-VT, whereas each of the analogous hydrogen bonds between Thr (B) and Asn (C) was mediated via a water molecule. The side chains of the active site triad residues (His57, Asp102, Ser195) and Asp189 of IIa, as well as the P1 residues (Lys, Thr and Asn) of the KD1L17R-VT are shown in stick representation. The water molecules ("W") are shown as spheres (Fig. 9B,C). Each of the cleavage sites is shown in Fig. 8B by an arrow in the indicated amino acid sequence.

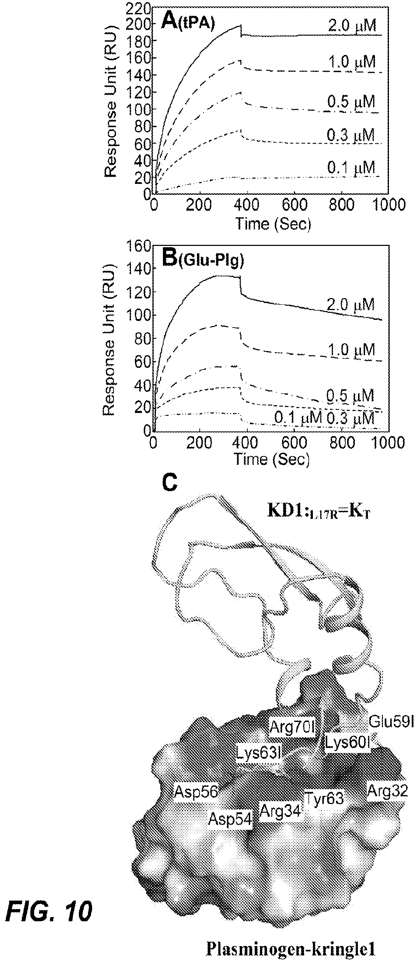

Figure 10. Interaction of KD1$_{L17R}$-K$_T$ with tPA and Glu-Plg as measured by SPR. (*A*) tPA binding to KD1$_{L17R}$-K$_T$. tPA was coupled to the CM5 chip by the amine coupling method and an immobilization level of 1193 response units (RU) was attained for the bound protein. Five concentrations (0.1 to 2 $\mu$M) of KD1$_{L17R}$-K$_T$ were used; six-minute association and 10-minute dissociation times (flow rate 10 $\mu$l/min) were employed. The RU value obtained with KD1$_{L17R}$-V$_T$ at 1 $\mu$M was 16 instead of 155 with KD1$_{L17R}$-K$_T$. (*B*) Glu-Plg binding to KD1$_{L17R}$-K$_T$. Glu-Plg was coupled to the CM5 chip and an immobilization level of 742 RU was attained for the bound protein. The concentrations of KD1$_{L17R}$-K$_T$ used and the analyte association and dissociation protocols were the same as in (*A*). The RU value obtained with KD1$_{L17R}$-V$_T$ at 1 $\mu$M was 12 instead of 86 with KD1$_{L17R}$-K$_T$. (*C*) Modeled complex of KD1$_{L17R}$-K$_T$ with plasminogen kringle domain1. The electrostatic surface of the plasminogen kringle domain1 (space-filled model) and a cartoon representation of the KD1$_{L17R}$-K$_T$ (ribbon model) are depicted. The residues that formed hydrogen bonds and salt-bridges (shown in dashed lines) between the kringle domain and the KD1$_{L17R}$-K$_T$ are shown in stick representation. KD1$_{L17R}$-K$_T$ residues are labeled with the suffix 'I'. In the electrostatic surface, dark areas represent charged regions and the white represents neutral areas.

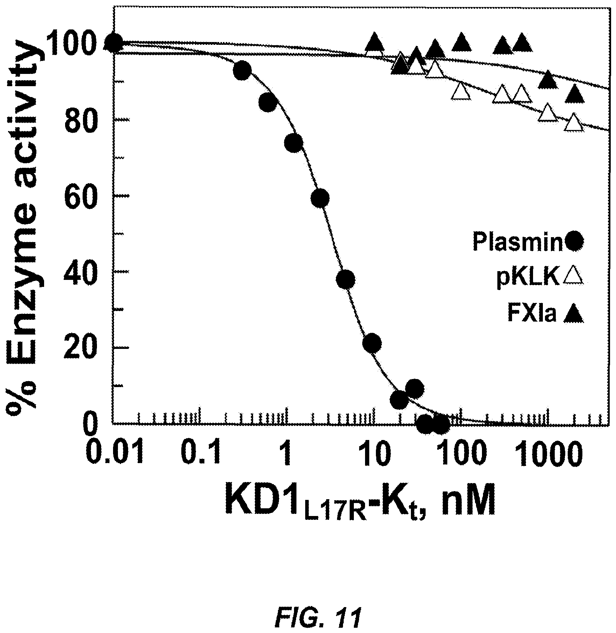

Figure 11. Determination of equilibrium inhibition constants ($K_i$) of KD1$_{L17R}$-K$_T$ with plasmin, FXIa and pKLK. The enzyme activity was expressed as the percent fractional activity (inhibited rate/uninhibited rate) at increasing inhibitor concentrations. The inhibition constants ($K_i$) were determined using equations 1 and 2 as outlined in Example 5 under "Experimental Procedures". Concentration of plasmin was 3 nM; concentrations of FXIa and pKLK were 1 nM each.

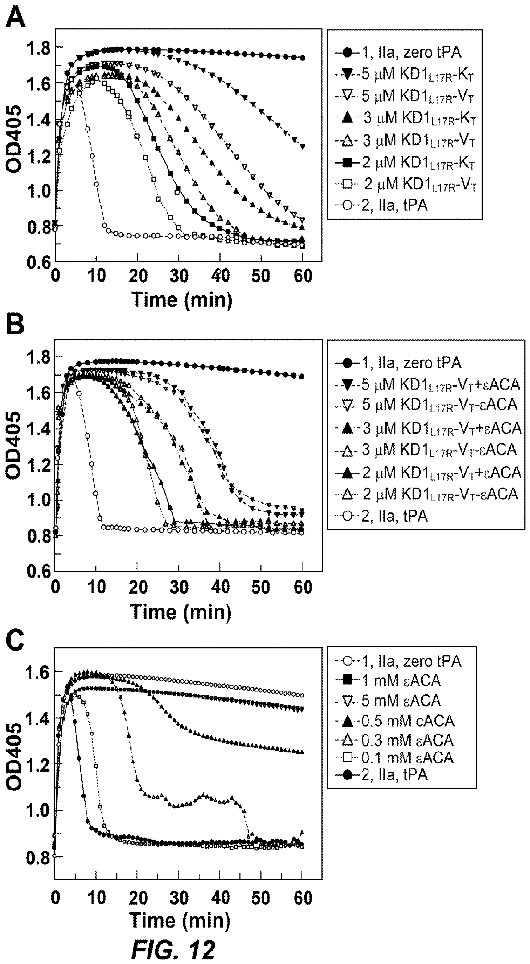

Figure 12. Effect of KD1$_{L17R}$-V$_T$, KD1$_{L17R}$-K$_T$ and $\varepsilon$ACA on fibrinolysis in human NPP. IIa was added to NPP to initiate clot formation, which was associated with an increase in optical density at 405 nm (curve 1 in A, B and C). Simultaneous addition of tPA converted plasminogen to plasmin, which dissolved the fibrin clot completely within -10 min as indicated by an initial increase followed by a decrease in OD$_{405}$ (curve 2 in A, B and C). (*A*) Inhibition of fibrinolysis by KD1$_{L17R}$-V$_T$ and KD1$_{L17R}$-K$_T$. KD1$_{L17R}$-V$_T$: 2 $\mu$M ("2 $\mu$M" open triangles), 3 $\mu$M ("3 $\mu$M" open triangles), and 5 $\mu$M ("5 $\mu$M" open triangles). KD1$_{L17R}$-K$_T$: 2 $\mu$M ("2 $\mu$M" closed triangles), 3 $\mu$M ("3 $\mu$M" closed triangles), and 5 $\mu$M ("5 $\mu$M" closed triangles). In each case, IIa was used to initiate clotting and tPA to initiate fibrinolysis. (*B*) The effect of $\varepsilon$ACA on inhibition of fibrinolysis by KD1$_{L17R}$-V$_T$. KD1$_{L17R}$-V$_T$: 2 $\mu$M ("2 $\mu$M" open triangles), 3 $\mu$M ("3 $\mu$M" open triangles), and 5 $\mu$M ("5 $\mu$M" open triangles). KD1$_{L17R}$-K$_T$ + equimolar $\varepsilon$ACA: 2 $\mu$M ("2 $\mu$M" closed triangles), 3 $\mu$M ("3 $\mu$M" closed triangles), and 5 $\mu$M ("5 $\mu$M" closed triangles). As in (*A*), in each case, IIa was used to initiate clotting and tPA to initiate fibrinolysis. (*C*) Inhibition of fibrinolysis by $\varepsilon$ACA alone. 0.1 mM ("0.1 mM" closed squares), 0.3 mM ("0.3 mM" open triangles), 0.5 mM ("0.5 mM" closed triangles), 1.0 mM ("1.0 mM" closed squares) and 5 mM ("5 mM" open circles). As in (*A*) and (*B*), in each case, IIa was used to initiate clotting and tPA to initiate fibrinolysis.

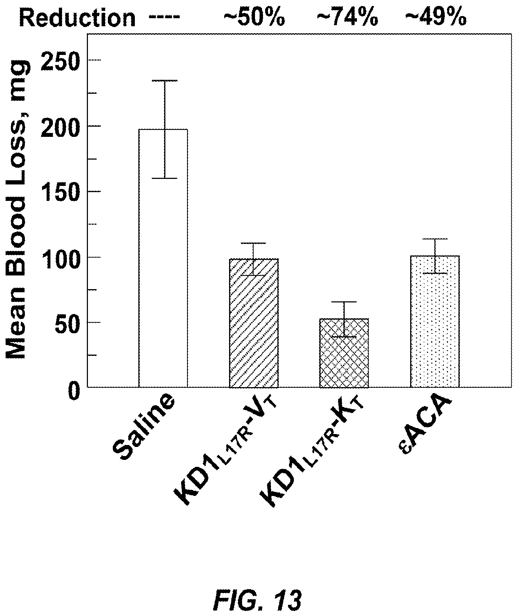

Figure 13. Effect of KD1$_{L17R}$-V$_T$, KD1$_{L17R}$-K$_T$ and $\varepsilon$ACA in a mouse liver laceration bleeding model. Animals were treated with saline, KD1$_{L17R}$-V$_T$, KD1$_{L17R}$-K$_T$, or $\varepsilon$ACA by intravenous injection. A 5-mm transverse incision was made on the left lobe of the liver. All blood oozing from the incision site was collected for a total duration of 30 min. Total blood loss was compared between different groups. Data are presented as mean $\pm$ S.E. of 10 animals each for saline, KD1$_{L17R}$-V$_T$, and KD1$_{L17R}$-K$_T$, and of 16 animals for $\varepsilon$ACA. Probability *p* values between different groups were as follows: saline versus KD1$_{L17R}$-V$_T$, 0.002; saline versus KD1$_{L17R}$-K$_T$, 0.001; saline versus $\varepsilon$ACA, 0.002; KD1$_{L17R}$-K$_T$ versus KD1$_{L17R}$-V$_T$, < 0.05; and KD1$_{L17R}$-K$_T$ versus $\varepsilon$ACA, < 0.05. *, *p* value < 0.01 compared with saline control.

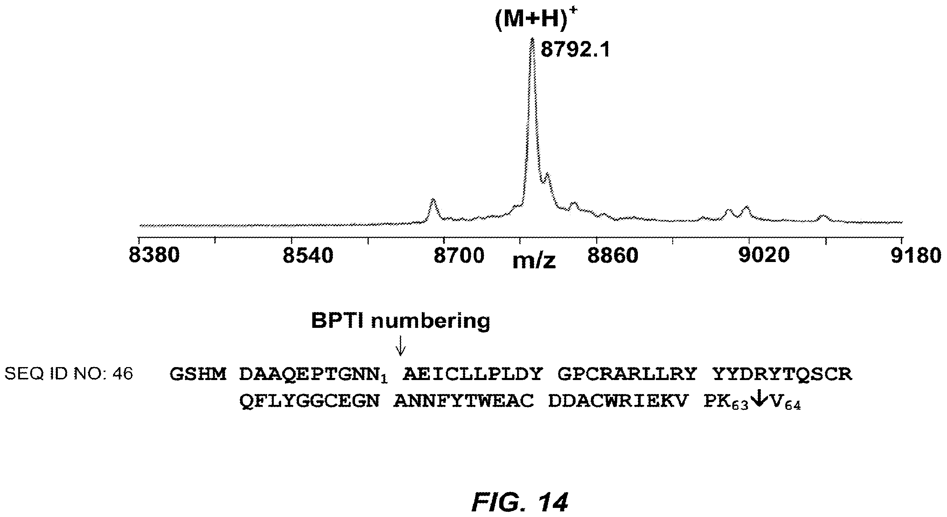

Figure 14 shows MALDI-TOF mass spectrometry data and the amino acid sequence of the KD1-L17R preparation previously characterized in the mouse liver laceration bleeding model (66, *infra*).

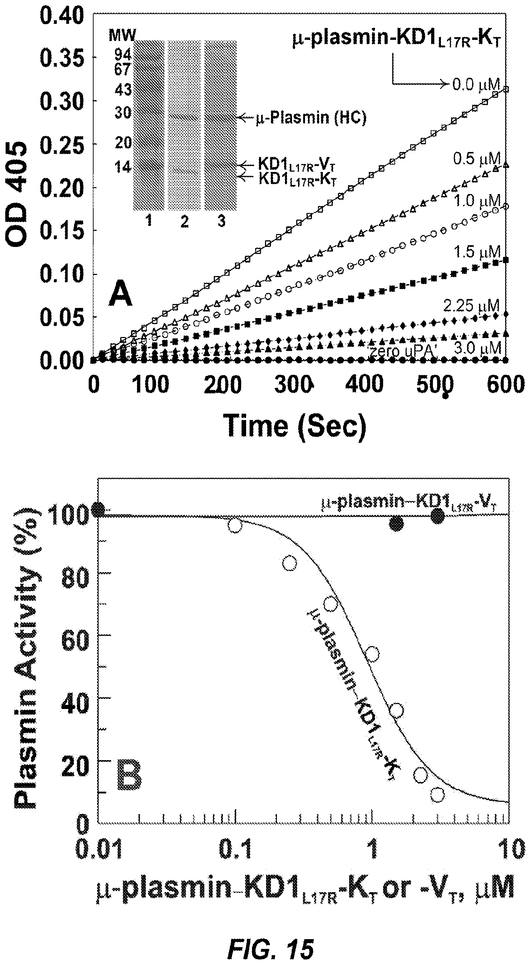

Figure 15. Inhibition of Glu-Plg binding to PMA-stimulated nonadherent U937 cells by $\mu$-plasmin-KD1$_{L17R}$-K$_T$. (A) Proteolytic activity of plasmin generated on the U937 cell surface. PMA-stimulated U937 cells (200 $\mu$l, 1 X 10$^6$ cells/ml) in HBS/BSA, pH 7.5 were incubated at 37 °C for 1 hour with 2 $\mu$M Glu-Plg containing various concentrations of $\mu$-plasmin-KD1$_{L17R}$-K$_T$ complex (ranging from 0 to 3 $\mu$M) or $\mu$-plasmin-KD1$_{L17R}$-V$_T$ complex (2 and 4 $\mu$M). The cells were washed and the cell surface-bound Glu-Plg was activated with 10 nM uPA for 20 min at 37 °C. The samples were diluted 10-fold and the plasmin formed was measured by S-2251 synthetic substrate hydrolysis (OD$_{405}$). $\mu$-Plasmin-KD1$_{L17R}$-K$_T$ reduced the plasmin formed in a dose dependent manner, whereas $\mu$-plasmin-KD1$_{L17R}$-V$_T$ had no effect (see panel B). *Inset,* Reduced SDS-PAGE (15% acrylamide) of the size exclusion gel purified $\mu$-plasmin-KD1$_{L17R}$-K$_T$ and $\mu$-plasmin-KD1$_{L17R}$-V$_T$ complexes. The expressed $\mu$-plasmin had residues 542-791 cleaved between R561 (R15 in chymotrypsin numbering) and V562 (V16 in chymotrypsin numbering). Heavy chain (HC) consists of residues 562-791 and the light chain consists of 20 residues of 542-561 and was not seen on the gel. Lane 1, MW markers; lane 2, $\mu$-plasmin-KD1$_{L17R}$-K$_T$ complex; lane 3, $\mu$-plasmin-KD1$_{L17R}$-V$_T$ complex. (*B*) Percent reduction of plasmin generation by purified $\mu$-plasmin-KD1$_{L17R}$-K$_T$ complex and of $\mu$-plasmin-KD1$_{L17R}$-V$_T$ complex. The plasmin activity was expressed as percent fractional activity (inhibited rate/uninhibited rate) at increasing inhibitor concentrations. ($\bigcirc$) $\mu$-plasmin-KD1$_{L17R}$-K$_T$; and ($\bullet$) $\mu$-plasmin-KD1$_{L17R}$-V$_T$.

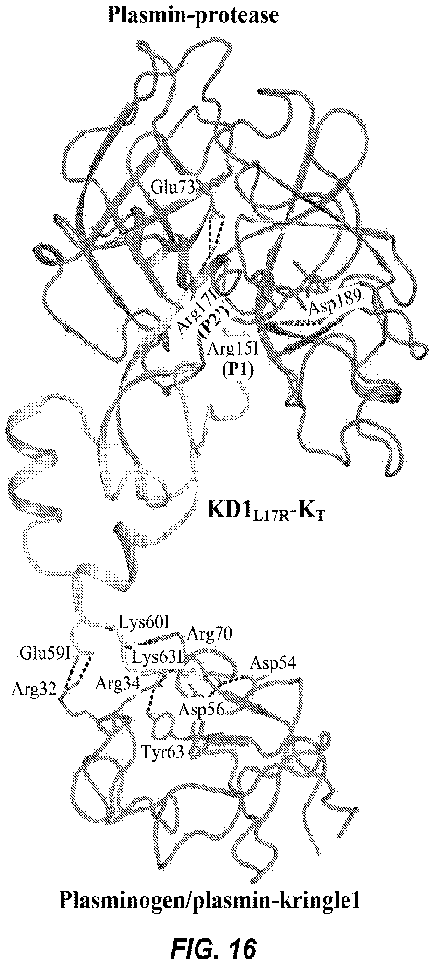

Figure 16. Modeled ternary complex of KD1$_{L17R}$-K$_T$ with the protease domain of plasmin and the kringle domain1 of plasminogen/plasmin. A cartoon representation of the KD1$_{L17R}$-K$_T$ (light ribbon structure, center) bound to the plasmin protease domain (dark ribbon structure, top) and plasminogen/plasmin kringle domain1 (dark ribbon structure, bottom) is shown. The KD1$_{L17R}$-K$_T$ C-terminal residues that make hydrogen bonds and salt-bridges with residues of the kringle domain1 are shown in stick representation. Similarly, residues Arg15 and Arg17 of the Kunitz domain that interact with residue Asp189 and Glu73 (chymotrypsin numbering) of the plasmin protease domain are shown in stick representation. The KD1$_{L17R}$-K$_T$ residues are labeled with the suffix 'I'.

DETAILED DESCRIPTION

Certain embodiments of the invention disclosed herein are based on the surprising discovery that a polypeptide engineered to contain minor structural modifications to the Kunitz-type inhibitor first polypeptide domain of human tissue factor pathway inhibitor-2 (TFPI-2, also known as matrix serine protease inhibitor or placental protein 5; Sprecher et al., 1994 Proc. Nat. Acad. Sci. USA 91:3353; Chand et al., 2004 J. Biol. Chem. 279:17500; SEQ ID NO:19) possesses unexpected potency as a plasmin inhibitor while having decreased (*e.g.,* reduced in a statistically significant manner) anti-coagulation activity when compared to the wild-type TFPI-2 polypeptide. In these and related embodiments there is thus provided a plasmin-inhibiting polypeptide that has superior plasmin-inhibiting ability relative to previously described plasmin inhibitors, and that also advantageously exhibits little or no immunogenicity in view of its high degree of structural similarity to native TFPI-2.

In particular, disclosed herein are engineered plasmin-inhibiting, Kunitz-type inhibitor domain 1-like containing polypeptides, including in certain embodiments plasmin-inhibiting polypeptides of the general formula:

N--(KD1)--C

wherein KD1 comprises a herein-disclosed Kunitz-type proteinase first inhibitor domain polypeptide having an amino acid sequence as set forth in SEQ ID NO:1 and also includes a KD1 carboxy-region polypeptide having an amino acid sequence as set forth in one of SEQ ID NOS:2, 20 or 21 (general formulae II, IV or V) or in general formula (III) as described herein. These and structurally related plasmin-inhibiting polypeptides are capable of inhibiting (*i.e.,* decreasing in a statistically significant manner, relative to an appropriate control) plasmin activity, and optionally in certain further embodiments may also exhibit decreased anti-coagulation activity compared to the wild-type TFPI-2 polypeptide.

The presently disclosed plasmin-inhibiting polypeptides will find uses in a wide variety of contexts where potent plasmin inhibition may be desired, such as for cancer therapy, treatment of autoimmune diseases and inflammation (*e.g.,* rheumatoid arthritis, systemic inflammatory response syndrome), modulating angiogenesis (*e.g.,* inhibiting, or reducing the likelihood or extent of angiogenesis in a statistically significant manner), as anti-fibrinolytic agents including such uses in surgery, transplantation, transfusion, and other situations where plasmin inhibition may be beneficial (see, *e.g.,* Bajaj et al., 2011 J. Biol. Chem. 286:4329; US 2009/0018069). As described herein, for example, plasmin may be advantageously targeted by using the presently disclosed plasmin-inhibiting polypeptides for cancer therapy given that (a) plasmin is predominantly present *in vivo* in its inactive zymogen (precursor) form, while its active form mostly occurs at local sites of tissue remodeling such as sites of tumor formation or metastasis, (b) plasmin has a major role, via both direct and indirect mechanisms, in ECM degradation that accompanies tumor growth and progression, and (c) active plasmin plays a role in the angiogenic process and so may be desirably inhibited to decrease the extent of solid tumor vascularization and thereby hinder tumor growth. Uses of other plasmin inhibitors have also been previously described (*e.g.,* US 2009/0018069), for instance, to impair excessive bleeding in surgery, thrombolytic therapy and organ transplantation, and other clinical conditions that are characterized by a high incidence of bleeding diathesis.

Embodiments described herein may thus find therapeutic uses, but the present disclosure is not intended to be so limited and also contemplates exploitation of plasmin-inhibiting properties in other applications, such as in methods for protecting proteins from proteolytic degradation, as may be desirable in research (*e.g.,* biomedical and biochemical research) and industrial (*e.g.,* bioreactors, fermentation, recombinant protein expression, natural products isolation, etc.) settings.

The presently disclosed plasmin-inhibiting polypeptide may, in certain preferred embodiments, comprise a polypeptide of general formula (I):

N-Y-J-Z-C          (I)

wherein:

(a) N is an amino terminus of the plasmin-inhibiting polypeptide and consists of 0, 1, 2, 3, 4, 5, 6, 7, 8, 9, 10, 11, 12, 13, 14, 15, 16, 17, 18, 19, 20, 21, 22, 23, 24, 25-30, 31-40, 41-50, or 51-60 amino acids that are independently selected from natural and non-natural amino acids,
(b) Y is either nothing or methionine or N-formylmethionine,

(c) J is a Kunitz-type proteinase first inhibitor domain (KD1) polypeptide of 57 amino acids having the amino acid sequence set forth in SEQ ID NO:1,
(d) Z is a KD1 carboxy-region polypeptide that is selected from:

(i) a KD1 carboxy-region polypeptide of general formula (II): I-X3-K-V-X4-K (II) [SEQ ID NO:2]
in which X3 and X4 are amino acids independently selected from natural and non-natural amino acids and are not C, F, R or W,
(ii) a KD1 carboxy-region polypeptide of general formula (III): I-X3-K (III)
in which X3 is an amino acid selected from natural and non-natural amino acids and is not C, F, R or W,
(iii) a KD1 carboxy-region polypeptide of general formula (IV): I-X3-K-V-X5 (IV) [SEQ ID NO:20]
in which X3 and X5 are independent, X3 is an amino acid selected from natural and non-natural amino acids and is not C, F, R or W, and X5 is an amino acid selected from natural and non-natural amino acids and is not C, F, R or W,
(iv) a KD1 carboxy-region polypeptide of general formula (V): I-X3-K-V (V) [SEQ ID NO:21]
in which X3 is an amino acid selected from natural and non-natural amino acids and is not C, F, R or W,
(v) a KD1 carboxy-region polypeptide of general formula (VI):

I-X3-K-X6          (VI) [SEQ ID NO:30]

in which X3 and X6 are amino acids independently selected from natural and non-natural amino acids, X3 is not C, F, R or W, and X6 is any natural or non-natural amino acid, and
(vi) a KD1 carboxy-region polypeptide of general formula (VII):

I-X3-K-X6-X7          (VII) [SEQ ID NO:31]

in which X3, X6 and X7 are amino acids independently selected from natural and non-natural amino acids, X3 is not C, F, R or W, X6 is any natural or non-natural amino acid, and X7 is any natural or non-natural amino acid,

(e) C is a carboxy terminus of the plasmin-inhibiting polypeptide and consists of 0, 1, 2, 3, 4, 5, 6, 7, 8, 9, 10, 11, 12, 13, 14, 15, 16 17, 18, 19, 20, 21, 22, 23, 24, 25-30, 31-40, 41-50, or 51-60 amino acids that are independently selected from natural and non-natural amino acids, and
(f) the plasmin-inhibiting polypeptide inhibits plasmin activity, and optionally has decreased anti-coagulation activity compared to a wild type human tissue factor pathway inhibitor-2 (TFPI-2) polypeptide first Kunitz-type proteinase inhibitor domain (KD1) having the amino acid sequence set forth in SEQ ID NO:19.

In certain embodiments the plasmin-inhibiting polypeptide is selected from:

(a) the plasmin-inhibiting polypeptide in which N, Y and C are each nothing, J is the KD1 polypeptide having the amino acid sequence set forth in SEQ ID NO:1, and Z is the KD1 carboxy-region polypeptide of general formula (II):

I-X3-K-V-X4-K          (II) (SEQ ID NO: 2)

in which X3 and X4 are amino acids independently selected from natural and non-natural amino acids and are not C, F, R or W,
(b) the plasmin-inhibiting polypeptide in which N, Y and C are each nothing, J is the KD1 polypeptide having the amino acid sequence set forth in SEQ ID NO:1, and Z is the KD1 carboxy-region polypeptide of general formula (III):

I-X3-K          (III)

in which X3 is an amino acid selected from natural and non-natural amino acids and is not C, F, R or W,
(c) the plasmin-inhibiting polypeptide in which N, Y and C are each nothing, J is the KD1 polypeptide having the amino acid sequence set forth in SEQ ID NO:1, and Z is the KD1 carboxy-region polypeptide of general formula (IV):

I-X3-K-V-X5          (IV) [SEQ ID NO:20]

in which X3 and X5 are independent, X3 is an amino acid selected from natural and non-natural amino acids and is not C, F, R or W, and X5 is an amino acid selected from natural and non-natural amino acids and is not C, F, R or W,
(d) the plasmin-inhibiting polypeptide in which N, Y and C are each nothing, J is the KD1 polypeptide having the amino acid sequence set forth in SEQ ID NO:1, and Z is the KD1 carboxy-region polypeptide of general formula (V):

I-X3-K-V          (V) [SEQ ID NO:21]

in which X3 is an amino acid selected from natural and non-natural amino acids and is not C, F, R or W,
(e) the plasmin-inhibiting polypeptide in which N and C are each nothing, Y is methionine or N-formylmethionine, J is the KD1 polypeptide having the amino acid sequence set forth in SEQ ID NO:1, and Z is the KD1 carboxy-region polypeptide of general formula (II):

I-X3-K-V-X4-K          (II) (SEQ ID NO: 2)

in which X3 and X4 are amino acids independently selected from natural and non-natural amino acids and are not C, F, R or W,
(f) the plasmin-inhibiting polypeptide in which N and C are each nothing, Y is methionine or N-formylmethionine, J is the KD1 polypeptide having the amino acid sequence set forth in SEQ ID NO:1, and Z is the KD1 carboxy-region polypeptide of general formula (III):

I-X3-K          (III)

in which X3 is an amino acid selected from natural and non-natural amino acids and is not C, F, R or W,
(g) the plasmin-inhibiting polypeptide in which N and C are each nothing, Y is methionine or N-formylmethionine, J is the KD1 polypeptide having the amino acid sequence set forth in SEQ ID NO:1, and Z is the KD1 carboxy-region polypeptide of general formula (IV):

I-X3-K-V-X5          (IV) [SEQ ID NO:20]

in which X3 and X5 are independent, X3 is an amino acid selected from natural and non-natural amino acids and is not C, F, R or W, and X5 is an amino acid selected from natural and non-natural amino acids and is not C, F, R or W, and
(h) the plasmin-inhibiting polypeptide in which N and C are each nothing, Y is methionine or N-formylmethionine, J is the KD1 polypeptide having the amino acid sequence set forth in SEQ ID NO:1, and Z is the KD1 carboxy-region polypeptide of general formula (V):

I-X3-K-V          (V) [SEQ ID NO:21]

in which X3 is an amino acid selected from natural and non-natural amino acids and is not C, F, R or W.

In certain embodiments the plasmin-inhibiting polypeptide is selected from:

(a) the plasmin-inhibiting polypeptide in which N, Y and C are each nothing, J is the KD1 polypeptide having the amino acid sequence set forth in SEQ ID NO:1, and Z is the KD1 carboxy-region polypeptide of formula (II) having the sequence IEKVPK [SEQ ID NO:2 in which X3 is E and X4 is P, SEQ ID NO: 39],
(b) the plasmin-inhibiting polypeptide in which N, Y and C are each nothing, J is the KD1 polypeptide having the amino acid sequence set forth in SEQ ID NO:1, and Z is the KD1 carboxy-region polypeptide of formula (III) having the sequence IEK,
(c) the plasmin-inhibiting polypeptide in which N, Y and C are each nothing, J is the KD1 polypeptide having the amino acid sequence set forth in SEQ ID NO:1, and Z is the KD1 carboxy-region polypeptide of formula (IV) having the sequence IEKVP [SEQ ID NO: 40; SEQ ID NO:20 in which X3 is E and X5 is P],
(d) the plasmin-inhibiting polypeptide in which N, Y and C are each nothing, J is the KD1 polypeptide having the amino acid sequence set forth in SEQ ID NO:1, and Z is the KD1 carboxy-region polypeptide of formula (V) having the sequence IEKV [SEQ ID NO: 41; SEQ ID NO:21 in which X3 is E],
(e) the plasmin-inhibiting polypeptide in which N and C are each nothing, Y is methionine or N-formylmethionine, J is theKD1 polypeptide having the amino acid sequence set forth in SEQ ID NO:1, and Z is the KD1 carboxy-region polypeptide of formula (II) having the sequence IEKVPK [SEQ ID NO:2 in which X3 is E and X4 is P, SEQ ID NO: 39],
(f) the plasmin-inhibiting polypeptide in which N and C are each nothing, Y is methionine or N-formylmethionine, J is the KD1 polypeptide having the amino acid sequence set forth in SEQ ID NO:1, and Z is the KD1 carboxy-region polypeptide of formula (III) having the sequence IEK,
(g) the plasmin-inhibiting polypeptide in which N and C are each nothing, Y is methionine or N-formylmethionine, J is the KD1 polypeptide having the amino acid sequence set forth in SEQ ID NO:1, and Z is the KD1 carboxy-region polypeptide of formula (IV) having the sequence IEKVP [SEQ ID NO: 40; SEQ ID NO:20 in which X3 is E and X5 is P], and

(h) the plasmin-inhibiting polypeptide in which N and C are each nothing, Y is methionine or N-formylmethionine, J is the KD1 polypeptide having the amino acid sequence set forth in SEQ ID NO:1, and Z is the KD1 carboxy-region polypeptide of formula (V) having the sequence IEKV [SEQ ID NO: 41; SEQ ID NO:21 in which X3 is E].

In certain embodiments there is provided a plasmin-inhibiting polypeptide of no more than 200, 190, 180, 170, 160, 150, 140, 130, 120, 110, 100, 90, 89, 88, 87, 86, 85, 84, 83, 82, 81, 80, 79, 78, 77, 76, 75, 74, 73, 72, 71, 70, 69, 68, 67, 66, 65, 64 or 63 amino acids, comprising the amino acid sequence set forth in any one of SEQ ID NOS:3-18, 22-29 and 32-35, wherein the plasmin-inhibiting polypeptide inhibits plasmin activity and optionally wherein the plasmin-inhibiting polypeptide has decreased anti-coagulation activity compared to a wild type human tissue factor pathway inhibitor-2 (TFPI-2) polypeptide first Kunitz-type proteinase inhibitor domain (KD1) having the amino acid sequence set forth in SEQ ID NO:19.

In certain embodiments there is provided a plasmin-inhibiting polypeptide of 60 or 61 amino acids that has an amino acid sequence that is at least 96, 97, 98 or 99% identical to the amino acid sequence of a plasmin-inhibiting polypeptide of general formula (I):

N-Y-J-Z-C          (I)

in which:

(a) N is an amino terminus of the plasmin-inhibiting polypeptide,
(b) Y is either nothing or methionine or N-formylmethionine,
(c) J is a Kunitz-type proteinase first inhibitor domain (KD1) polypeptide of 57 amino acids having the amino acid sequence set forth in SEQ ID NO:1, in which amino acid X1 at position 11 in SEQ ID NO:1 is D, N or S, amino acid X2 at position 17 in SEQ ID NO:1 is R, and a cysteine residue is present at each of amino acid positions 14 and 38 in SEQ ID NO:1,
(d) Z is a KD1 carboxy-region polypeptide of general formula (III) :

I-X3-K          (III)

in which X3 is an amino acid selected from natural and non-natural amino acids and is not C, F, R or W,
(e) C is a carboxy terminus of the plasmin-inhibiting polypeptide, and
(f) the plasmin-inhibiting polypeptide inhibits plasmin activity, and optionally has decreased anti-coagulation activity compared to a wild type human tissue factor pathway inhibitor-2 (TFPI-2) polypeptide first Kunitz-type proteinase inhibitor domain (KD1) having the amino acid sequence set forth in SEQ ID NO:19.

In certain other embodiments there is provided a plasmin-inhibiting polypeptide of 63 or 64 amino acids that has an amino acid sequence that is at least 94, 95, 96, 97, 98 or 99% identical to the amino acid sequence of a plasmin-inhibiting polypeptide of general formula (I):

N-Y-J-Z-C          (I)

in which:

(a) N is an amino terminus of the plasmin-inhibiting polypeptide,
(b) Y is either nothing or methionine or N-formylmethionine,
(c) J is a Kunitz-type proteinase first inhibitor domain (KD1) polypeptide of 57 amino acids having the amino acid sequence set forth in SEQ ID NO:1, in which amino acid X1 at position 11 in SEQ ID NO:1 is D, N or S, amino acid X2 at position 17 in SEQ ID NO:1 is R, and a cysteine residue is present at each of amino acid positions 14 and 38 in SEQ ID NO:1,
(d) Z is a KD1 carboxy-region polypeptide of general formula (II):

I-X3-K-V-X4-K          (II) [SEQ ID NO:2]

in which X3 and X4 are amino acids independently selected from natural and non-natural amino acids and are not C, F, R or W,

(e) C is a carboxy terminus of the plasmin-inhibiting polypeptide, and

(f) the plasmin-inhibiting polypeptide inhibits plasmin activity, and optionally has decreased anti-coagulation activity compared to a wild type human tissue factor pathway inhibitor-2 (TFPI-2) polypeptide first Kunitz-type proteinase inhibitor domain (KD1) having the amino acid sequence set forth in SEQ ID NO:19.

In certain other embodiments there is provided a plasmin-inhibiting polypeptide of 61, 62, 63 or 64 amino acids that has an amino acid sequence that is at least 94, 95, 96, 97, 98 or 99% identical to the amino acid sequence of a plasmin-inhibiting polypeptide of general formula (I):

$$N-Y-J-Z-C \qquad (I)$$

in which:

(a) N is an amino terminus of the plasmin-inhibiting polypeptide,
(b) Y is either nothing or methionine or N-formylmethionine,
(c) J is a Kunitz-type proteinase first inhibitor domain (KD1) polypeptide of 57 amino acids having the amino acid sequence set forth in SEQ ID NO:1, in which amino acid X1 at position 11 in SEQ ID NO:1 is D, N or S, amino acid X2 at position 17 in SEQ ID NO:1 is R, and a cysteine residue is present at each of amino acid positions 14 and 38 in SEQ ID NO:1,
(d) Z is a KD1 carboxy-region polypeptide that is selected from:

(i) a KD1 carboxy-region polypeptide of general formula (II):

$$I-X3-K-V-X4-K \qquad (II) \text{ [SEQ ID NO:2]}$$

in which X3 and X4 are amino acids independently selected from natural and non-natural amino acids and are not C, F, R or W,
(ii) a KD1 carboxy-region polypeptide of general formula (III):

$$I-X3-K \qquad (III)$$

in which X3 is an amino acid selected from natural and non-natural amino acids and is not C, F, R or W,
(iii) a KD1 carboxy-region polypeptide of general formula (IV):

$$I-X3-K-V-X5 \qquad (IV) \text{ [SEQ ID NO:20]}$$

in which X3 and X5 are independent, X3 is an amino acid selected from natural and non-natural amino acids and is not C, F, R or W, and X5 is an amino acid selected from natural and non-natural amino acids and is not C, F, R or W,
(iv) a KD1 carboxy-region polypeptide of general formula (V):

$$I-X3-K-V \qquad (V) \text{ [SEQ ID NO:21]}$$

in which X3 is an amino acid selected from natural and non-natural amino acids and is not C, F, R or W,
(v) a KD1 carboxy-region polypeptide of general formula (VI):

$$I-X3-K-X6 \qquad (VI) \text{ [SEQ ID NO:30]}$$

in which X3 and X6 are amino acids independently selected from natural and non-natural amino acids, X3 is not C, F, R or W, and X6 is any natural or non-natural amino acid, and
(vi) a KD1 carboxy-region polypeptide of general formula (VII):

$$I-X3-K-X6-X7 \qquad (VII) \text{ [SEQ ID NO:31]}$$

in which X3, X6 and X7 are amino acids independently selected from natural and non-natural amino acids, X3 is not C, F, R or W, X6 is any natural or non-natural amino acid, and X7 is any natural or non-natural amino acid,

(e) C is a carboxy terminus of the plasmin-inhibiting polypeptide, and

(f) the plasmin-inhibiting polypeptide inhibits plasmin activity, and optionally has decreased anti-coagulation activity compared to a wild type human tissue factor pathway inhibitor-2 (TFPI-2) polypeptide first Kunitz-type proteinase inhibitor domain (KD1) having the amino acid sequence set forth in SEQ ID NO:19.

The wild type first Kunitz-type proteinase inhibitor domain (KD1) of human tissue factor pathway inhibitor-2 (TFPI-2; SEQ ID NO:19; for the complete TFPI-2 KD1 sequence see also Fig. 4 in Chand et al., 2004 J. Biol. Chem. 279:17500, at page 17505) may be referred to as a reference sequence in which numbered amino acid positions are described as having corresponding positions in the presently described plasmin-inhibiting polypeptides when similar sequences are aligned using sequence homology tools as described herein. As described herein, three-dimensional protein modeling studies revealed that the substitution of tyrosine by threonine at position 11 in KD1 (e.g., Y11T, as in SEQ ID NOS:3, 4, 11, 15, 24, 25, 32, 33) in addition to the substitution of leucine by arginine at position 17 (L17R) to arrive at the present plasmin-inhibiting polypeptides, allowed for hydrogen bond formation between the plasmin-inhibiting polypeptide and plasmin. This hydrogen bonding strengthens the interactions between plasmin and the plasmin-inhibiting polypeptide, relative to the interactions that are permitted when only the L17R substitution has been made (e.g., as in SEQ ID NOS:5, 6, 12, 23). In the modeling studies it was also revealed that the side chain of the lysine residue at position 60 in the plasmin-inhibiting polypeptide (e.g., SEQ ID NO:3) was observed to fit into the lysine binding site (LSB) of the human plasmin kringle domain.

Accordingly, in certain embodiments there are provided 60-amino acid (e.g., SEQ ID NO:5) and 63-amino acid plasmin-inhibiting polypeptides containing the L17R substitution (e.g., SEQ ID NO:6) and having carboxy-terminal lysine, which polypeptides are active as inhibitors of the plasmin active site and also bind to the lysine binding site(s) in the Kringle domain(s) of plasmin, thus providing two modes of inhibitory activity of plasmin (i.e., dual activity). Further, the 63-amino acid forms retain the ability to interact with the lysine binding site even following removal of the C-terminal K63 by activated thrombin activated fibrinolysis inhibitor (TAFI) in vivo, because the K60 residue persists in the resulting 62-amino acid form and is available to interact with the lysine binding site in plasmin. The 60-amino acid and 63-amino acid plasmin inhibiting polypeptides containing both the substitution at position 11 (e.g., SEQ ID NO:3, for instance, Y11T) and the substitution at position 17 (e.g., SEQ ID NO:3, for instance L17R) are even more potent plasmin inhibitors and are dually reactive by virtue of binding to the lysine binding sites on plasminogen/plasmin via C-terminal lysine.

POLYPEPTIDES AND PROTEINS

The terms "polypeptide" "protein" and "peptide" and "glycoprotein" are used interchangeably and mean a polymer of amino acids not limited to any particular length. The term does not exclude modifications such as myristylation, sulfation, glycosylation, phosphorylation, formylation, and addition or deletion of signal sequences. The terms "polypeptide" or "protein" means one or more chains of amino acids, wherein each chain comprises amino acids covalently linked by peptide bonds, and wherein said polypeptide or protein can comprise a plurality of chains non-covalently and/or covalently linked together by peptide bonds, having the sequence of native proteins, that is, proteins produced by naturally-occurring and specifically non-recombinant cells, or genetically-engineered or recombinant cells, and comprise molecules having the amino acid sequence of the native protein, or molecules having deletions from, additions to, and/or substitutions of one or more amino acids of the native sequence. Thus, a "polypeptide" or a "protein" can comprise one (termed "a monomer") or a plurality (termed "a multimer") of amino acid chains. The terms "peptide," "polypeptide" and "protein" specifically encompass the immunomodulatory polypeptides of the present disclosure, or sequences that have deletions from, additions to, and/or substitutions of one or more amino acid of an immunomodulatory polypeptide.

The term "isolated" means that the material is removed from its original environment (e.g., the natural environment if it is naturally occurring). For example, a naturally occurring polypeptide or nucleic acid present in a living animal is not isolated, but the same polypeptide or nucleic acid, separated from some or all of the co-existing materials in the natural system, is isolated. Such nucleic acid could be part of a vector and/or such nucleic acid or polypeptide could be part of a composition (e.g., a cell lysate), and still be isolated in that such vector or composition is not part of the natural environment for the nucleic acid or polypeptide. The term "gene" means the segment of DNA involved in producing a polypeptide chain; it includes regions preceding and following the coding region "leader and trailer" as well as intervening sequences (introns) between individual coding segments (exons).

The terms "isolated protein" and "isolated polypeptide" referred to herein means that a subject protein or polypeptide (1) is free of at least some other proteins or polypeptides with which it would typically be found in nature, (2) is essentially free of other proteins or polypeptides from the same source, e.g., from the same species, (3) is expressed by a cell from a different species, (4) has been separated from at least about 50 percent of polynucleotides, lipids, carbohydrates, or other materials with which it is associated in nature, (5) is not associated (by covalent or noncovalent interaction) with portions of a protein or polypeptide with which the "isolated protein" or "isolated polypeptide" may be associated in nature, (6) is operably associated (by covalent or noncovalent interaction) with a polypeptide with which it is not associated in nature, or (7) does not occur in nature. Such an isolated protein or polypeptide can be encoded by genomic DNA, cDNA, mRNA or other RNA, of may be of synthetic origin according to any of a number of well known chemistries for artificial peptide and protein synthesis, or any combination thereof. In certain embodiments, the isolated protein or polypeptide is substantially free from proteins or polypeptides or other contaminants that are found in its natural environment that would interfere with its use (therapeutic, diagnostic, prophylactic, research or otherwise).

The term "polypeptide fragment" refers to a polypeptide, which can be monomeric or multimeric, that has an amino-terminal deletion, a carboxyl-terminal deletion, and/or an internal deletion or substitution of a naturally-occurring or recombinantly-produced polypeptide. As used herein, "contiguous amino acids" refers to covalently linked amino acids corresponding to an uninterrupted linear portion of a disclosed amino acid sequence. In certain embodiments, a polypeptide fragment can comprise an amino acid chain at least 5 to about 500 amino acids long. It will be appreciated that in certain embodiments, fragments are at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 110, 150, 200, 250, 300, 350, 400, or 450 amino acids long.

Polypeptides may comprise a signal (or leader) sequence at the N-terminal end of the protein, which co-translationally or post-translationally directs transfer of the protein. The polypeptide may also be fused in-frame or conjugated to a linker or other sequence for ease of synthesis, purification or identification of the polypeptide (*e.g.,* poly-His), or to enhance binding of the polypeptide to a solid support. Fusion domain polypeptides may be joined to the polypeptide at the N-terminus and/or at the C-terminus, and may include as non-limiting examples, immunoglobulin-derived sequences such as Ig constant region sequences or portions thereof, affinity tags such as His tag (*e.g.,* hexahistidine or other polyhistidine), FLAG™ or myc or other peptide affinity tags, detectable polypeptide moieties such as green fluorescent protein (GFP) or variants thereof (*e.g.,* yellow fluorescent protein (YFP), blue fluorescent protein (BFP), other aequorins or derivatives thereof, etc.) or other detectable polypeptide fusion domains, enzymes or portions thereof such as glutathione-S-transferase (GST) or other known enzymatic detection and/or reporter fusion domains, and the like, as will be familiar to the skilled artisan.

Recombinant protein expression systems are known in the art and may in certain embodiments be used to produce the herein described plasmin-inhibiting polypeptides, as also described below. For example, certain bacterial expression systems such as *E. coli* recombinant protein expression systems yield polypeptide products having N-terminal formylated methionine. In some situations the plasmin-inhibiting polypeptide may therefore comprise an N-terminal methionine residue (which may be unmodified methionine or formylmethionine or another methionine analog, variant, mimetic or derivative as provided herein), sometimes referred to as initiator methionine, immediately preceding the Kunitz-type proteinase first inhibitor domain (KD1) polypeptide sequence. Non-limiting examples of the presently described plasmin-inhibiting polypeptides that contain such an N-terminal methionine (*e.g.,* as methionine or N-formylmethionine) include those having the amino acid sequences set forth in SEQ ID NOS:7-10, 13, 14, 17, 18, 26-29, 34 and 35. Also contemplated are embodiments in which one or more of these or other herein described plasmin-inhibiting polypeptides containing N-terminal methionine (*e.g.,* as methionine or N-formylmethionine) may be recombinantly expressed according to art-accepted practices in a host cell that also expresses methionine aminopeptidase (MAP), an enzyme that is capable of cleaving the N-terminal methionine to remove it from the nascent polypeptide product. See, *e.g.,* Natarajan et al., 2011 PLoS ONE 6(5): e20176; Shen et al., 1993 Proc. Natl. Acad. Sci. USA 90:8108; Shen et al., 1997 Prot. Eng. 10:1085. Alternatively, the MAP enzyme itself may be produced recombinantly (*e.g.,* Tsunasawa et al., 1997 J. Biochem. 122:843; Bradshaw et al., 1998 Trends Bioch. Sci. 23:263; Ben-Bassat et al., 1987 J. Bacteriol. 169:751) or obtained commercially (Sigma-Aldrich, St. Louis, MO, *e.g.,* catalog number M6435) and used to remove N-terminal methionine from the present plasmin-inhibiting polypeptides post-synthesis.

Cysteine-containing peptides may be used as fusion peptides that can be joined to the N- and/or C-terminus of the herein described plasmin-inhibiting polypeptides to permit ready assembly of such polypeptides into disulfide-crosslinked dimers, trimers, tetramers or higher multimers according to established methodologies. For example, fusion polypeptides containing immunoglobulin gene superfamily member-derived sequences that include cysteine residues capable of forming interchain disulfide bridges are well known, as also are other strategies for engineering S-S linked multimers (*e.g.,* Reiter et al., 1994 Prot. Eng. 7:697; Zhu et al., 1997 Prot. Sci. 6:781; Mabry et al., 2010 Mabs 2:20; Gao et al., 1999 Proc. Nat. Acad. Sci. USA 96:6025; Lim et al., 2010 Biotechnol. Bioeng. 106:27) Alternative approaches are also contemplated for grafting peptide sequences that promote multimer assembly as fusion domains onto a desired polypeptide such as the herein described immunomodulatory peptides (*e.g.,* Fan et al., 2008 FASEB J. 22:3795).

Polypeptide modifications may be effected biosynthetically and/or chemically according to a wide variety of well known methodologies, and may also include conjugation to carrier proteins (*e.g.,* keyhole limpet hemocyanin (KLH), bovine serum albumin (BSA), ovalbumin (OVA) or other molecules), and covalent or non-covalent immobilization on solid supports. Chemical or biosynthetic conjugation to a carrier is contemplated, according to certain embodiments.

A peptide linker/spacer sequence may also be employed to separate multiple polypeptide components by a distance sufficient to ensure that each polypeptide folds into its secondary and/or tertiary structures, if desired. Such a peptide linker sequence can be incorporated into a fusion polypeptide using standard techniques well known in the art. Certain peptide spacer sequences may be chosen, for example, based on: (1) their ability to adopt a flexible extended conformation; (2) their inability to adopt a secondary structure that could interact with functional epitopes on the first and second polypeptides; and/or (3) the lack of hydrophobic or charged residues that could react with the polypeptide functional epitopes.

In one illustrative embodiment, peptide spacer sequences contain, for example, Gly, Asn and Ser residues. Other near neutral amino acids, such as Thr and Ala, may also be included in the spacer sequence. Other amino acid sequences which may be usefully employed as spacers include those disclosed in Maratea et al., Gene 40:39 46 (1985); Murphy et al., Proc. Natl. Acad. Sci. USA 83:8258 8262 (1986); U.S. Pat. No. 4,935,233 and U.S. Pat. No. 4,751,180. Other illustrative spacers may include, for example, Glu-Gly-Lys-Ser-Ser-Gly-Ser-Gly-Ser-Glu-Ser-Lys-Val-Asp (SEQ ID NO: 36) (Chaudhary et al., 1990, Proc. Natl. Acad. Sci. U.S.A. 87:1066-1070) and Lys-Glu-Ser-Gly-Ser-Val-Ser-Ser-Glu-Gln-Leu-Ala-Gln-Phe-Arg-Ser-Leu-Asp (SEQ ID NO: 37) (Bird et al., 1988, Science 242:423-426).

In some embodiments, spacer sequences are not required when the first and second polypeptides have non-essential N-terminal amino acid regions that can be used to separate the functional domains and prevent steric interference. Two coding sequences can be fused directly without any spacer or by using a flexible polylinker composed, for example, of the pentamer Gly-Gly-Gly-Gly-Ser (SEQ ID NO: 38) repeated one to three times.

In certain illustrative embodiments, a peptide spacer is between 1 to 5 amino acids, between 5 to 10 amino acids, between 5 to 25 amino acids, between 5 to 50 amino acids, between 10 to 25 amino acids, between 10 to 50 amino acids, between 10 to 100 amino acids, or any intervening range of amino acids. In other illustrative embodiments, a peptide spacer comprises about 1, 5, 10, 15, 20, 25, 30, 35, 40, 45, 50 or more amino acids in length.

According to certain preferred embodiments a plasmin-inhibiting polypeptide may comprise a peptide, polypepetide or peptidomimetic that includes, or that shares close sequence identity to or structural features with, a polypeptide of no more than 200, 190, 180, 170, 160, 150, 140, 130, 120, 110, 100, 90, 89, 88, 87, 86, 85, 84, 83, 82, 81, 80, 79, 78, 77, 76, 75, 74, 73, 72, 71, 70, 69, 68, 67, 66, 65, 64, or 63 amino acids, comprising the amino acid sequence set forth in any one of SEQ ID NOS:3-18, 22-29 and 32-35, wherein the plasmin-inhibiting polypeptide inhibits plasmin activity and has decreased anti-coagulation activity compared to a wild type human tissue factor pathway inhibitor-2 (TFPI-2) polypeptide first Kunitz-type proteinase inhibitor domain (KD1) having the amino acid sequence set forth in SEQ ID NO:19. In certain embodiments the plasmin-inhibiting polypeptide comprises or consists of the 60-amino acid sequence set forth in any one of SEQ ID NOS:3, 5, 15 or 16. In certain embodiments the plasmin-inhibiting polypeptide comprises or consists of the 61-amino acid sequence set forth in any one of SEQ ID NOS:7, 9, 17, 18, 22, 24 or 32. In certain embodiments the plasmin-inhibiting polypeptide comprises or consists of the 62-amino acid sequence set forth in any one of SEQ ID NOS:23, 25, 26, 28, 33 or 34. In certain embodiments the plasmin-inhibiting polypeptide comprises or consists of the 63-amino acid sequence set forth in any one of SEQ ID NOS:4, 6, 10, 11, 13, 14, 27, 29 or 35.

Methods for the determination of plasmin-inhibiting activity and anti-coagulation activity are known in the art and are described, for example, in Chand et al. (2004 J. Biol. Chem. 279:17500), Petersen et al. (1996 Biochem. 35:266) and in US 2009/0018069 (*e.g.,* at paragraphs 0062-0070, 0124-0127, 0133-0135, 0137-0138, and elsewhere). Accordingly, a plasmin-inhibiting polypeptide may be identified on the basis of inhibition of plasmin activity (*e.g.,* plasmin proteolytic activity) that is detectable in a statistically significant manner, which in certain embodiments may be detectable inhibition of at least 10%, 20%, 30%, 40%, 50%, 60%, 70%, 80%, 90%, 95% of the plasmin activity that is present in the absence of the plasmin-inhibiting polypeptide. An exemplary assay for plasmin inhibition measures changes in the solution optical density at 405 nm that reflect liberation by plasmin, in the absence and presence of a candidate plasmin inhibitor, of *p*-nitroaniline from the chromogenic plasmin substrate H-D-valyl-leucyl-lysine p-nitroaniline dihydrochloride (S2251, Chromogenix/DiaPharma Group, West Chester, OH).

As generally referred to in the art, and as used herein, sequence identity and sequence homology may be used interchangeably and generally refer to the percentage of nucleotides or amino acid residues in a candidate sequence that are identical with, respectively, the nucleotides or amino acid residues in a reference polynucleotide or polypeptide sequence, after aligning the sequences and introducing gaps, if necessary, to achieve the maximum percent sequence identity, and optionally not considering any conservative substitutions as part of the sequence identity. In certain embodiments, a plasmin-inhibiting polypeptide or encoding polynucleotide of the embodiments disclosed herein shares at least about 75%, at least about 80%, at least about 85%, at least about 90%, 91%, 92%, 93% or 94%, or at least about 95%, 96%, 97%, 98%, or 99% of the amino acid residues (or of the nucleotides in a polynucleotide encoding such a plasmin-inhibiting polypeptide) with the sequence of the plasmin-inhibiting polypeptide of general formula (I) [N-Y-J-Z-C] as described herein in which J is SEQ ID NO:1 and Z is selected from general formula (II) [SEQ ID NO:2], general formula (III) (I-X3-K in which X3 is a natural or non-natural amino acid that is not C, F, R or W), general formula (IV) [SEQ ID NO:20] or general formula (V) [SEQ ID NO:21]. Such sequence identity may be determined according to well known sequence analysis algorithms, including those available from the University of Wisconsin Genetics Computer Group (Madison, WI), such as FASTA, Gap, Bestfit, BLAST, or others.

"Natural or non-natural amino acid" includes any of the common naturally occurring amino acids which serve as building blocks for the biosynthesis of peptides, polypeptides and proteins (*e.g.,* alanine (A), cysteine (C), aspartic acid (D), glutamic acid (E), phenylalanine (F), glycine (G), histidine (H), isoleucine (I), lysine (K), leucine (L), methionine (M), asparagine (N), proline (P), glutamine (Q), arginine (R), serine (S), threonine (T), valine (V), tryptophan (W), tyrosine(Y)) and also includes modified, derivatized, enantiomeric, rare and/or unusual amino acids, whether naturally occurring or synthetic, for instance, N-formylmethionine, hydroxyproline, hydroxylysine, desmosine, isodesmosine, ε-N-methyllysine, ε-N-trimethyllysine, methylhistidine, dehydrobutyrine, dehydroalanine, α-aminobutyric acid, β-alanine, γ-aminobutyric acid, homocysteine, homoserine, citrulline, ornithine and other amino acids that may be isolated from a natural source and/or that may be chemically synthesized, for instance, as may be found in Proteins, Peptides and Amino Acids Sourcebook (White, J.S. and White, D.C., 2002 Humana Press, Totowa, NJ) or in Amino Acid and Peptide Synthesis (Jones, J., 2002 Oxford Univ. Press USA, New York) or in Unnatural Amino Acids, ChemFiles Vol. 1, No. 5 (2001 Fluka Chemie GmbH; Sigma-Aldrich, St. Louis, MO) or in Unnatural Amino Acids II, ChemFiles Vol. 2, No. 4 (2002 Fluka Chemie GmbH; Sigma-Aldrich, St. Louis, MO). Additional descriptions of natural and/or non-natural amino acids may be found, for example, in Kotha, 2003 Acc. Chem. Res. 36:342; Maruoka et al., 2004 Proc. Nat. Acad. Sci. USA 101:5824; Lundquist et al., 2001 Org. Lett. 3:781; Tang et al., 2002 J. Org. Chem. 67:7819; Rothman et al., 2003 J. Org. Chem. 68:6795; Krebs et al., 2004 Chemistry 10:544; Goodman et al., 2001 Biopolymers 60:229; Sabat et al., 2000 Org. Lett. 2:1089; Fu et al., 2001 J. Org. Chem. 66:7118; and Hruby et al., 1994 Meths. Mol. Biol. 35:249. The standard three-letter abbreviations and one-letter symbols are used herein to designate natural and non-natural amino acids.

Other non-natural amino acids or amino acid analogues are known in the art and include, but are not limited to, non-natural L or D derivatives (such as D-amino acids present in peptides and/or peptidomimetics such as those presented above and elsewhere herein), fluorescent labeled amino acids, as well as specific examples including O-methyl-L-tyrosine, an L-3-(2-naphthyl)alanine, a 3-methyl-phenylalanine, 3-idio-tyrosine, O-propargyl-tyrosine, homoglutamine, an O-4-allyl-L-tyrosine, a 4-propyl-L-tyrosine, a 3-nitro-L-tyrosine, a tri-O-acetyl-GlcNAcβ-serine, an L-Dopa, a fluorinated phenylalanine, an isopropyl-L-phenylalanine, a p-azido-L-phenylalanine, a p-acyl-L-phenylalanine, a p-acetyl-L-phenylalanine, an m-acetyl-L-phenylalanine, selenomethionine, telluromethionine, selenocysteine, an alkyne phenylalanine, an O-allyl-L-tyrosine, an O-(2-propynyl)-L-tyrosine, a p-ethylthiocarbonyl-L-phenylalanine, a p-(3-oxobutanoyl)-L-phenylalanine, a p-benzoyl-L-phenylalanine, an L-phosphoserine, a phosphonoserine, a phosphonotyrosine, homopropargylglycine, azidohomoalanine, a p-iodo-phenylalanine, a p-bromo-L-phenylalanine, dihydroxy-phenylalanine, dihydroxyl-L-phenylalanine, a p-nitro-L-phenylalanine, an m-methoxy-L-phenylalanine, a p-iodo-phenylalanine, a p-bromophenylalanine, a p-amino-L-phenylalanine, and an isopropyl-L-phenylalanine, trifluoroleucine, norleucine ("Nle"), D-norleucine ("dNle" or "D-Nle"), 5-fluoro-tryptophan, para-halo-phenylalanine, homo-phenylalanine ("homo-Phe"), seleno-methionine, ethionine, S-nitroso-homocysteine, thia-proline, 3-thienyl-alanine, homo-allyl-glycine, trifluoroisoleucine, trans and cis-2-amino-4-hexenoic acid, 2-butynyl-glycine, allyl-glycine, para-azido-phenylalanine, para-cyano-phenylalanine, para-ethynyl-phenylalanine, hexafluoroleucine, 1,2,4-triazole-3-alanine, 2-fluoro-histidine, L-methyl histidine, 3-methyl-L-histidine, β-2-thienyl-L-alanine, β-(2-thiazolyl)-DL-alanine, homoproparglyglycine (HPG) and azidohomoalanine (AHA) and the like.

In certain embodiments a natural or non-natural amino acid may be present that comprises an aromatic side chain, as found, for example, in phenylalanine or tryptophan or analogues thereof including in other natural or non-natural amino acids based on the structures of which the skilled person will readily recognize when an aromatic ring system is present, typically in the form of an aromatic monocyclic or multicyclic hydrocarbon ring system consisting only of hydrogen and carbon and containing from 6 to 19 carbon atoms, where the ring system may be partially or fully saturated, and which may be present as a group that includes, but need not be limited to, groups such as fluorenyl, phenyl and naphthyl.

In certain embodiments a natural or non-natural amino acid may be present that comprises a hydrophobic side chain as found, for example, in alanine, valine, isoleucine, leucine, proline, phenylalanine, tryptophan or methionine or analogues thereof including in other natural or non-natural amino acids based on the structures of which the skilled person will readily recognize when a hydrophobic side chain (*e.g.*, typically one that is non-polar when in a physiological milieu) is present. In certain embodiments a natural or non-natural amino acid may be present that comprises a basic side chain as found, for example, in lysine, arginine or histidine or analogues thereof including in other natural or non-natural amino acids based on the structures of which the skilled person will readily recognize when a basic (*e.g.*, typically polar and having a positive charge when in a physiological milieu) is present.

Plasmin-inhibiting polypeptides and proteins disclosed herein may include L- and/or D- amino acids so long as the biological activity of the polypeptide is maintained (*e.g.*, inhibition of plasmin proteolytic activity, and/or decreased anti-coagulation activity compared to TFPI-2 KD1 (SEQ ID NO:19)). The isolated plasmin-inhibiting polypeptides also may comprise in certain embodiments any of a variety of known natural and artificial post-translational or post-synthetic covalent chemical modifications by reactions that may include glycosylation (*e.g.*, N-linked oligosaccharide addition at asparagine residues, O-linked oligosaccharide addition at serine or threonine residues, glycation, or the like), fatty acylation, acetylation, formylation, PAGylation, PEGylation, and phosphorylation. Polypeptides herein disclosed may further include analogs, alleles and allelic variants which may contain amino acid deletions, or additions or substitutions of one or more amino acid residues with other naturally occurring amino acid residues or non-natural amino acid residues.

Peptide and non-peptide analogs may be referred to as peptide mimetics or peptidomimetics, and are known in the pharmaceutical industry (Fauchere, J. Adv. Drug Res. 15:29 (1986); Evans et al. J. Med. Chem. 30: 1229 (1987)). These compounds may contain one or more non-natural amino acid residue(s), one or more chemical modification moieties (for example, glycosylation, pegylation, fluorescence, radioactivity, or other moiety), and/or one or more non-natural peptide bond(s) (for example, a reduced peptide bond: --$CH_2$-$NH_2$--). Peptidomimetics may be developed by a variety of methods, including by computerized molecular modeling, random or site-directed mutagenesis, PCR-based strategies, chemical mutagenesis, and others.

Hence according to certain presently disclosed embodiments a plasmin-inhibiting polypeptide may comprise a polypeptide of no more than 200, 190, 180, 170, 160, 150, 140, 130, 120, 110, 100, 90, 89, 88, 87, 86, 85, 84, 83, 82, 81, 80, 79, 78, 77, 76, 75, 74, 73, 72, 71, 70, 69, 68, 67, 66, 65, 64 or 63 amino acids, comprising a polypeptide of general formula (I) [N-Y-J-Z-C] as described herein, which may in certain embodiments comprise the amino acid sequence set forth in any one of SEQ ID NOS:3-18, 22-29 and 32-35 wherein the plasmin-inhibiting polypeptide inhibits plasmin activity and has decreased anti-coagulation activity compared to a wild type human tissue factor pathway inhibitor-2 (TFPI-2) polypeptide first Kunitz-type proteinase inhibitor domain (KD1) having the amino acid sequence set forth in SEQ ID NO:19.

Accordingly in these and other embodiments it will be appreciated that the amino terminus of certain plasmin-inhibiting polypeptides may consist of 0, 1, 2, 3, 4, 5, 6, 7, 8, 9, 10, 11, 12, 13, 14, 15, 16, 17, 18, 19, 20, 21, 22, 23, 24, 25-30, 31-40, 41-50, or 51-60 independently selected natural or non-natural amino acids, and/or that in certain embodiments the carboxy terminus of the plasmin-inhibiting polypeptide may consist of 0, 1, 2, 3, 4, 5, 6, 7, 8, 9, 10, 11, 12, 13, 14, 15, 16, 17, 18, 19, 20, 21, 22, 23, 24, 25-30, 31-40, 41-50, or 51-60 independently selected natural or non-natural amino acids, where such amino and carboxy termini may have any sequence so long as the isolated plasmin-inhibiting polypeptide is of no more than 200, 190, 180, 170, 160, 150, 140, 130, 120, 110, 100, 90, 89, 88, 87, 86, 85, 84, 83, 82, 81, 80, 79, 78, 77, 76, 75, 74, 73, 72, 71, 70, 69, 68, 67, 66, 65, 64 or 63 amino acids and comprises a polypeptide of general formula (I) [N-Y-J-Z-C] as recited herein, and is capable of inhibiting plasmin activity while exhibiting decreased anti-coagulation activity compared to a wild type TFPI-2 KD1 having the amino acid sequence set forth in SEQ ID NO:19.

As described herein, in a polypeptide of general formula (I) [N-Y-J-Z-C] Y is either nothing or methionine (*e.g.,* N-terminal methionine as may be introduced in the course of recombinantly engineering and expressing the plasmin-inhibiting polypeptide; this may be N-formylmethionine when produced in bacteria); J is a Kunitz-type proteinase first inhibitor domain (KD1, see Chand et al. 2004 J. Biol. Chem. 279:17500) except that J is a polypeptide of 57 amino acids having the amino acid sequence set forth in SEQ ID NO:1; and Z is a KD1 carboxy-region polypeptide that may be 3, 4, 5, or 6 amino acids in length, depending on whether the KD1 carboxy-region polypeptide of general formula (III) [I-X3-K], general formula (V) [SEQ ID NO:21], general formula (VI) [SEQ ID NO:30], general formula (VII) [SEQ ID NO:31], general formula (IV) [SEQ ID NO:20], or general formula (II) [SEQ ID NO:2] is present. A number of representative plasmin-inhibiting polypeptides of general formula (I) are disclosed herein, such that in view of the present disclosure those familiar with the art will be able readily to make and use additional plasmin-inhibiting polypeptides according to the present embodiments.

For example, determination of the three-dimensional structures of representative plasmin-inhibiting polypeptides may be made through routine methodologies such that substitution of one or more amino acids with selected natural or non-natural amino acids can be virtually modeled for purposes of determining whether a so derived structural variant retains the space-filling properties of presently disclosed species. *See,* for instance, Bajaj et al., 2011 J. Biol. Chem. 286:4329; Chand et al., 2004 J. Biol. Chem. 279:17500; Bajaj, US 2009/0018069; Huber et al., 1974 J Mol Biol. 89:73-101; Schmidt et al., 2005 J Biol Chem. 280:27832-27838; Wang et al., 1998 Science 281, 1662-1665; Bajaj et al., 2001 Thromb. Haemost. 86, 959-972; see also, *e.g.,* Donate et al., 1994 Prot. Sci. 3:2378; Bradley et al., Science 309: 1868-1871 (2005); Schueler-Furman et al., Science 310:638 (2005); Dietz et al., Proc. Nat. Acad. Sci. USA 103:1244 (2006); Dodson et al., Nature 450:176 (2007); Qian et al., Nature 450:259 (2007); Raman et al. Science 327:1014-1018 (2010). Some additional non-limiting examples of computer algorithms that may be used for these and related embodiments, such as for rational design of immunomodulatory polypeptides as provided herein, include VMD which is a molecular visualization program for displaying, animating, and analyzing large biomolecular systems using 3-D graphics and built-in scripting (see the website for the Theoretical and Computational Biophysics Group, University of Illinois at Urbana-Champagne, at ks.uiuc.edu/Research/vmd/.

Many other computer programs are known in the art and available to the skilled person and which allow for determining atomic dimensions from space-filling models (van der Waals radii) of energy-minimized conformations; GRID, which seeks to determine regions of high affinity for different chemical groups, thereby enhancing binding, Monte Carlo searches, which calculate mathematical alignment, and CHARMM (Brooks et al. (1983) J. Comput. Chem. 4:187-217) and AMBER (Weiner et al (1981) J. Comput. Chem. 106: 765), which assess force field calculations, and analysis (see also, Eisenfield et al. (1991) Am. J. Physiol. 261:C376-386; Lybrand (1991) J. Pharm. Belg. 46:49-54; Froimowitz (1990) Biotechniques 8:640-644; Burbam et al. (1990) Proteins 7:99-111; Pedersen (1985) Environ. Health Perspect. 61:185-190; and Kini et al. (1991) J. Biomol. Struct. Dyn. 9:475-488). A variety of appropriate computational computer programs are also commercially available, such as from Schrödinger (Munich, Germany).

It is well known in the art that polyalkylene glycols, such as polyethylene glycol (PEG), may be attached to a therapeutic agent. Polyalkylene glycolated (PAGylated) therapeutic agents, and in particular, PEGylated therapeutic agents, have been reported to increase solubility, circulating life, safety, decrease renal excretion, and decrease immunogenicity thus potentially providing a method of improved drug delivery. A PEGylated therapeutic agent may exhibit: (a) increased plasma circulatory half lives *in vivo* compared to the corresponding non-PEGylated compound, (b) enhanced therapeutic indices compared to the corresponding non-PEGylated compounds and (c) increased solubility compared to the corresponding non-PEGylated compounds, effecting possible improved drug delivery.

PEGylation is, according to non-limiting theory, a process by which oligosaccharides and synthetic polymers such as polyethylene glycol (PEG) are site-specifically and covalently attached to therapeutic protein target molecules. PEGylation can significantly enhance protein half-life by shielding the polypeptide from proteolytic enzymes and increasing the apparent size of the protein, thus reducing clearance rates. Moreover, PEG conjugates can enhance protein solubility and have beneficial effects on biodistribution. The physical and pharmacological properties of PEGylated proteins are affected by the number and the size of PEG chains attached to the polypeptide, the location of the PEG sites, and the chemistry used for PEGylation. Examples of PEG conjugation to proteins include reactions of N-hydroxysuccinimidyl ester derivatized PEGs with lysine, 1,4-addition reactions of maleimide and vinylsulfone derivatized PEGs with cysteine, and condensation of hydrazide containing PEGs with aldehydes generated by oxidation of glycoproteins. Details of compositions and methods for PEGylation are described herein and known in the art, for instance, in U.S. Patent No. 7,829,659.

Since PEG is water-soluble as well as soluble in many organic solvents, PEG is a useful polymer. PEG is generally non-toxic and non-immunogenic. When PEG is chemically attached to a water insoluble compound, the resulting conjugate generally becomes water soluble as well as soluble in many organic solvents. Thus, as used herein, a PEGylated polypeptide may have chemically attached to it a PEG moiety, which is intended to include but not be limited to, linear and branched PEG, methoxy PEG, hydrolytically or enzymatically degradable PEG, pendent PEG, dendrimer PEG, copolymers of PEG and one or more polyols, and copolymers of PEG and PLGA (poly(lactic/glycolic acid) of any weight and/or size.

Examples in which PEGylation has been used to effect drug delivery are disclosed, for example, in U.S. Pat. No. 6,623,729; U.S. Pat. No. 6,517,824; U.S. Pat. No. 6,515,017; U.S. Pat. No. 6,217,869; U.S. Pat. No. 6,191,105; U.S. Pat. No. 5,681,811; U.S. Pat. No. 5,455,027; U.S. Published Patent Application No. 20040018960; U.S. Published Patent Application No. 20030229010; U.S. Published Patent Application No. 20030229006; U.S. Published Patent Application No. 20030186869; U.S. Published Patent Application No. 20030026764; and U.S. Published Patent Application No. 20030017131. U.S. Pat. No. 6,214,966, U.S. Published Patent Application No. 2003000447, and U.S. Published Patent Application No. 2001021763 describe soluble, degradable poly(ethylene glycol) derivatives for controlled release of bound molecules into solution.

Reviews on PEGylation are provided in, for example, Greenwald et al., Adv. Drug Del. Rev. 2003, 55:217; Molineux, Pharmacotherapy 2003 (8 Pt 2): 3S-8S; Roberts et al., Adv. Drug Deliv. Rev. 2002, 54:459; Bhadra et al., Pharmazie 2002, 57:5, Greenwald, Controlled Release 2001, 74:159; Veronese et al., Farmaco. 1999, 54:497; and Zalipsky, Adv. Drug Deliv. Rev. 1995:16, 157. Methods for preparing PEGylated derivatives are described therein, and, for example, in Greenwald et al., J. Med. Chem. 1996, 39:424, and in U.S. Pat. No. 7,786,119. Alternate reaction steps would be readily recognized by one of skill in the art and include the reaction steps described, for example, in "Comprehensive Organic Transformations: A Guide to Functional Group Preparations", Richard C. Larock, Wiley-VCH: 1999, and in "March's Advanced Organic Chemistry: Reactions, Mechanisms and Structure", Jerry March & Michael Smith, John Wiley & Sons Inc: 2001.

Commercially available PEGs of many different types and molecular weights can be obtained, for instance, from Nektar Therapeutics, SunBio, Serva (Crescent Chemical Co.) and Fluka (Sigma-Aldrich), and include examples such as but not limited to activated PEG-NHS ester reagents and NHS PEG vinyl sulfone. These activated PEG reagent examples may be used to conjugate directly with the aminocycloalkyl ether compounds. Polypure AS, Norway, supplies monodisperse PEG and PEG derivatives comprised substantially of only one oligomer. Where appropriate, these monodisperse PEG and PEG derivatives may be advantageously utilized to form more well defined PEG derivatives.

As also described above, certain embodiments also relate to peptidomimetics, or "artificial" polypeptides. Such polypeptides may contain one or more amino acid insertions, deletions or substitutions, one or more altered or artificial peptide bond, one or more chemical moiety (such as polyethylene glycol, glycosylation, label, toxin, or other moiety), and/or one or more non-natural amino acid. Synthesis of peptidomimetics is well known in the art and may include altering naturally occurring proteins or polypeptides by chemical mutagenesis, single or multi-site-directed mutagenesis, PCR shuffling, use of altered aminoacyl tRNA or aminoacyl tRNA synthetase molecules, the use of "stop" codons such as amber suppressors, use of four or five base-pair codons, or other means.

POLYNUCLEOTIDES

Certain embodiments relate to nucleic acid molecules encoding a herein-described plasmin-inhibiting polypeptide. Methods for production of desired nucleic acids and/or polypeptides are well known in the art. For example, nucleic acids and/or polypeptides may be isolated from cells or synthesized *de novo* by chemical synthesis. Such nucleic acids or polypeptides may be incorporated into a vector, and transformed into a host cell. Host cells may be cultured in standard nutrient media plus necessary supplements or additives for inducing promoters, selecting transformants or amplifying the appropriate sequences.

In addition, encoding polynucleotides or polypeptide variants of a plasmin-inhibiting polypeptide may contain, respectively, one or more nucleotide or amino acid substitutions, additions, deletions, and/or insertions relative to a native (*e.g.* wildtype, or a predominant or naturally occurring allelic form). In some embodiments, a variant comprises a molecule in which the N-terminal L-amino acid is replaced with a D-amino acid, and in certain other embodiments one or more other amino acids (*e.g.,* not situated at the N-terminus) may, additionally or alternatively, be replaced with a D-amino acid. In certain embodiments, a variant comprises a molecule in which the N-terminal alpha amino acid is replaced with a beta or gamma amino acid. Variants preferably exhibit at least about 75%, 78%, 80%, 85%, 87%, 88% or 89% identity and more preferably at least about 90%, 91%, 92%, 93%, 94%, 95%, 96%, 97%, 98%, or 99% identity to a portion of a plasmin-inhibiting polypeptide sequence or of a polynucleotide sequence that encodes such a polypeptide. The percent identity may be readily determined by comparing sequences of the polypeptide or polynucleotide variants with the corresponding portion of a full-length polynucleotide or polypeptide. Some techniques for sequence comparison include using computer algorithms well known to those having ordinary skill in the art, such as Align or the BLAST algorithm (Altschul, J. Mol. Biol. 219:555-565, 1991; Henikoff and Henikoff, PNAS USA 89:10915-10919, 1992), which is available at the NCBI website (see [online] Internet:<URL: http://www/ncbi.nlm.nih.gov/cgi-bin/BLAST). Default parameters may be used.

The term "operably linked" means that the components to which the term is applied are in a relationship that allows them to carry out their inherent functions under suitable conditions. For example, a transcription control sequence "operably linked" to a protein coding sequence is ligated thereto so that expression of the protein coding sequence is achieved under conditions compatible with the transcriptional activity of the control sequences.

The term "control sequence" as used herein refers to polynucleotide sequences that can affect expression, processing or intracellular localization of coding sequences to which they are ligated or operably linked. The nature of such control sequences may depend upon the host organism. In particular embodiments, transcription control sequences for prokaryotes may include a promoter, ribosomal binding site, and transcription termination sequence. In other particular embodiments, transcription control sequences for eukaryotes may include promoters comprising one or a plurality of recognition sites for transcription factors, transcription enhancer sequences, transcription termination sequences and polyadenylation sequences. In certain embodiments, "control sequences" can include leader sequences and/or fusion partner sequences.

The term "polynucleotide" as referred to herein means single-stranded or double-stranded nucleic acid polymers. In certain embodiments, the nucleotides comprising the polynucleotide can be ribonucleotides or deoxyribonucleotides or a modified form of either type of nucleotide. Such modifications may include base modifications such as bromouridine, ribose modifications such as arabinoside and 2',3'-dideoxyribose and internucleotide linkage modifications such as phosphorothioate, phosphorodithioate, phosphoroselenoate, phosphorodiselenoate, phosphoroanilothioate, phoshoraniladate and phosphoroamidate. The term "polynucleotide" specifically includes single and double stranded forms of DNA.

The term "naturally occurring nucleotides" includes deoxyribonucleotides and ribonucleotides. The term "modified nucleotides" includes nucleotides with modified or substituted sugar groups and the like. The term "oligonucleotide linkages" includes oligonucleotide linkages such as phosphorothioate, phosphorodithioate, phosphoroselenoate, phosphorodiselenoate, phosphoroanilothioate, phoshoraniladate, phosphoroamidate, and the like. See, *e.g.,* LaPlanche et al., 1986, Nucl. Acids Res., 14:9081; Stec et al., 1984, J. Am. Chem. Soc., 106:6077; Stein et al., 1988, Nucl. Acids Res., 16:3209; Zon et al., 1991, Anti-Cancer Drug Design, 6:539; Zon et al., 1991, Oligonucleotides And Analogues: A Practical Approach, pp. 87-108 (F. Eckstein, Ed.), Oxford University Press, Oxford England; Stec et al., U.S. Pat. No. 5,151,510; Uhlmann and Peyman, 1990, Chemical Reviews, 90:543. An oligonucleotide can include a detectable label to enable detection of the oligonucleotide or hybridization thereof.

The term "vector" is used to refer to any molecule (*e.g.,* nucleic acid, plasmid, or virus) used to transfer coding information to a host cell. The term "expression vector" refers to a vector that is suitable for transformation of a host cell and contains nucleic acid sequences that direct and/or control expression of inserted heterologous nucleic acid sequences. Expression includes, but is not limited to, processes such as transcription, translation, and RNA splicing, if introns are present.

As will be understood by those skilled in the art, polynucleotides may include genomic sequences, extra-genomic and plasmid-encoded sequences and smaller engineered gene segments that express, or may be adapted to express, proteins, polypeptides, peptides and the like. Such segments may be naturally isolated, or modified synthetically by the skilled person.

As will be also recognized by the skilled artisan, polynucleotides may be single-stranded (coding or antisense) or double-stranded, and may be DNA (genomic, cDNA or synthetic) or RNA molecules. RNA molecules may include HnRNA molecules, which contain introns and correspond to a DNA molecule in a one-to-one manner, and mRNA molecules, which do not contain introns. Additional coding or non-coding sequences may, but need not, be present within a polynucleotide according to the present disclosure, and a polynucleotide may, but need not, be linked to other molecules and/or support materials. Polynucleotides may comprise a native sequence or may comprise a sequence that encodes a variant or derivative of such a sequence.

Therefore, according to these and related embodiments, the present disclosure also provides polynucleotides encoding the plasmin-inhibiting polypeptides described herein. In certain embodiments, polynucleotides are provided that comprise some or all of a polynucleotide sequence encoding a plasmin-inhibiting polypeptide as described herein, and complements of such polynucleotides.

In other related embodiments, polynucleotide variants may have substantial identity to a polynucleotide sequence encoding a plasmin-inhibiting polypeptide described herein. For example, a polynucleotide may be a polynucleotide comprising at least 70% sequence identity, preferably at least 75%, 80%, 85%, 90%, 95%, 96%, 97%, 98%, or 99% or higher, sequence identity compared to a reference polynucleotide sequence such as a sequence encoding a plasmin-inhibiting polypeptide having an amino acid sequence that is disclosed herein, using the methods described herein, (e.g., BLAST analysis using standard parameters, as described below). One skilled in this art will recognize that these values can be appropriately adjusted to determine corresponding identity of proteins encoded by two nucleotide sequences by taking into account codon degeneracy, amino acid similarity, reading frame positioning and the like.

Typically, polynucleotide variants will contain one or more substitutions, additions, deletions and/or insertions, preferably such that the binding affinity for plasmin of the plasmin-inhibiting polypeptide encoded by the variant polynucleotide is not substantially diminished relative to that of a plasmin-inhibiting polypeptide having an amino acid sequence that is specifically set forth herein.

In certain other related embodiments, polynucleotide fragments may comprise or consist essentially of various lengths of contiguous stretches of sequence identical to or complementary to a sequence encoding a plasmin-inhibiting polypeptide as described herein. For example, polynucleotides are provided that comprise or consist essentially of at least about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 55, 60, 65, 70, 75, 80, 85, 90, 95, 100, 110, 120, 130, 140, 150, 160, 170, 180, 190, 200, 220, 240, 260, 280, 300, 400, 500, 600, 700, 800, 900 or 1000 or more contiguous nucleotides of a sequence that encodes a plasmin-inhibiting polypeptide, or variant thereof, disclosed herein as well as all intermediate lengths therebetween. It will be readily understood that "intermediate lengths", in this context, means any length between the quoted values, such as 50, 51, 52, 53, *etc.*; 100, 101, 102, 103, *etc.*; 150, 151, 152, 153, *etc.*; including all integers through 200-500; 500-1,000, and the like. A polynucleotide sequence as described here may be extended at one or both ends by additional nucleotides not found in the native sequence. This additional sequence may consist of 1, 2, 3, 4, 5, 6, 7, 8, 9, 10, 11, 12, 13, 14, 15, 16, 17, 18, 19, or 20 nucleotides at either end of a polynucleotide encoding a plasmin-inhibiting polypeptide described herein or at both ends of a polynucleotide encoding a plasmin-inhibiting polypeptide described herein.

In another embodiment, polynucleotides are provided that are capable of hybridizing under moderate to high stringency conditions to a polynucleotide sequence encoding a plasmin-inhibiting polypeptide, or variant thereof as provided herein, or a fragment thereof, or a complementary sequence thereof. Hybridization techniques are well known in the art of molecular biology. For purposes of illustration, suitable moderately stringent conditions for testing the hybridization of a polynucleotide as provided herein with other polynucleotides include prewashing in a solution of 5 X SSC, 0.5% SDS, 1.0 mM EDTA (pH 8.0); hybridizing at 50°C-60°C, 5 X SSC, overnight; followed by washing twice at 65°C for 20 minutes with each of 2X, 0.5X and 0.2X SSC containing 0.1% SDS. One skilled in the art will understand that the stringency of hybridization can be readily manipulated, such as by altering the salt content of the hybridization solution and/or the temperature at which the hybridization is performed. For example, in another embodiment, suitable highly stringent hybridization conditions include those described above, with the exception that the temperature of hybridization is increased, *e.g.,* to 60°C-65°C or 65°C-70°C.

Determination of the three-dimensional structures of representative plasmin-inhibiting polypeptides (*e.g.,* a polypeptide of 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101-110, 111-120, 121-130, 131-140, 141-150, 151, 160, 161-170, 171-180, 181-190, or 191-200 amino acids), including the three-dimensional structures of such polypeptides when complexed with the plasmin proteinase domain, may be made through routine methodologies (*e.g.,* Chand et al., 2004 J. Biol. Chem. 17:17500; Bajaj, US 2009/0018069; Huber et al., 1974 J Mol Biol. 89:73-101; Schmidt et al., 2005 J Biol Chem. 280:27832-27838; Wang et al., 1998 Science 281, 1662-1665; Bajaj et al., 2001 Thromb. Haemost. 86, 959-972) such that substitution, addition, deletion or insertion of one or more amino acids with selected natural or non-natural amino acids can be virtually modeled for purposes of determining whether a so derived structural variant retains the space-filling properties of the presently disclosed plasmin-inhibiting species. A variety of computer programs are known to the skilled artisan for determining appropriate amino acid substitutions (or appropriate polynucleotides encoding the amino acid sequence) within a plasmin-inhibiting polypeptide such that, for example, affinity for the plasmin proteinase domain is maintained or better affinity is achieved.

The polynucleotides described herein, or fragments thereof, regardless of the length of the coding sequence itself, may be combined with other DNA sequences, such as promoters, polyadenylation signals, additional restriction enzyme sites, multiple cloning sites, other coding segments, and the like, such that their overall length may vary considerably. It is therefore contemplated that a nucleic acid fragment of almost any length may be employed, with the total length preferably being limited by the ease of preparation and use in the intended recombinant DNA protocol. For example, illustrative polynucleotide segments with total lengths of about 10,000, about 9000, about 8000, about 7000, about 6000, about 5000, about 4000, about 3000, about 2,000, about 1,000, about 900, about 800, about 700, about 600, about 500, about 400, about 300, about 200, about 190, about 180, about 170 base pairs in length, and the like, (including all intermediate lengths therebetween) are contemplated to be useful.

When comparing polynucleotide sequences, two sequences are said to be "identical" if the sequence of nucleotides in the two sequences is the same when aligned for maximum correspondence, as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, 40 to about 50, in which a sequence may be compared to a reference sequence of the same number of contiguous positions after the two sequences are optimally aligned.

Optimal alignment of sequences for comparison may be conducted using the Megalign™ program in the Lasergene™ suite of bioinformatics software (DNASTAR, Inc., Madison, WI), using default parameters. This program embodies several alignment schemes described in the following references: Dayhoff, M.O. (1978) A model of evolutionary change in proteins - Matrices for detecting distant relationships. In Dayhoff, M.O. (ed.) Atlas of Protein Sequence and Structure, National Biomedical Research Foundation, Washington DC Vol. 5, Suppl. 3, pp. 345-358; Hein J., Unified Approach to Alignment and Phylogenes, pp. 626-645 (1990); Methods in Enzymology vol. 183, Academic Press, Inc., San Diego, CA; Higgins, D.G. and Sharp, P.M., CABIOS 5:151-153 (1989); Myers, E.W. and Muller W., CABIOS 4:11-17 (1988); Robinson, E.D., Comb. Theor 11:105 (1971); Santou, N. Nes, M., Mol. Biol. Evol. 4:406-425 (1987); Sneath, P.H.A. and Sokal, R.R., Numerical Taxonomy - the Principles and Practice of Numerical Taxonomy, Freeman Press, San Francisco, CA (1973); Wilbur, W.J. and Lipman, D.J., Proc. Natl. Acad., Sci. USA 80:726-730 (1983).

Alternatively, optimal alignment of sequences for comparison may be conducted by the local identity algorithm of Smith and Waterman, Add. APL. Math 2:482 (1981), by the identity alignment algorithm of Needleman and Wunsch, J. Mol. Biol. 48:443 (1970), by the search for similarity methods of Pearson and Lipman, Proc. Natl. Acad. Sci. USA 85: 2444 (1988), by computerized implementations of these algorithms (GAP, BESTFIT, BLAST, FASTA, and TFASTA in the Wisconsin Genetics Software Package, Genetics Computer Group (GCG), 575 Science Dr., Madison, WI), or by inspection.

One preferred example of algorithms that are suitable for determining percent sequence identity and sequence similarity are the BLAST and BLAST 2.0 algorithms, which are described in Altschul et al., Nucl. Acids Res. 25:3389-3402 (1977), and Altschul et al., J. Mol. Biol. 215:403-410 (1990), respectively. BLAST and BLAST 2.0 can be used, for example with the parameters described herein, to determine percent sequence identity among two or more the polynucleotides. Software for performing BLAST analyses is publicly available through the National Center for Biotechnology Information. In one illustrative example, cumulative scores can be calculated using, for nucleotide sequences, the parameters M (reward score for a pair of matching residues; always >0) and N (penalty score for mismatching residues; always <0). Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLASTN program (for nucleotide sequences) uses as defaults a wordlength (W) of 11, and expectation (E) of 10, and the BLOSUM62 scoring matrix (see Henikoff and Henikoff, Proc. Natl. Acad. Sci. USA 89:10915 (1989)) alignments, (B) of 50, expectation (E) of 10, M=5, N=-4 and a comparison of both strands.

In certain embodiments, the "percentage of sequence identity" is determined by comparing two optimally aligned sequences over a window of comparison of at least 20 positions, wherein the portion of the polynucleotide sequence in the comparison window may comprise additions or deletions (i.e., gaps) of 20 percent or less, usually 5 to 15 percent, or 10 to 12 percent, as compared to the reference sequences (which does not comprise additions or deletions) for optimal alignment of the two sequences. The percentage is calculated by determining the number of positions at which the identical nucleic acid bases occurs in both sequences to yield the number of matched positions, dividing the number of matched positions by the total number of positions in the reference sequence (i.e., the window size) and multiplying the results by 100 to yield the percentage of sequence identity.

It will be appreciated by those of ordinary skill in the art that, as a result of the degeneracy of the genetic code, there are many nucleotide sequences that encode a plasmin-inhibiting polypeptide as described herein. Some of these polynucleotides bear minimal sequence identity to the nucleotide sequence of the original polynucleotide sequence that encodes a plasmin-inhibiting polypeptide having an amino acid sequence that is disclosed herein. Nonetheless, polynucleotides that vary due to differences in codon usage are expressly contemplated by the present disclosure. In certain embodiments, sequences that have been codon-optimized for mammalian expression are specifically contemplated.

Therefore, in another embodiment of the invention, a mutagenesis approach, such as site-specific mutagenesis, may be employed for the preparation of variants and/or derivatives of the plasmin-inhibiting polypeptides described herein. By this approach, specific modifications in a polypeptide sequence can be made through mutagenesis of the underlying polynucleotides that encode them. These techniques provides a straightforward approach to prepare and test sequence variants, for example, incorporating one or more of the foregoing considerations, by introducing one or more nucleotide sequence changes into the polynucleotide.

Site-specific mutagenesis allows the production of mutants through the use of specific oligonucleotide sequences which encode the DNA sequence of the desired mutation, as well as a sufficient number of adjacent nucleotides, to provide a primer sequence of sufficient size and sequence complexity to form a stable duplex on both sides of the deletion junction being traversed. Mutations may be employed in a selected polynucleotide sequence to improve, alter, decrease, modify, or otherwise change the properties of the polynucleotide itself, and/or alter the properties, activity, composition, stability, or primary sequence of the encoded polypeptide.

In certain embodiments, it is contemplated that the mutagenesis of the polynucleotide sequences that encode a plasmin-inhibiting polypeptide disclosed herein, or a variant thereof, to alter one or more properties of the encoded polypeptide, such as the plasmin-binding affinity of the peptide or the variant thereof, or the plasmin-inhibiting effects. The techniques of site-specific mutagenesis are well-known in the art, and are widely used to create variants of both polypeptides and polynucleotides. For example, site-specific mutagenesis is often used to alter a specific portion of a DNA molecule. In such embodiments, a primer comprising typically about 14 to about 25 nucleotides or so in length is employed, with about 5 to about 10 residues on both sides of the junction of the sequence being altered.

As will be appreciated by those of skill in the art, site-specific mutagenesis techniques have often employed a phage vector that exists in both a single stranded and double stranded form. Typical vectors useful in site-directed mutagenesis include vectors such as the M13 phage. These phage are readily commercially-available and their use is generally well-known to those skilled in the art. Double-stranded plasmids are also routinely employed in site directed mutagenesis that eliminates the step of transferring the gene of interest from a plasmid to a phage.

In general, site-directed mutagenesis in accordance herewith is performed by first obtaining a single-stranded vector or melting apart of two strands of a double-stranded vector that includes within its sequence a DNA sequence that encodes the desired peptide. An oligonucleotide primer bearing the desired mutated sequence is prepared, generally synthetically. This primer is then annealed with the single-stranded vector, and subjected to DNA polymerizing enzymes such as *E. coli* polymerase I Klenow fragment, in order to complete the synthesis of the mutation-bearing strand. Thus, a heteroduplex is formed wherein one strand encodes the original non-mutated sequence and the second strand bears the desired mutation. This heteroduplex vector is then used to transform appropriate cells, such as *E. coli* cells, and clones are selected which include recombinant vectors bearing the mutated sequence arrangement.

The preparation of sequence variants of the selected peptide-encoding DNA segments using site-directed mutagenesis provides a means of producing potentially useful species and is not meant to be limiting as there are other ways in which sequence variants of peptides and the DNA sequences encoding them may be obtained. For example, recombinant vectors encoding the desired peptide sequence may be treated with mutagenic agents, such as hydroxylamine, to obtain sequence variants. Specific details regarding these methods and protocols are found in the teachings of Maloy *et al.,* 1994; Segal, 1976; Prokop and Bajpai, 1991; Kuby, 1994; and Maniatis *et al.,* 1982.

As used herein, the term "oligonucleotide directed mutagenesis procedure" refers to template-dependent processes and vector-mediated propagation which result in an increase in the concentration of a specific nucleic acid molecule relative to its initial concentration, or in an increase in the concentration of a detectable signal, such as amplification. As used herein, the term "oligonucleotide directed mutagenesis procedure" is intended to refer to a process that involves the template-dependent extension of a primer molecule. The term template dependent process refers to nucleic acid synthesis of an RNA or a DNA molecule wherein the sequence of the newly synthesized strand of nucleic acid is dictated by the well-known rules of complementary base pairing (see, for example, Watson, 1987). Typically, vector mediated methodologies involve the introduction of the nucleic acid fragment into a DNA or RNA vector, the clonal amplification of the vector, and the recovery of the amplified nucleic acid fragment. Examples of such methodologies are provided by U. S. Patent No. 4,237,224.

In another approach for the production of polypeptide variants, recursive sequence recombination, as described in U.S. Patent No. 5,837,458, may be employed. In this approach, iterative cycles of recombination and screening or selection are performed to "evolve" individual polynucleotide variants having, for example, increased binding affinity for plasmin. Certain embodiments also provide constructs in the form of plasmids, vectors, transcription or expression cassettes which comprise at least one polynucleotide as described herein.

According to certain related embodiments there is provided a recombinant host cell which comprises one or more constructs as described herein; a nucleic acid encoding a plasmin-inhibiting polypeptide or variant thereof; and a method of producing of the encoded product, which method comprises expression from the encoding nucleic acid therefor. Expression may conveniently be achieved by culturing under appropriate conditions recombinant host cells containing the nucleic acid. Following production by expression, a plasmin-inhibiting polypeptide may be isolated and/or purified using any suitable technique, and then used as desired.

Systems for cloning and expression of a polypeptide in a variety of different host cells are well known. Suitable host cells include bacteria, mammalian cells, yeast and baculovirus systems. Mammalian cell lines available in the art for expression of a heterologous polypeptide include Chinese hamster ovary cells, HeLa cells, baby hamster kidney cells, NSO mouse melanoma cells and many others. A common, preferred bacterial host is *E. coli.*

The expression of peptides in prokaryotic cells such as *E. coli* is well established in the art. For a review, see for example Pluckthun, A. Bio/Technology 9: 545-551 (1991). Expression in eukaryotic cells in culture is also available to those skilled in the art as an option for production of recombinant polypeptides, see recent reviews, for example Ref, (1993) Curr. Opinion Biotech. 4: 573-576; Trill et al. (1995) Curr. Opinion Biotech 6: 553-560.

Suitable vectors can be chosen or constructed, containing appropriate regulatory sequences, including promoter sequences, terminator sequences, polyadenylation sequences, enhancer sequences, marker genes and other sequences as appropriate. Vectors may be plasmids, viral e.g. phage, or phagemid, as appropriate. For further details see, for example, Molecular Cloning: a Laboratory Manual: 2nd edition, Sambrook et al., 1989, Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in Current Protocols in Molecular Biology, Second Edition, Ausubel et al. eds., John Wiley & Sons, 1992, or subsequent updates thereto.

The term "host cell" is used to refer to a cell into which has been introduced, a nucleic acid sequence encoding one or more of the herein described immunomodulatory polypeptides, and which further expresses or is capable of expressing a selected gene of interest, such as a gene encoding any herein described plasmin-inhibiting polypeptide. The term includes the progeny of the parent cell, whether or not the progeny are identical in morphology or in genetic make-up to the original parent, so long as the selected gene is present. Accordingly there is also contemplated a method comprising introducing such nucleic acid into a host cell. The introduction may employ any available technique. For eukaryotic cells, suitable techniques may include calcium phosphate transfection, DEAE-Dextran, electroporation, liposome-mediated transfection and transduction using retrovirus or other virus, *e.g.* vaccinia or, for insect cells, baculovirus. For bacterial cells, suitable techniques may include calcium chloride transformation, electroporation and transfection using bacteriophage. The introduction may be followed by causing or allowing expression from the nucleic acid, *e.g.* by culturing host cells under conditions for expression of the gene. In one embodiment, the nucleic acid is integrated into the genome (*e.g.* chromosome) of the host cell. Integration may be promoted by inclusion of sequences which promote recombination with the genome, in accordance with standard techniques.

The present invention also provides, in certain embodiments, a method which comprises using a construct as stated above in an expression system in order to express a particular polypeptide such as a plasmin-inhibiting polypeptide as described herein. The term "transduction" is used to refer to the transfer of genes from one bacterium to another, usually by a phage. "Transduction" also refers to the acquisition and transfer of eukaryotic cellular sequences by retroviruses. The term "transfection" is used to refer to the uptake of foreign or exogenous DNA by a cell, and a cell has been "transfected" when the exogenous DNA has been introduced inside the cell membrane. A number of transfection techniques are well known in the art and are disclosed herein. See, *e.g.,* Graham et al., 1973, Virology 52:456; Sambrook et al., 2001, Molecular Cloning, A Laboratory Manual, Cold Spring Harbor Laboratories; Davis et al., 1986, Basic Methods In Molecular Biology, Elsevier; and Chu et al., 1981, Gene 13:197. Such techniques can be used to introduce one or more exogenous DNA moieties into suitable host cells.

The term "transformation" as used herein refers to a change in a cell's genetic characteristics, and a cell has been transformed when it has been modified to contain a new DNA. For example, a cell is transformed where it is genetically modified from its native state. Following transfection or transduction, the transforming DNA may recombine with that of the cell by physically integrating into a chromosome of the cell, or may be maintained transiently as an episomal element without being replicated, or may replicate independently as a plasmid. A cell is considered to have been stably transformed when the DNA is replicated with the division of the cell. The term "naturally occurring" or "native" when used in connection with biological materials such as nucleic acid molecules, polypeptides, host cells, and the like, refers to materials which are found in nature and are not manipulated by a human. Similarly, "non-naturally occurring" or "non-native" as used herein refers to a material that is not found in nature or that has been structurally modified or synthesized by a human.

Certain embodiments also contemplate transgenic animals or transgenic plants comprising one or more nucleic acid molecule(s) such as polynucleotides encoding one or more of the herein described plasmin-inhibiting polypeptides, that may be used to generate "knock-out" animals or that may be used to produce plasmin-inhibiting polypeptides. Some examples of transgenic animals that may be used include goats, cows, horses, pigs, rats, mice, rabbits, hamsters, or other animals. The transgenic animals may be chimeric, non-chimeric heterozygotes, or non-chimeric homozygotes. (See, for example, Hogan et al., Manipulating the Mouse Embryo: A Laboratory Manual 2 ed., Cold Spring Harbor Press (1999); Jackson et al., Mouse Genetics and Transgenics: A Practical Approach, Oxford Univ. Press (2000); Pinkert, Transgenic Animal Technology: A Laboratory Handbook, Academic Press (1999).)

CANCER

As also noted above, certain presently disclosed embodiments are directed to a method for preventing or attenuating cancer progression or blocking metastasis in a subject known to have, or suspected of being at risk for having, a cancer characterized by deleterious or otherwise inappropriate proteolysis that is a direct or indirect consequence of undesirable plasmin activity and/or plasmin overexpression, such as a number of solid and/or malignant tumors, for instance lung cancer, breast cancer, prostate cancer or colon cancer. Certain illustrative examples are described herein with reference to particular types of cancer, but the invention is not intended to be so limited and may also find uses in the treatment of other cancers that are characterized by harmful plasmin activity.

The presence of cancer or of a malignant condition in a subject refers to the presence of dysplastic, cancerous and/or transformed cells in the subject, including, for example neoplastic, tumor, non-contact inhibited or oncogenically transformed cells, or the like (*e.g.,* lung, breast, colon or prostate cancer, or endometrial or ovarian cancer, or carcinomas such as adenocarcinoma, renal cell carcinoma, squamous cell carcinoma, small cell carcinoma, oat cell carcinoma, etc., sarcomas such as chondrosarcoma, osteosarcoma, etc., melanoma) which are known to the art and for which criteria for diagnosis and classification are established, as are signs and symptoms and/or risk factors according to which an individual may be identified as having, or as suspected of being at risk for having (or progressing to) a particular type of cancer or malignant condition (*e.g.,* Roulston, J.E. and Bartlett, J.M.S. (Eds.), Molecular Diagnosis of Cancer: Methods and Protocols (2nd Ed.), 2004, Humana Press, Totowa, NJ; Hayat, M.A. (Ed.), Cancer Imaging, 2007, Academic Press, NY; Skarin, Atlas of Diagnostic Oncology, 2002, Mosby/Elsevier, Philadelphia, PA; Nakamura, R.M. et al., Cancer Diagnostics, 2004, Humana Press, Totowa, NJ; *etc.*). These and related criteria are thus known and may further include determination of cancer progression and of metastasis or metastatic disease, *i.e.,* the spread of one or a plurality of cancer cells from an initial or primary tumor site in a tissue or organ, to one or more distinct secondary sites where tumor cells lodge and proliferate to form secondary tumors having deleterious clinical consequences for the subject.

In certain embodiments contemplated by the present disclosure, for example, such cancer cells are neoplastically transformed epithelial cells such as carcinoma cells. Certain preferred embodiments contemplate application of the compositions and methods described herein for the treatment of a cancer that is characterized by occurrence in tissues in which excessive plasmin activity and/or overexpression is present.

For example, cancers that form solid tumors may be classified according to recognized stages in the progression of disease, such as by being graded according to the Tumor-Node-Metastasis (TNM) staging system (*e.g.,* Chang et al., 2008 CA Cancer J Clin 58:54-59). As is presently practiced in the art, early stages of cancer represent early steps in the progression of disease wherein current clinical practices include so-called "watchful waiting" as a management strategy. In "watchful waiting", periodic assessment of disease progression is made but no major invasive procedures (*e.g.,* surgery) are undertaken so long as the cancer remains confined by surrounding epithelium without disrupting the underlying basement membrane.

Similar staging paradigms are established for ovarian cancer progression, for instance, that of the International Federation of Gynecology and Obstetrics (FIGO) according to which, generally, epithelial ovarian cancer stages I (occurrence of cancer cells limited to intraovarian sites), II (intraovarian plus pelvic cancer cells), III (occurrence of cancer cells limited to intraovarian, pelvic, and abdominal or proximate regional sites), and IV (as III plus involvement of other (distal) organs as cancer sites), and recurrent ovarian cancer, have been classified (TNM Classification of Malignant Tumours, Sixth Edition, UICC, 2002). Endometrial cancer progression has also been classified by stages in like fashion (see, e.g., NIH/NCI PDQ® cancer information database, National Cancer Institute, Frederick, MD): stage I (confined to occurrence of cancer cells within uterus only), II (cancer detected in uterus and cervix), III (cancer cells beyond uterus and cervix but restricted to pelvic region), and IV (cancer detected beyond pelvis, including bladder, bowel, abdominal or groin lymph nodes or beyond).

Renal cell carcinoma progression has also been defined according to the TNM staging system (American Joint Committee on Cancer (AJCC), TNM Classification of Malignant Tumours, Sixth Edition, UICC, 2002). Hence, stage I renal cell carcinoma involves intrarenal tumors of no more than seven centimetres in diameter, stage II involves intrarenal tumors in excess of seven centimetres, stage III involves confinement of tumors to renal (Gerota's) fascia and up to one lymph node in the vicinity of the affected kidney, and in stage IV renal carcinoma cancer cells have invaded tissues beyond the renal fascia as evidenced by cancer in more than one lymph node in the vicinity of the affected kidney, or in at least one distal site.

Because many of the sequelae of surgical intervention to remove cancerous tissue containing invasive and/or metastatic cancer cells include undesirable but frequently unavoidable side-effects, it may be desirable according to certain herein disclosed embodiments to prevent or attenuate cancer progression or to block metastasis, by treatments that prevent progression of a cancer to invasive and/or metastatic stages, *i.e.,* to maintain the "watchful waiting" status. Accordingly and without wishing to be bound by theory, certain herein disclosed embodiments relate to maintenance of cancer in a subject at such a pre-invasive, pre-metastatic stage, by administering one or more of the herein disclosed plasmin-inhibiting polypeptides in a manner that interferes with cancer progression and thereby precludes the need for surgery.

In related embodiments there is thus provided a method of inhibiting plasmin activity, comprising administering a therapeutically effective amount of a plasmin-inhibiting polypeptide as provided herein under conditions and for a time sufficient for inhibition of plasmin activity. In these and related embodiments it may further be contemplated to determine inhibition of the proteolytic activity of plasmin by the plasmin-inhibiting polypeptide, for example, by detecting a level of enzymatic cleavage by plasmin of a detectable serine protease substrate (*e.g.,* a chromogenic substrate) in the absence of the plasmin-inhibiting polypeptide that differs (with statistical significance) from the level of enzymatic cleavage by the plasmin of the detectable serine protease substrate (*e.g.,* a chromogenic substrate) in the presence of the plasmin-inhibiting polypeptide.

PROTECTING PROTEINS FROM PROTEOLYTIC DEGRADATION

Certain expressly contemplated embodiments of the present disclosure are directed to the use of any one or more of the herein described plasmin-inhibiting polypeptides to protect proteins from proteolytic degradation by protein-degrading enzymes such as proteases or proteinases, which in particular embodiments may be serine proteases. Accordingly there are provided a large number of methods for inhibiting or substantially reducing proteolytic degradation (*e.g.,* decreasing in a statistically significant manner relative to proteolytic degradation that is detectable when the inhibitor is not present) by including in any of a variety of known or contemplated processes one or more of the present plasmin-inhibiting polypeptides. Persons familiar with the art will be aware of a wide variety of proteins and protein products that will be beneficially protected from undesirable degradation by proteases. Such persons will also be aware of a wide range of known enzymatic, biochemical and other assays for determining whether proteolysis is present, and for determining further whether any one or more of the present plasmin-inhibiting polypeptides may be effective at inhibiting or substantially reducing such proteolysis.

Examples of such beneficial uses of the present plasmin-inhibiting polypeptides include their inclusion, for preventing proteolysis, in cell cultures in which expression of desired protein products is taking place; during purification of proteins from natural sources including biological samples as well as from cell cultures; and in milk of transgenic animals used for expression of proteins that may otherwise be degradable by plasmin, trypsin and other proteases. These and related embodiments will thus find application in research, manufacturing, industrial and other contexts.

ACTIVE SITE INACTIVATED PLASMIN TO PURIFY PLASMIN-INHIBITING POLYPEPTIDES HAVING CARBOXY-TERMINAL LYSINE(S)

As described herein, a number of the present plasmin-inhibiting polypeptides comprise a KD1 carboxy-region polypeptide sequence that has the amino acid lysine at the carboxy terminus. Without wishing to be bound by theory and in view of plasmin-binding, plasmin inhibition and inhibitor-plasmin interaction properties of the present plasmin-inhibiting polypeptides described herein, it is believed that carboxy-terminal lysine residues in these polypeptides contribute to the functional activities of the plasmin-inhibiting polypeptides. In particular, and further according to non-limiting theory, carboxy-terminal lysine appears to permit or promote binding of the plasmin-inhibiting polypeptide to plasmin via a lysine-binding site that is spatially and functionally distinct from the plasmin enzymatic active site. In this way the carboxy-terminal lysine residue that is present in certain of the herein described plasmin-inhibiting polypeptide confers a distinct plasmin-binding property on the plasmin-inhibiting polypeptide, which binding may also interfere with plasmin binding to fibrin. The dual reactivity of the present plasmin-inhibiting polypeptides may thus be manifest in part as direct inhibition of the plasmin active site via the altered amino acid residues in the herein described polypeptides (*e.g.,* SEQ ID NOS:3-18, 22-29 and 32-35) that correspond to positions 11 and 17 in the unaltered TFPI-2 KD1 sequence of SEQ ID NO:19, and also in part as binding of the plasmin-inhibiting polypeptide to the lysine binding sites on plasminogen/plasmin via C-terminal lysine.

Accordingly, the present disclosure envisions embodiments in which the binding affinity of the herein described plasmin-inhibiting polypeptides for plasmin, and in particular embodiments such binding affinity that derives from carboxy-terminal lysine interaction with the lysine binding site on plasmin, may be exploited by using plasminogen or inactivated plasmin as an immobilized affinity ligand with which to isolate KD1-containing polypeptides that have carboxy-terminal lysine.

For example, plasminogen may be isolated from plasma obtained from outdated blood (or fresh blood) using a lysine-Sepharose column according to standard accepted procedures, wherein plasma is passed over a lysine-Sepharose column and plasminogen is captured via its Kringle domains. Impurities from the plasma that bind non-specifically to the column are removed by washing with a high-salt buffer, and the bound plasminogen is then specifically eluted with a buffer containing lysine as a competitive affinity ligand. The eluted plasminogen is then made free of lysine (*e.g.,* by dialysis or ultrafiltration or other suitable means) and optionally is activated to convert it to plasmin, the active site of which can then be blocked by treatment with a known chemical inhibitor such as a chloromethylketone inhibitor (*e.g.,* D-Val-Phe-Lys chloromethylketone). The plasminogen, or blocked plasmin, is then coupled to a solid phase matrix such as Sepharose or other suitable carrier by standard methods, for instance, following cyanogen bromide activation of the Separharose. The plasminogen/plasmin Sepharose column can then be used to capture wild-type KD1-containing polypeptides having carboxy-terminal lysine, or any of the herein described KD1-containing plasmin-inhibiting polypeptides having carboxy-terminal lysine (*e.g.,* SEQ ID NOS:3-18, 22-29 and 32-35), by affinity interactions. The bound KD1-containing polypeptide is then competitively eluted with a buffer containing lysine, which disrupts the interaction between the carboxy-terminal lysine of the KD1-containing polypeptide and the lysine-binding site of plasmin. The KD1-containing polypeptides are then concentrated, and free lysine is removed from the preparation by standard procedures such as a desalting column or dialysis. By this method or a related variation that will be apparent to the skilled person based on the present disclosure, there can be obtained wild-type KD1-containing polypeptides having carboxy-terminal lysine, or any of the herein described KD1-containing plasmin-inhibiting polypeptides having carboxy-terminal lysine (*e.g.,* SEQ ID NOS:3-18, 22-29 and 32-35), which can then be used as desired, for example, according to any of the methods described herein.

Pharmaceutical Compositions and Administration

The present invention also relates in certain embodiments to pharmaceutical compositions containing the plasmin-inhibiting polypeptides that are disclosed herein. In one embodiment, the pharmaceutical composition comprises a plasmin-inhibiting polypeptide in a pharmaceutically acceptable excipient, carrier or diluent and in an amount effective to prevent or attenuate cancer progression or block metastasis, when administered to an animal, preferably a mammal, most preferably a human. In other embodiments, the pharmaceutical composition comprises a plasmin-inhibiting polypeptide in a pharmaceutically acceptable excipient, carrier or diluent and in an amount effective to treat a subject in need of inhibition of a plasmin activity, for instance, in a method comprising administering to the subject an effective amount of a plasmin-inhibiting polypeptide disclosed herein.

Examples of diseases, disorders, and treatments relating to the need of inhibition of plasmin include, but are not limited to, tumorogenesis, angiogenesis, bone remodeling, surgery, hemophilia, orthopedic surgery, coronary artery bypass grafting (CABG), and systemic inflammatory response syndrome (SIRS). Also disclosed is a method of treating rheumatoid arthritis in a subject in need thereof, comprising administering to the subject an effective amount of a plasmin-inhibiting polypeptide disclosed herein. Also contemplated are uses of pharmaceutical compositions comprising the herein described plasmin-inhibiting polypeptides to control bleeding in other contexts, for instance, as antifibrinolytic compositions and as antidotes to plasmin overdoses or to overdoses of tPA or other hematologically active substances that may directly or indirectly promote the activity of plasmin or other relevant proteases.

Administration of the plasmin-inhibiting polypeptide in pure form or in an appropriate pharmaceutical composition, can be carried out via any of the accepted modes of administration of agents for serving similar utilities. The pharmaceutical composition can be prepared by combining a plasmin-inhibiting polypeptide with an appropriate pharmaceutically acceptable carrier, diluent or excipient, and may be formulated into preparations in solid, semi-solid, liquid or gaseous forms, such as tablets, capsules, powders, granules, ointments, solutions, suppositories, injections, inhalants, gels, microspheres, and aerosols. Typical routes of administering such pharmaceutical compositions include, without limitation, oral, topical, transdermal, inhalation, parenteral, sublingual, rectal, vaginal, intranasal, intraperitoneal, intravenous, intraarterial, transdermal, sublingual, subcutaneous, intramuscular, rectal, transbuccal, intranasal, liposomal, via inhalation, intraoccular, via catheter (*e.g.,* as in angioplasty), via local delivery, subcutaneous, intraadiposal, intraarticularly or intrathecally. The term parenteral as used herein includes subcutaneous injections, intravenous, intramuscular, intrasternal injection or infusion techniques. Pharmaceutical compositions are formulated so as to allow the active ingredients contained therein to be bioavailable upon administration of the composition to a patient. Compositions that will be administered to a subject or patient take the form of one or more dosage units, where for example, a tablet may be a single dosage unit, and a container of a compound of the invention in aerosol form may hold a plurality of dosage units. Actual methods of preparing such dosage forms are known, or will be apparent, to those skilled in this art; for example, see The Science and Practice of Pharmacy, 20th Edition (Philadelphia College of Pharmacy and Science, 2000). The composition to be administered will, in any event, contain a therapeutically effective amount of a plasmin-inhibiting polypeptide for treatment of a disease or condition of interest in accordance with the present teachings.

The pharmaceutical compositions useful herein also contain a pharmaceutically acceptable carrier, including any suitable diluent or excipient, which includes any pharmaceutical agent that does not itself induce the production of antibodies harmful to the individual receiving the composition, and which may be administered without undue toxicity.

Pharmaceutically acceptable carriers include, but are not limited to, liquids, such as water, saline, glycerol and ethanol, and the like. A thorough discussion of pharmaceutically acceptable carriers, diluents, and other excipients is presented in Remington's Pharmaceutical Sciences (Mack Pub. Co., N.J. current edition).

A pharmaceutical composition may be in the form of a solid or liquid. In one aspect, the carrier(s) are particulate, so that the compositions are, for example, in tablet or powder form. The carrier(s) may be liquid, with the compositions being, for example, an oral syrup, injectable liquid or an aerosol, which is useful in, for example, inhalatory administration. When intended for oral administration, the pharmaceutical composition is preferably in either solid or liquid form, where semi-solid, semi-liquid, suspension and gel forms are included within the forms considered herein as either solid or liquid.

As a solid composition for oral administration, the pharmaceutical composition may be formulated into a powder, granule, compressed tablet, pill, capsule, chewing gum, wafer or the like form. Such a solid composition will typically contain one or more inert diluents or edible carriers. In addition, one or more of the following may be present: binders such as carboxymethylcellulose, ethyl cellulose, microcrystalline cellulose, gum tragacanth or gelatin; excipients such as starch, lactose or dextrins, disintegrating agents such as alginic acid, sodium alginate, Primogel, corn starch and the like; lubricants such as magnesium stearate or Sterotex; glidants such as colloidal silicon dioxide; sweetening agents such as sucrose or saccharin; a flavoring agent such as peppermint, methyl salicylate or orange flavoring; and a coloring agent.

When the pharmaceutical composition is in the form of a capsule, for example, a gelatin capsule, it may contain, in addition to materials of the above type, a liquid carrier such as polyethylene glycol or oil.

The pharmaceutical composition may be in the form of a liquid, for example, an elixir, syrup, solution, emulsion or suspension. The liquid may be for oral administration or for delivery by injection, as two examples. When intended for oral administration, preferred composition contain, in addition to the present compounds, one or more of a sweetening agent, preservatives, dye/colorant and flavor enhancer. In a composition intended to be administered by injection, one or more of a surfactant, preservative, wetting agent, dispersing agent, suspending agent, buffer, stabilizer and isotonic agent may be included.

The liquid pharmaceutical compositions, whether they be solutions, suspensions or other like form, may include one or more of the following adjuvants: sterile diluents such as water for injection, saline solution, preferably physiological saline, Ringer's solution, isotonic sodium chloride, fixed oils such as synthetic mono or diglycerides which may serve as the solvent or suspending medium, polyethylene glycols, glycerin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes or multiple dose vials made of glass or plastic. Physiological saline is a preferred adjuvant. An injectable pharmaceutical composition is preferably sterile.

A liquid pharmaceutical composition intended for either parenteral or oral administration should contain an amount of a plasmin-inhibiting polypeptide such that a suitable dosage will be obtained. Typically, this amount is at least 0.01% of a plasmin-inhibiting polypeptide in the composition. When intended for oral administration, this amount may be varied to be between 0.1 and about 70% of the weight of the composition. Preferred oral pharmaceutical compositions contain between about 4% and about 50% of the plasmin-inhibiting polypeptide. Preferred pharmaceutical compositions and preparations according to the present invention are prepared so that a parenteral dosage unit contains between 0.01 to 10% by weight of the plasmin-inhibiting polypeptide.

The pharmaceutical composition may be intended for topical administration, in which case the carrier may suitably comprise a solution, emulsion, ointment or gel base. The base, for example, may comprise one or more of the following: petrolatum, lanolin, polyethylene glycols, bee wax, mineral oil, diluents such as water and alcohol, and emulsifiers and stabilizers. Thickening agents may be present in a pharmaceutical composition for topical administration. If intended for transdermal administration, the composition may include a transdermal patch or iontophoresis device. Topical formulations may contain a concentration of the plasmin-inhibiting polypeptide from about 0.1 to about 10% w/v (weight per unit volume).

In particular, certain embodiments contemplate formulation of the herein disclosed plasmin-inhibiting polypeptide into a patch to promote hemostasis such as a fibrinogen patch or a fibrinogen-containing patch. Such patches may find uses in any of a number of described applications, for example, at major wound sites. A variety of topical and other hemostatic patches are known (e.g., Erdogan et al., 2008 J. Biomed. Mat. Res. B Appl. Biomat. 85:272; Spotnitz et al., 2012 Transfusion 52:2243; Schuetz et al., 2004 Ann. Thorac. Surg. 78:569; van de Walle et al., 2012 Acta. Biomater. 8:4080), and as such, those skilled in the art can readily envision incorporation of the presently disclosed plasmin-inhibiting polypeptide into such patches based on the teachings herein.

The present pharmaceutical composition may in other embodiments be intended for rectal administration, in the form, for example, of a suppository, which will melt in the rectum and release the plasmin-inhibiting polypeptide. The composition for rectal administration may contain an oleaginous base as a suitable nonirritating excipient. Such bases include, without limitation, lanolin, cocoa butter and polyethylene glycol.

The present pharmaceutical composition which comprises a plasmin-inhibiting polypeptide as provided herein may also include various materials, which modify the physical form of a solid or liquid dosage unit. For example, the composition may include materials that form a coating shell around the active ingredients. The materials that form the coating shell are typically inert, and may be selected from, for example, sugar, shellac, and other enteric coating agents. Alternatively, the active ingredients may be encased in a gelatin capsule. The pharmaceutical composition in solid or liquid form may include an agent that binds to the plasmin-inhibiting polypeptide and thereby assists in the delivery of the polypeptide. Suitable agents that may act in this capacity include a monoclonal or polyclonal antibody, a protein or a liposome.

The pharmaceutical composition may consist of dosage units that can be administered as an aerosol. The term aerosol is used to denote a variety of systems ranging from those of colloidal nature to systems consisting of pressurized packages. Delivery may be by a liquefied or compressed gas or by a suitable pump system that dispenses the active ingredients. Aerosols may be delivered in single phase, bi-phasic, or tri-phasic systems in order to deliver the active ingredient(s). Delivery of the aerosol includes the necessary container, activators, valves, subcontainers, and the like, which together may form a kit. One skilled in the art, without undue experimentation may determine preferred aerosols.

The present pharmaceutical compositions may be prepared by methodology well known in the pharmaceutical art. For example, a pharmaceutical composition intended to be administered by injection can be prepared by combining a plasmin-inhibiting polypeptide with sterile, distilled water and optionally suitable stabilizing agents, solutes and/or osmolytes so as to form a solution. A surfactant may be added to facilitate the formation of a homogeneous solution or suspension. Surfactants are compounds that non-covalently interact with the plasmin-inhibiting polypeptide so as to facilitate dissolution or homogeneous suspension of the polypeptide in the aqueous delivery system.

The plasmin-inhibiting polypeptide is administered in a therapeutically effective amount, which will vary depending upon a variety of factors including the activity of the specific polypeptide; the metabolic stability and length of action of plasmin-inhibiting polypeptide; the age, body weight, general health, sex, and diet of the patient; the mode and time of administration; the rate of excretion; the drug combination; the severity of the particular disorder or condition; and the subject undergoing therapy. Generally, a therapeutically effective daily dose is (for a 70 Kg mammal) from about 0.001 mg/Kg (*i.e.,* 0.07 mg) to about 100 mg/Kg (*i.e.,* 7.0 g); preferaby a therapeutically effective dose is (for a 70 Kg mammal) from about 0.01 mg/Kg (*i.e.,* 0.7 mg) to about 50 mg/Kg (*i.e.,* 3.5 g); more preferably a therapeutically effective dose is (for a 70 Kg mammal) from about 1 mg/kg (*i.e.,* 70 mg) to about 25 mg/Kg (*i.e.,* 1.75 g).

The ranges of effective doses provided herein are not intended to be limiting and represent preferred dose ranges. However, the most preferred dosage will be tailored to the individual subject, as is understood and determinable by one skilled in the relevant arts. (see, *e.g.*, Berkow et al., eds., The Merck Manual, 16th edition, Merck and Co., Rahway, N.J., 1992; Goodman et al., eds.,Goodman and Gilman's The Pharmacological Basis of Therapeutics, 10th edition, Pergamon Press, Inc., Elmsford, N.Y., (2001); Avery's Drug Treatment: Principles and Practice of Clinical Pharmacology and Therapeutics, 3rd edition, ADIS Press, LTD., Williams and Wilkins, Baltimore, MD. (1987), Ebadi, Pharmacology, Little, Brown and Co., Boston, (1985); Osol et al., eds., Remington's Pharmaceutical Sciences, 18th edition, Mack Publishing Co., Easton, PA (1990); Katzung, Basic and Clinical Pharmacology, Appleton and Lange, Norwalk, CT (1992)).

The total dose required for each treatment can be administered by multiple doses or in a single dose over the course of the day, if desired. Generally, treatment is initiated with smaller dosages, which are less than the optimum dose of the plasmin-inhibiting polypeptide. Thereafter, the dosage is increased by small increments until the optimum effect under the circumstances is reached. The plasmin-inhibiting polypeptide can be administered alone or in conjunction with other diagnostics and/or pharmaceuticals directed to the pathology, or directed to other symptoms of the pathology. The recipients of administration of the plasmin-inhibiting polypeptide can be any vertebrate animal, such as mammals. Among mammals, the preferred recipients are mammals of the Orders Primate (including humans, apes and monkeys), Arteriodactyla (including horses, goats, cows, sheep, pigs), Rodenta (including mice, rats, rabbits, and hamsters), and Carnivora (including cats, and dogs). Among birds, the preferred recipients are turkeys, chickens and other members of the same order. The most preferred recipients are humans.

For topical applications, it is preferred to administer an effective amount of the present pharmaceutical composition containing a plasmin-inhibiting polypeptide to target area, *e.g.,* skin surfaces, mucous membranes, and the like, which are adjacent to those tissues which are to be treated. This amount will generally range from about 0.0001 mg to about 1 g of a plasmin-inhibiting polypeptide per application, depending upon the area to be treated, whether the use is diagnostic, prophylactic or therapeutic, the severity of the symptoms, and the nature of the topical vehicle employed. A preferred topical preparation is an ointment, wherein about 0.001 to about 50 mg of active ingredient is used per cc of ointment base. The pharmaceutical composition can be formulated as transdermal compositions or transdermal delivery devices ("patches"). Such compositions include, for example, a backing, active compound reservoir, a control membrane, liner and contact adhesive. Such transdermal patches may be used to provide continuous pulsatile, or on demand delivery of the present plasmin-inhibiting polypeptide as desired.

The plasmin-inhibiting polypeptide can be formulated so as to provide quick, sustained or delayed release of the active ingredient after administration to the patient by employing procedures known in the art. Controlled release drug delivery systems include osmotic pump systems and dissolutional systems containing polymer-coated reservoirs or drug-polymer matrix formulations. Examples of controlled release systems are given in U.S. Pat. Nos. 3,845,770 and 4,326,525 and in P. J. Kuzma et al., Regional Anesthesia 22 (6): 543-551 (1997).

The plasmin-inhibiting polypeptide can also be delivered through intra-nasal drug delivery systems for local, systemic, and nose-to-brain medical therapies. Controlled Particle Dispersion (CPD)™ technology, traditional nasal spray bottles, inhalers or nebulizers are known by those skilled in the art to provide effective local and systemic delivery of drugs by targeting the olfactory region and paranasal sinuses.

Certain embodiments also relate to an intravaginal shell or core drug delivery device suitable for administration to the human or animal female. The device may be comprised of the active pharmaceutical ingredient in a polymer matrix, surrounded by a sheath, and capable of releasing the plasmin-inhibiting polypeptide in a substantially zero order pattern on a daily basis similar to devices used to apply testosterone as described in PCT Published Patent No. WO 98/50016.

Current methods for ocular delivery include topical administration (eye drops), subconjunctival injections, periocular injections, intravitreal injections, surgical implants and iontophoresis (uses a small electrical current to transport ionized drugs into and through body tissues). Those skilled in the art would combine the best suited excipients with the plasmin-inhibiting polypeptide for safe and effective intra-ocular administration.

The most suitable route will depend on the nature and severity of the condition being treated. Those skilled in the art are also familiar with determining administration methods (*e.g.* oral, intravenous, inhalation, subcutaneous, rectal, etc.), dosage forms, suitable pharmaceutical excipients and other matters relevant to the delivery of the plasmin-inhibiting polypeptide to a subject in need of inhibition of a plasmin activity.

According to various contemplated embodiments the subject in need of inhibition of a plasmin activity may have or be suspected of being at risk for having cancer (*e.g.,* a solid tumor such as lung, breast, prostate or colon cancer, or another cancer), hemophilia, rheumatoid arthritis or systemic inflammatory response syndrome (SIRS), or the subject may be in need of or may have undergone angiogenesis, bone remodeling or coronary artery bypass grafting (CABG). The subject may be undergoing surgery or may have recently (*e.g.,* within 1, 2, 4, 6, 8, 10, 12 or 24 hours, or within 1, 2, 3, 4, 5, 6, 7, 8, 9 or 10 days) undergone surgery, for example, cardiovascular surgery, oncological surgery, genitourinary surgery, orthopedic surgery, thoracic surgery, plastic surgery, trauma surgery, abdominal surgery, transplant surgery, neurologic surgery or otolaryngological surgery.

Persons skilled in the relevant arts will be familiar with any number of diagnostic, surgical and other clinical criteria to which can be adapted administration of the pharmaceutical compositions described herein. See, *e.g.*, Humar et al., Atlas of Organ Transplantation, 2006, Springer; Kuo et al., Comprehensive Atlas of Transplantation, 2004 Lippincott, Williams & Wilkins; Gruessner et al., Living Donor Organ Transplantation, 2007 McGraw-Hill Professional; Antin et al., Manual of Stem Cell and Bone Marrow Transplantation, 2009 Cambridge University Press; Wingard et al. (Ed.), Hematopoietic Stem Cell Transplantation: A Handbook for Clinicians, 2009 American Association of Blood Banks; Sabiston, Textbook of Surgery, 2012 Saunders & Co.; Mulholland, Greenfield's Surgery, 2010 Lippincott, Williams & Wilkins; Schwartz's Principles of Surgery, 2009 McGraw-Hill; Lawrence, Essentials of General Surgery 2012 Lippincott, Williams & Wilkins.

It will be appreciated that the practice of the several embodiments of the present invention will employ, unless indicated specifically to the contrary, conventional methods in virology, immunology, microbiology, molecular biology and recombinant DNA techniques that are within the skill of the art, and many of which are described below for the purpose of illustration. Such techniques are explained fully in the literature. See, *e.g.,* Current Protocols in Molecular Biology or Current Protocols in Immunology, John Wiley & Sons, New York, N.Y.(2009); Ausubel et al., Short Protocols in Molecular Biology, 3rd ed., Wiley & Sons, 1995; Sambrook and Russell, Molecular Cloning: A Laboratory Manual (3rd Edition, 2001); Maniatis et al. Molecular Cloning: A Laboratory Manual (1982); DNA Cloning: A Practical Approach, vol. I & II (D. Glover, ed.); Oligonucleotide Synthesis (N. Gait, ed., 1984); Nucleic Acid Hybridization (B. Hames & S. Higgins, eds., 1985); Transcription and Translation (B. Hames & S. Higgins, eds., 1984); Animal Cell Culture (R. Freshney, ed., 1986); Perbal, A Practical Guide to Molecular Cloning (1984) and other like references.

Standard techniques may be used for recombinant DNA, oligonucleotide synthesis, and tissue culture and transformation (*e.g.,* electroporation, lipofection). Enzymatic reactions and purification techniques may be performed according to manufacturer's specifications or as commonly accomplished in the art or as described herein. These and related techniques and procedures may be generally performed according to conventional methods well known in the art and as described in various general and more specific references that are cited and discussed throughout the present specification. Unless specific definitions are provided, the nomenclature utilized in connection with, and the laboratory procedures and techniques of, molecular biology, analytical chemistry, synthetic organic chemistry, and medicinal and pharmaceutical chemistry described herein are those well known and commonly used in the art. Standard techniques may be used for recombinant technology, molecular biological, microbiological, chemical syntheses, chemical analyses, pharmaceutical preparation, formulation, and delivery, and treatment of patients.

As used in this specification and the appended claims, the singular forms "a," "an" and "the" include plural references unless the content clearly dictates otherwise. Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers. Each embodiment in this specification is to be applied *mutatis mutandis* to every other embodiment unless expressly stated otherwise.

Equivalents

While particular steps, elements, embodiments and applications of the present invention have been shown and described herein for purposes of illustration, it will be understood, of course, that the invention is not limited thereto since modifications may be made by persons skilled in the art, particularly in light of the foregoing teachings, without deviating from the spirit and scope of the invention. Accordingly, the invention is not limited except as by the appended claims.

The following Examples are presented by way of illustration and not limitation.

EXAMPLES

EXAMPLE 1

INHIBITORY ACTIVITIES OF PLASMIN-INHIBITING POLYPEPTIDES

Plasmin-inhibiting polypeptides according to the present disclosure were engineered by site-directed mutagenesis of coding sequences for the human tissue factor pathway inhibitor-2 first Kunitz domain (KD1), expressed in *E. coli* using recombinant techniques, and purified essentially according to established methodologies (Bajaj et al., 2011 J. Biol. Chem. 286:4329), except that the present plasmin-inhibiting polypeptide sequences are disclosed herein for the first time. This example describes characterization of plasmin-inhibiting polypeptides having the amino acid sequences set forth in SEQ ID NOS:3 and 5.

Plasmin inhibition by the plasmin-inhibiting polypeptides was tested in an assay for plasmin activity as described (Bajaj et al., 2011 J. Biol. Chem. 286:4329) using human plasmin (Hematologic Technologies, Essex Junction, VT), H-D-Val-Leu-Lys-p-nitroanilide (S-2251, Diapharma, West Chester, OH) as a plasmin substrate, and varying concentrations of the indicated plasmin-inhibiting polypeptide. Figure 1 shows inhibition of plasmin activity by a plasmin-inhibiting polypeptide comprising the amino acid sequence set forth in SEQ ID NO:5. The calculated inhibition constant ($K_i$) was 2.36 nM. Inhibition of plasmin activity by a plasmin-inhibiting polypeptide comprising the amino acid sequence set forth in SEQ ID NO:3 is shown in Figure 2. The calculated inhibition constant ($K_i$) was 0.49 nM.

The plasmin-inhibiting polypeptide comprising SEQ ID NO:3 was also tested at varying concentrations for inhibition of factor VIIa-tissue factor amidolytic activity in an assay using soluble human tissue factor (100 nM) and factor VIIa (50 nM) the chromogenic substrate S-2288 (H-D-Ile-Pro-Arg-*p*-nitroanilide, Diapharma) essentially as described (Chand et al., 2004 J. Biol. Chem. 279:17500; Bajaj et al., 2011 J. Biol. Chem. 286:4329). Figure 3 shows the effect on factor VIIa-tissue factor amidolytic activity of the plasmin-inhibiting polypepide comprising the amino acid sequence set forth in SEQ ID NO:3. The calculated inhibition constant ($K_i$) was 5869.5 nM.

Figure 4 shows the effects of the plasmin-inhibiting polypepide comprising the amino acid sequence set forth in SEQ ID NO:3 on assays for inhibition of Factor XIa amidolytic activity (closed circles) and kallikrein amidolytic activity (open circles). These assays were performed according to the procedures described in Bajaj et al., 2011 J. Biol. Chem. 286:4329.

The effects on clotting time in a plasma clot fibrinolysis assay were also tested, according to the procedures described in Bajaj et al., 2011 J. Biol. Chem. 286:4329, but using varying concentrations of the herein described plasmin-inhibiting polypeptide comprising the amino acid sequence set forth in SEQ ID NO:5 (SM), and of the herein described plasmin-inhibiting polypeptide comprising the amino acid sequence set forth in SEQ ID NO:3 (DM), and of aprotinin (BPTI). Typical results are shown in Figure 5.

EXAMPLE 2

PLASMIN-INHIBITORY ACIVITIES OF PLASMIN-INHIBITING POLYPEPTIDES

In a separate set of experiments, purified recombinantly produced plasmin-inhibiting polypeptides comprising the amino acid sequences set forth in SEQ ID NOS:3, 4, 5 and 6 were tested for their respective abilities to inhibit human plasmin activity *in vitro* using H-D-valyl-leucyl-lysine p-nitroaniline dihydrochloride (S2251, Chromogenix/Diapharma) as the substrate. Aprotinin was used as a positive control. Plasmin inhibitors were serially diluted in Tris-buffered saline (TBS) and distributed into wells of a 96-well plate to final concentrations ranging from 0.1 nM to 60 nM. Plasmin was added to yield a final concentration of 1.2 nM in each reaction. The wells were incubated for one hour at room temperature, S2251 was then added to each well to achieve a final concentration of 300 $\mu$M, and the plate was incubated for an additional hour at 37°C. Plasmin activity was calculated based on the rate of change of optical density at 405 nm due to release of p-nitroaniline from the substrate. Data were then fit to a 4-parameter logistic curve to determine the apparent $IC_{50}$. Two separate experiments were performed and separate determinations of plasmin inhibition activity were conducted for each sample. The results are shown in Table 1:

| Plasmin-Inhibiting Polypeptide | $IC_{50}$ (nM) |
| --- | --- |
| I. | |
| SEQ ID NO:3 | 1.8 |
| SEQ ID NO:5 | 3.4 |
| Aprotinin (positive control) | 0.48 |
| II. | |
| SEQ ID NO:4 | 0.80 |
| SEQ ID NO:6 | 1.8 |
| Aprotinin | 0.57 |

EXAMPLE 3

DIRECT BINDING OF PLASMIN-INHIBITING POLYPEPTIDES TO PLASMIN

Surface plasmon resonance (SPR) experiments were performed to characterize binding interactions between immobilized plasmin and each of the herein described plasmin-inhibiting polypeptides comprising SEQ ID NOS:3, 5 and 6. The BiaCore™ SPR instrument (GE Healthcare) was used for these assays according to the manufacturer's instructions. The active site of human plasmin was blocked with a chloromethylketone inhibitor (*e.g.,* D-Val-Phe-Lys chloromethylketone dihydrochloride, EMD Biosciences, La Jolla, CA) and the active site-blocked plasmin was immobilized on a BiaCore™ CM5 chip.

Figure 6 shows binding by plasmin-inhibiting polypeptides at varying concentrations (1, 2, 4, 8 and 10 $\mu$M for SEQ ID NOS:3 and 5; 1, 2, 4, 8, 10 and 12 $\mu$M for SEQ ID NO:6) to immobilized active site-blocked plasmin in the surface plasmon resonance assay. The top panel (60A) shows binding to plasmin of the plasmin-inhibiting polypeptide comprising the amino acid sequence set forth in SEQ ID NO:5. The affinity binding constant was determined by SPR to be 130 nM. The middle panel (60B) shows binding to plasmin of the plasmin-inhibiting polypeptide comprising the amino acid sequence set forth in SEQ ID NO:3. The affinity binding constant was determined by SPR to be 313 nM. The bottom panel (63-residue) shows binding to plasmin of the plasmin-inhibiting polypeptide comprising the amino acid sequence set forth in SEQ ID NO:6. The affinity binding constant was determined by SPR to be 290 nM. Aprotinin did not bind to active site-blocked plasmin in the surface plasmon resonance assay. All three plasmin-inhibiting polypeptides bound to plasmin with 4-8 fold higher affinity than tranexamic acid or $\varepsilon$-amino caproic acid.

EXAMPLE 4

MODELED BINDING OF PLASMIN-INHIBITING POLYPEPTIDES TO PLASMIN

Interaction between the human plasmin Kringle1 domain and the 63-amino acid plasmin-inhibiting polypeptide having the amino acid sequence set forth in SEQ ID NO:4 was also modeled three dimensionally according to described methodology (Bajaj et al., 2011 J. Biol. Chem. 286:4329). In SEQ ID NO:4 the KD1 carboxy-region polypeptide (amino acids 58-63) has the sequence I-X3-K-V-X4-K as set forth in SEQ ID NO:2, in which X3 is E and X4 is P. The side chain of the lysine residue at position 60 in SEQ ID NO:4 was observed to point outward and did not interfere with or contribute to binding interactions between plasmin and the plasmin-inhibiting polypeptide. The lysine residue at position 63 in SEQ ID NO:4 is expected to be cleaved *in vivo* by activated TAFI (thrombin activated fibrinolysis inhibitor) which thus results in the 62 amino acid plasmin-inhibiting polypeptide of SEQ ID NO:25 having the KD1 carboxy-region polypeptide (amino acids 58-62) I-X3-K-V-X5 as set forth in SEQ ID NO:20, in which X3 is E and X5 is P to give the C-terminal sequence IEKVP (SEQ ID NO: 40).

Interaction between the human plasmin Kringle1 domain and the 60-amino acid plasmin-inhibiting polypeptide having the amino acid sequence set forth in SEQ ID NO:3 was also modeled three dimensionally according to described methodology (Bajaj et al., 2011 J. Biol. Chem. 286:4329). In SEQ ID NO:3 the KD1 carboxy-region polypeptide (amino acids 58-60) has the sequence I-X3-K- in which X3 is E. In this binding interaction the side chain of the lysine residue at position 60 in SEQ ID NO:3 was observed to fit into the lysine binding site (LSB) of the human plasmin kringle domain. These interactions are similar to those observed when plasmin interaction was modeled using the 62-amino acid plasmin-inhibiting polypeptide of SEQ ID NO:25 in which the KD1 carboxy-region polypeptide (amino acids 58-62) has the sequence IEKVP (SEQ ID NO: 40; SEQ ID NO:20 in which X3 is E and X5 is P).

By way of non-limiting theory, removal by TAFI of C-terminal K60 from the 60-amino acid plasmin-inhibiting polypeptide of SEQ ID NO:3 is believed to result in a molecule that has no ability to bind to the LSB in the human plasmin kringle domain. By contrast, and as also noted above, if one starts instead with the 63-amino acid plasmin-inhibiting polypeptide of SEQ ID NO:4 having $IEK_{60}VPK63$ as the KD1 carboxy-region polypeptide, removal of K63 by TAFI would still result in a 62-amino acid plasmin-inhibiting polypeptide (SEQ ID NO:25) that retains the ability of KD1 (with C-terminal IEKVP62) to bind to the LSB in plasmin. This 62-amino acid plasmin-inhibiting polypeptide (SEQ ID NO:25) is not a substrate for TAFI and is therefore not subject to lose its potential to bind to the LSB in plasmin.

The three-dimensional modeling studies revealed that, relative to the wild type first Kunitz-type proteinase inhibitor domain (KD1) of human tissue factor pathway inhibitor-2 (TFPI-2; SEQ ID NO:19), the substitution of tyrosine by threonine at position 11 (*e.g.,* Y11T, as in SEQ ID NOS:3, 4, 11, 15, 24, 25, 32, 33) in addition to the substitution of leucine by arginine at position 17 (L17R), allows hydrogen bond formation between the plasmin-inhibiting polypeptide and plasmin that strengthens the interactions between plasmin and the plasmin-inhibiting polypeptide, relative to the interactions that are permitted when only the L17R substitution has been made (*e.g.,* as in SEQ ID NOS:5, 6, 12, 23).

Accordingly and in view of the foregoing, both the 60-amino acid (*e.g.,* SEQ ID NO:5) and the 63-amino acid plasmin-inhibiting polypeptides containing the L17R substitution (*e.g.,* SEQ ID NO:6) have activity as inhibitors of the plasmin active site and bind to the lysine binding site(s) in the Kringle domain(s), providing two modes of inhibitory activity of plasmin. Thus, both forms possess dual activity. Further, the 63-amino acid form retains its ability to interact with the lysine binding site following removal of the C-terminal K63 by activated TAFI *in vivo,* because the K60 remains in the resulting 62-amino acid form and interacts with the lysine binding site. The 60-amino acid and 63-amino acid plasmin inhibiting polypeptides containing both the substitution at position 11 (*e.g.,* SEQ ID NO:3, for instance, Y11T) and the substitution at position 17 (*e.g.,* SEQ ID NO:3, for instance L17R) are even more potent plasmin inhibitors and are dually reactive by virtue of binding to the lysine binding sites on plasminogen/plasmin via C-terminal lysine.

EXAMPLE 5

DECOY PLASMINOGEN RECEPTOR CONTAINING A SELECTIVE KUNITZ-INHIBITORY DOMAIN

This example describes a Kunitz domain 1 (KD1) of tissue factor pathway inhibitor-2, in which P2' residue Leu17 (bovine pancreatic trypsin inhibitor numbering) is mutated to Arg, and which selectively inhibits the active-site of plasmin with ~5-fold improved affinity. Thrombin cleavage (24-hour extended incubation at a 1:50 enzyme to substrate ratio) of the KD1 mutant (Leu17Arg) yielded a smaller molecule containing the intact Kunitz domain with no detectable change in the active-site inhibitory function. $NH_2$-terminal sequencing and MALDI-TOF/ESI data revealed that the starting molecule had C-terminal valine ($KD1_{L17R}$-$V_T$), whereas the smaller molecule had C-terminal lysine ($KD1_{L17R}$-$K_T$). $KD1_{L17R}$-$K_T$ having C-terminal lysine was examined for potential activity as a decoy receptor for plasminogen/plasmin, including inhibition of plasminogen activation as well as the active-site of generated plasmin.

In surface plasmon resonance experiments, tissue plasminogen activator (tPA) and Glu-plasminogen bound to $KD1_{L17R}$-$K_T$ ($K_d$ ~0.2-0.3 $\mu$M) but not to $KD1_{L17R}$-$V_T$. Further, $KD1_{L17R}$-$K_T$ inhibited tPA-induced plasma clot fibrinolysis more efficiently than $KD1_{L17R}$-$V_T$. Additionally, compared to $\varepsilon$-aminocaproic acid, $KD1_{L17R}$-$K_T$ was more effective in reducing blood loss in a mouse liver laceration injury model, in which the fibrinolytic system is activated. The micro($\mu$)-plasmin-$KD1_{L17R}$-$K_T$ complex inhibited urokinase-induced plasminogen activation on phorbol-*12*-myristate-13-acetate stimulated U937 monocyte-like cells, whereas the $\mu$-plasmin-$KD1_{L17R}$-$V_T$ complex failed to inhibit this process.

$KD1_{L17R}$-$K_T$ inhibited the active-site of plasmin and was a decoy receptor for the kringle domain(s) of plasminogen/plasmin; hence, it was capable of limiting both plasmin generation and plasmin activity. $KD1_{L17R}$-$K_T$ with dual function may thus in certain preferred embodiments be a preferred agent for controlling plasminogen activation in pathological processes.

Abbreviations used in Example 5 are: BPTI, bovine pancreatic trypsin inhibitor; BSA, bovine serum albumin; $\varepsilon$ACA, $\varepsilon$-aminocaproic acid; ECM, extracellular matrix; FXIa, factor XIa; Glu-Plg, Glu-plasminogen; HBS/BSA, pH 7.5, 50 mM Hepes containing 100 mM NaCl, 0.1 mg/ml BSA, pH 7.5; HBS-P, 20 mM Hepes containing 100 mM NaCl, 0.005% (v/v) P20, pH 7.4; IIa, thrombin; IPTG, isopropyl thiogalactopyranoside; KD1, Kunitz domain1; MMP, matrix metalloproteases; NPP, normal pooled plasma; PAI-1, plasminogen activator inhibitor-1; PAI-2, plasminogen activator inhibitor-2; PMA, 12-0-tetradecanoylphorbol-13-acetate; S-2251, H-D-Val-Leu-Lys-*p*-nitroanilide; S-2366, pyroGlu-Pro-Arg-*p*-nitroanilide; SPR, Surface Plasmon Resonance; TBS, pH 7.5, 50 mM Tris-HCl containing 100 mM NaCl, pH 7.5; TBS/BSA, TBS containing 0.1 mg/ml BSA; TE, tranexamic acid; TFPI-2, Tissue factor pathway inhibitor-2; tPA, tissue plasminogen activator; pKLK, plasma kallikrein; uPA, urokinase plasminogen activator.

*KD1 and BPTI residue numbering.* KD1 and BPTI residues P2, P1, P1', P2' are numbered according to Schechter and Berger.[65] The corresponding subsites are S2, S1, S1', S2' in the enzyme, respectively. The residue numbering corresponds to BPTI such that residue 15 is at P1 position and residue 17 is at P2' position. For comparison, KD1 is numbered using the BPTI numbering system such that residues 15 and 17 in BPTI correspond to residues 24 and 26 in KD1, respectively. Thus, the residues in KD1 differ by nine from BPTI.

*KD1$_{L17R}$-V$_T$ designation.* KD1$_{L17R}$-V$_T$ contains residues 1-73 of human TFPI-2; it also has four additional residues (Gly-Ser-His-Met) at the N-terminus that were introduced during cloning. It has C-terminal residue valine.[66]

*KD1$_{L17R}$-K$_T$ designation.* Approximately 80% of the KD1$_{L17R}$-K$_T$ contains residues 8-72 and the remaining 20% has residues 10 (residue 1 in BPTI)-72 of human TFPI-2; it has C-terminal residue lysine. Here, these two species are collectively referred to as KD1$_{L17R}$-K$_T$.

The chymotrypsin amino acid numbering system is used for the protease domain of plasmin and IIa. Residue 195 in chymotrypsin numbering corresponds to 741 in plasmin and 525 in IIa. Similarly, residue 189 in chymotrypsin numbering corresponds to 735 in plasmin and 519 in IIa.

By way of a further brief background, plasmin is a multifunctional proteolytic enzyme (MW 92,000) that circulates in blood as a single chain inactive zymogen, plasminogen.[1] Although plasminogen is expressed ubiquitously in the body,[2] hepatocytes represent the predominant site of its synthesis.[3,4] Circulating plasminogen has NH$_2$-terminal glutamic acid (Glu-Plg), and contains a Pan-apple domain, five kringle domains and the carboxy-terminal latent serine protease domain.[5] Native plasminogen exists in a tight conformation in which Pan-apple domain is bound to the kringle domain 5.[6,7] Upon cleavage of the Arg561-Val562 scissile bond, single-chain plasminogen is converted to a disulfide-linked two-chain active protease plasmin.[8,9] The heavy chain of Glu-plasmin consists of the Pan-apple domain, and five kringle domains, whereas the light chain contains the C-terminal protease domain.[9] Plasminogen can be activated by either urokinase plasminogen activator (uPA) or tissue plasminogen activator (tPA).[9-11] Through self catalysis, Lys-plasmin (and Lys-plasminogen) is formed that lacks the Pan-apple domain.[5,8] Due to absence of interaction(s) between the Pan-apple domain and the kringle 5 domain, Lys-plasminogen exists in a more open conformation than the Glu-Plg.[12,13] Lys-plasminogen is more readily activated by plasminogen activators as compared to Glu-Plg.[14,15] Plasminogen activators, tPA and uPA, are inhibited by plasminogen activator inhibitor-1 (PAI-1)[16] and PAI-2;[17,18] whereas plasmin is inhibited by $\alpha_2$-antiplasmin[19] and $\alpha_2$-macroglobulin.[20]

Plasmin plays an important role in fibrinolysis and is responsible for clot lysis at the site of thrombus formation.[9] During fibrinolysis, plasminogen is primarily activated by tPA, released from the damaged endothelium.[21,22] Starting at the N-terminus, tPA consists of a finger domain, an epidermal growth factor-like domain, two kringle domains and a C-terminal protease domain.[23,24] The finger domain and the second kringle domain of tPA bind to the C-terminal lysine residues exposed in the thrombus clot.[25,26] Plasminogen is also localized to the fibrin clot via its kringle domains 1 and 4, where tPA locally converts it to plasmin.[27] During degradation of fibrin, plasmin generates additional C-terminal lysine residues, which enhances tPA and plasminogen/plasmin binding to the clot for efficient lysis.[28] Further, as compared to the circulating plasmin, fibrin bound plasmin is poorly inhibited by $\alpha_2$-antiplasmin.[21] Thus, such localized mechanism of fibrinolysis prevents degradation of circulating fibrinogen.

In severe trauma[29] and during major surgical procedures, such as cardiac surgery, the fibrinolytic system is hyperactivated.[30,31] In trauma, uncontrolled bleeding is the leading cause of preventable death.[32] Antifibrinolytic agents when used prophylactically can significantly reduce blood loss and the need for extensive blood transfusions.[30,33,34] Aprotinin (bovine pancreatic trypsin inhibitor, BPTI), a potent inhibitor of the plasmin active site, had been the leading antifibrinolytic agent used to prevent blood loss in cardiovascular bypass surgery.[35] However, because of its side effects such as kidney damage, myocardial infarction and anaphylactic potential, it has been removed from the clinical market.[36,37,38] The presently used antifibrinolytic agents are tranexamic acid (TE) and ε-aminocaproic acid (εACA);[39] although both of these lysine analogs are less effective than aprotinin and are also associated with kidney failure and seizures.[39,40] Thus a molecule with superior efficacy is desirable for use an antifibrinolytic agent.

The plasminogen system also plays an essential role in pericellular proteolysis-dependent degradation of extracellular matrix (ECM)[41-46] and activation of cytokines.[47] Plasmin activates several pro-matrix metalloproteases (proMMPs) including proMMPs 3, 9, 12 and 13[48,49] that degrade other matrix components such as collagens.[50,51] Further, plasmin plays a significant role in angiogenesis by releasing certain matrix-associated growth factors such as fibroblast growth factor and vascular endothelial growth factor,[52,53] and in the activation of latent transforming growth factor-β.[54] Thus, plasmin is involved in inflammation, wound healing, cell migration, tumor growth and apoptosis.[9] All of these functions are attributed to uPA (in association with uPA receptor) mediated activation of plasminogen bound to the cell surface receptors present on monocytes/macrophages, smooth muscle cells, endothelial and epithelial cells, keratinocytes, and fibroblasts as well as platelets.[55] Plasminogen receptors with C-terminal lysine residues are abundantly expressed by different cell types[56-58] and invasive properties of tumor cells are dependent on plasmin-mediated proteolysis of ECM.[59,60] Thus, attenuating plasminogen activation by preventing its binding to the cell surface receptors and inhibiting the formed plasmin is expected to be useful for controlling these pathological processes.

Tissue factor pathway inhibitor-2 (TFPI-2), also known as placental protein 5 or matrix serine protease inhibitor, contains three Kunitz-type inhibitory domains in tandem.[61-63] Kunitz domain1 (KD1) of TFPI-2 is the only inhibitory domain and it inhibits plasmin, factor (F) XIa, plasma kallikrein (pKLK) and FVIIa/tissue factor.[64] Using serine protease subsite[a] S2'/P2' profiling,[65] a variant KD1-L17R molecule was designed that is a much more potent inhibitor of plasmin; further, it does not inhibit pKLK or FXIa.[66] The *Escherichia coli* expressed KD1-L17R[66] has C-terminal valine and has nine residues at the N-terminus that are not part of the Kunitz domain; it is termed here as $KD1_{L17R}$-$V_T$.[b]

Described herein according to certain embodiments is a smaller KD1 molecular species that has C-terminal lysine and lacks the first 7-9 residues; it is referred to herein as $KD1_{L17R}$-$K_T$.[c] Structural and functional characterization of $KD1_{L17R}$-$K_T$ including its inhibition of plasmin and binding to the kringle domains of plasminogen are disclosed here. $KD1_{L17R}$-$K_T$ with its dual function may thus according to certain herein disclosed embodiments be a preferred agent for controlling plasmin generation and its inhibition in pathological processes.

## EXPERIMENTAL PROCEDURES

**Materials.** *Escherichia coli* strain BL21(DE3) pLysS and pET28a expression vector were obtained from Novagen Inc. (Madison, WI). Amicon® centrifugal filter devices (3000 Mr cutoff) were purchased from Millipore (Bedford, MA). Q-Sepharose® FF, SP-Sepharose® FF, Superdex®-200, and His-Trap™ HP columns were obtained from Amersham Biosciences. Kanamycin, isopropyl thiogalactopyranoside (IPTG), and 12-0-tetradecanoylphorbol-13-acetate (PMA) were obtained from Sigma (St. Louis, MO). Human α-thrombin (IIa), FXIa, pKLK and plasmin were purchased from Haematologic Technologies Inc. tPA (Alteplase™) was purchased from Genentech (South San Francisco, CA). uPA was obtained from Calbiochem, EMD Biosciences Inc. (San Diego, CA). Glu-Plg was obtained from Enzyme Research Laboratories (South Bend, IN). εACA (Amicar™) was obtained from ICN Biomedicals Inc. (Aurora, Ohio). Normal pooled plasma (NPP) was purchased from George King Bio-Medical Inc. (Overland Park, Kansas). Plasmin substrate S-2251 (H-D-Val-Leu-Lys-p-nitroanilide), pKLK and FXIa substrate S-2366 (pyroGlu-Pro-Arg-p-nitroanilide) were obtained from Diapharma Inc. All other reagents were of the highest purity commercially available.

**Expression and Purification of $KD1_{L17R}$-$V_T$.** The coding sequence for residues 1-73 of human TFPI-2 containing the KD1 cDNA sequence was cloned and overexpressed as an amino-terminal His[6]-tagged fusion protein in *E. coli* strain BL21(DE3) pLysS using the T7 promoter system.[66-69] The point mutant $KD1_{L17R}$-$V_T$ was generated using the QuikChange® site-directed mutagenesis kit (Stratagene, La Jolla CA). The His[6]-tagged $KD1_{L17R}$-$V_T$ was expressed in *E. coli* grown in Luria broth containing 15 mg/liter kanamycin, and induced at 37 °C with 1 mM IPTG. The His[6]-tagged $KD1_{L17R}$-$V_T$ was purified from the inclusion bodies and refolded as described.[66,68-70]

Purified His[6]-tagged $KD1_{L17R}$-$V_T$ was digested with IIa at a 1:1000 enzyme/substrate molar ratio for 2 hours at 37 °C. Complete digestion of KD1 by IIa was confirmed by SDS-PAGE analysis of temporal aliquots.[69] His[6]-tag free protein was separated from the His[6]-tag and IIa using Superdex™-200 gel filtration chromatography equilibrated with 50 mM Tris-HCl, pH 7.5 containing 100 mM NaCl (TBS).[66] The preparation was characterized with respect to protein concentration using an extinction coefficient ($A_{280}$) of 2.45 at 1 mg/ml, purity (SDS-PAGE), N-terminal sequence and mass spectrometry prior to use in biochemical experiments.

**Preparation and Isolation of $KD1_{L17R}$-$K_T$.** Incubation of $KD1_{L17R}$-$V_T$ (1 mg/ml, in TBS at pH 7.5) with IIa at a 1:50 enzyme/substrate molar ratio for 24 hours at 37 °C resulted in a smaller $KD1_{L17R}$-$K_T$ molecular species. The $KD1_{L17R}$-$K_T$ was separated from IIa using Superdex™-200 gel filtration chromatography equilibrated with TBS, pH 7.5.[66] Purified $KD1_{L17R}$-$K_T$ was characterized with respect to protein concentration using an extinction coefficient ($A_{280}$) of 2.84 at 1 mg/ml, purity (SDS-PAGE), N-terminal sequence and mass spectrometry prior to use in biochemical experiments.

**SDS-PAGE.** SDS-PAGE was performed using the Laemmli buffer system.[71] The acrylamide concentration used was between 10% and 15%, and the gels were stained with Coomassie Brilliant Blue dye.

**Amino Acid Sequence Analysis and Mass Spectrometry.** For N-terminal sequence analysis, proteins were transferred to a polyvinylidene difluoride (PVDF) membrane from the reduced SDS-PAGE. Automated N-terminal protein sequencing was performed using a pulsed-liquid solid phase sequenator with on-line phenylthiohydantoin (PTH)-amino acid analysis (Applied Biosystems cLC system) at the UCLA Biopolymer Core facility (UCLA, Los Angeles, CA). Mass spectrometry (MS) data were collected by matrix-assisted laser desorption ionization (MALDI)-MS and electrospray ionization (ESI)-MS. The protein solution was desalted and concentrated prior to MS measurements using reversed-phase media packed into pipet tips (C4-resin ZipTip™, Millipore, Billerica, MA USA). A Voyager™ DE-STR MALDI time-of-flight mass spectrometer (Applied Biosystems, Foster City, CA USA) operating in the linear mode was used for the MALDI-MS measurements. The protein sample solution (0.5 μL) was spotted onto a stainless steel sample plate followed by 0.5 μL of the MALDI matrix solution, α-cyano-4-hydroxycinnamic acid (CHCA). The spotted samples were allowed to dry at room temperature prior to data acquisition. ESI-MS data were collected using a nanospray source and a solariX™ hybrid 15-Tesla Fourier transform ion cyclotron resonance (FT-ICR) mass spectrometer (Bruker Daltonics, Billerica, MA, USA).

**KD1$_{L17R}$-K$_T$ binding to tPA and Glu-Plg using surface plasmon resonance (SPR).** Binding studies were performed on a Biacore® T100 flow biosensor (Biacore, Uppsala, Sweden) at 25 °C. Glu-Plg (-98% purity using SDS-PAGE) or tPA (>98% purity using SDS-PAGE) was immobilized on carboxymethyl-dextran flow cell (CM5 sensor chips, GE Healthcare) using amine-coupling chemistry. Flow cell surfaces were activated with a mixture of 1-ethyl-3-(3-dimethylaminopropyl)carbodiimide and *N*-hydroxysulfosuccinimide for 5 min (flow rate 10 μl/min), after which the protein (20 μg/ml in 10 mM sodium acetate, pH 5.5) was injected onto the surface. Unreacted sites were blocked for 5 min with 1 M ethanolamine. Each analyte (KD1$_{L17R}$-V$_T$ and KD1$_{L17R}$-K$_T$, 100 to 2000 nM) was perfused through flow cells in HBS-P buffer (20 mM HEPES, pH 7.4, 100 mM NaCl, 0.005% (v/v) P20) at 10 μl/min for 6 min. After changing to HBS-P buffer without the protein, analyte dissociation was monitored for 10 min. Flow cells were regenerated with HBS-P containing 20 mM εACA. Data were corrected for nonspecific binding by subtracting signals obtained with the analyte infused through a flow cell without the coupled protein. Binding was analyzed with BIAevaluation™ software (Biacore) using a 1:1 binding model. $K_d$ values were calculated from the quotient of the derived dissociation ($k_d$) and association ($k_a$) rate constants.

**Protease Inhibition Assay.** All reactions were carried out in TBS, pH 7.5 containing 0.1 mg BSA/ml (TBS/BSA) and 2 mM Ca$^{2+}$ (TBS/BSA/Ca$^{2+}$, pH 7.5) as described.[66,68] Each enzyme (plasmin, pKLK, and FXIa) was incubated with various concentrations (10$^{-1}$ to 2 x 10$^3$ nM) of KD1$_{L17R}$-K$_T$ for 1 hour at RT in a 96-well microtitration plate (total volume 100 μL). Synthetic substrate (5 μL) appropriate for each enzyme was then added to a final concentration of 1 $K_M$ and residual amidolytic activity was measured in a Molecular Devices V$_{max}$ kinetic microplate reader. The inhibition constant, $K_i^*$ was determined using the non-linear regression data analysis program Grafit. Data for KD1$_{L17R}$-K$_T$ was analyzed with an equation for a tight-binding inhibitor (equation 1) where $v_i$ and $v_0$ are the inhibited and uninhibited rates, respectively, and $[I]_0$ and $[E]_0$ are the total concentrations of inhibitor and enzyme, respectively.[72,73]

$$v_i = v_0 \frac{\left(\left(K_i^* + [I]_0 + [E]_0\right)^2 - 4[I]_0[E]_0\right)^{1/2} - \left(K_i^* + [I]_0 - [E]_0\right)}{2[E]_0} \qquad \text{Equation 1}$$

$K_i$ values were obtained by correcting for the effect of substrate according to Beith,[72] using equation 2, where [S] is substrate concentration and $K_M$ is specific for each enzyme.

$$K_i = \frac{K_i^*}{(1 + [S]/K_M)} \qquad \text{Equation 2}$$

**Fibrinolysis (Clot lysis) Assay.** The method of Sperzel and Huetter[74] was followed with minor modifications as outlined earlier.[66] Briefly, IIa was used to initiate fibrin formation in NPP and the lysis of the formed clot (fibrinolysis) was induced by simultaneous addition of tPA. Clot formation and lysis were monitored with a Molecular Devices microplate reader (SPECTRAmax® 190) measuring the optical density at 405 nm. Briefly, 10 μL of each test compound (KD1$_{L17R}$-V$_T$, KD1$_{L17R}$-K$_T$, εACA) or saline control was added to 240 μL of NPP. Two hundred twenty-five μL of this mixture was then added to 25 μL IIa and tPA in TBS/BSA containing 25 mM CaCl$_2$. In the 250 μL final volume, the concentration of IIa was 0.15 μg/ml and that of tPA was 1 μg/ml. Under control conditions (zero tPA and zero test compound), OD$_{405}$ increased immediately indicating clotting followed by an extremely slow decrease representing fibrinolysis. As clotting was almost complete after 5 min, fibrinolysis induced by tPA was evaluated as a relative decrease of OD$_{405}$ up to 60 min. KD1$_{L17R}$-V$_T$ (plus/minus εACA) and KD1$_{L17R}$-K$_T$ were tested at final concentrations from 2 μM to 5 μM and εACA (without KD1$_{L17R}$-V$_T$) from 0.1 mM to 5 mM.

**Mouse Liver laceration model.** A protocol using this mouse model to study the efficacy of KD1$_{L17R}$-V$_T$ and of KD1$_{L17R}$-K$_T$ was approved by the UCLA Chancellor's Animal Research Committee. C57/BL6 mice (8-10 weeks old) were used, each weighing -20 gm in weight. Animals were fed standard rodent chow and water, and housed in a vivarium room with normal room temperature and 12 hour light-dark cycles. Dosage of KD1$_{L17R}$-V$_T$ or KD1$_{L17R}$-K$_T$ was based upon the aprotinin dose used in humans *i.e.,* 4 μg/gm adjusted for mouse weight.[75] A calculated blood level achieved by this dose was ~8 μg/ml (~1 μM). Animals injected with εACA (100 μg/gm, calculated blood level of 200 μg/ml or ~1.3 mM) were used as positive controls, and those receiving saline as negative controls. Dosage of εACA was based upon the dose used in human protocols.[39,75] Polymixin B immobilized resin (Detoxi-Gel™ Endotoxin Removing Gel from Pierce, Rockford, IL) was used to remove lipopolysaccharide from saline, KD1$_{L17R}$-V$_T$ and KD1$_{L17R}$-K$_T$. εACA was dissolved at 20 mg/ml in lipopolysaccharide-free saline. Both antifibrinolytic agents and saline were free of lipopolysaccharide as tested using the Limulus Amebocyte lysates kit (Biowhittaker Inc., Walkersville, MD).

Mice were injected intravenously via the tail vein, with the drug in a volume of 100 $\mu$L of sterile balanced salt solution immediately preceding anesthesia. Anesthesia was induced and maintained with Isoflurane. The animals were placed on a heating pad in supine position throughout the procedure to maintain rectal temperature at 37 °C. The abdomen was shaved and prepped. Liver was exposed via an anterior right subcostal incision. The left lobe of the liver was exteriorized by gentle continuous pressure on the left flank posteriorly. A 5-mm transverse incision was made on the left lobe at 1 cm from its inferior edge, using a sharp sterile # 10 scalpel blade. All blood oozing from the site of the left lobe incision was collected in a sterile preweighed 3 cmx3cm plastic tray for a total duration of 30 min. Animals were euthanized at the end of the 30-min period.

Factorial one-way analysis of variance (ANOVA) method was used to compare mean total blood loss in mg from the mouse liver across the four treatment groups (saline, $\varepsilon$ACA, $KD1_{L17R}$-$V_T$ and $KD1_{L17R}$-$K_T$). The p value for comparing any two means was computed using a post hoc test under this ANOVA model. Examination of the mean total blood loss error distribution pooled across all four treatments showed that it was normal (Gaussian) after controlling for the effect of treatment. The Shapiro-Wilk goodness of fit test was used to formally compare this distribution to a Gaussian distribution. Thus, no transformations of total blood loss were needed.

**Reduction in Glu-Plg Binding to U937 Cell Surface Receptors by Micro($\mu$)-Plasmin-$KD1_{L17R}$-$K_T$.** Recombinant N-terminal $His_6$-tagged $\mu$-plasminogen, containing residues 542-791 of the human plasminogen protease domain,[76] was expressed as inclusion bodies using pET28a vector, in *E. Coli* strain BL21(DE3). The $His_6$-tagged $\mu$-plasminogen was isolated using nickel-charged His-Trap column, refolded and purified using an SP-Sepharose® FF column as outlined.[76] Isolated $\mu$-plasminogen (1 mg/ml) was digested with IIa to remove $His_6$-tag [66], and activated to $\mu$-plasmin by uPA at a 1:1000 enzyme/substrate molar ratio for 2 hours at 37 °C. Removal of the $His_6$-tag permitting activation to $\mu$-plasmin was confirmed by reduced SDS-PAGE (15% acrylamide). IIa, uPA and the $His_6$-tag were separated from $\mu$-plasmin using a Superdex™-200 gel filtration column.[76] $KD1_{L17R}$-$V_T$ and $KD1_{L17R}$-$K_T$ each was incubated with purified $\mu$-plasmin at a molar ratio of 1.2:1 for 2 hours at room temperature. Each KD1-plasmin complex was then separated from the free $KD1_{L17R}$-$V_T$ or $KD1_{L17R}$-$K_T$ using Superdex™-200 gel filtration column.[76]

U937 monocyte-like cells were maintained at 37 °C with 5% $CO_2$ using RPMI-1640 medium supplemented with 10% fetal calf serum as outlined earlier.[77,78] The cells were stimulated with 40 nM PMA for 18 hours under sterile conditions as described.[78] The nonadherent cells were recovered by gentle decanting, washed and suspended in 50 mM Hepes containing 100 mM NaCl, 0.1 mg/ml BSA, pH 7.5 (HBS/BSA, pH 7.5). The cells ($10^6$/ml, 200 $\mu$l) were then incubated at 37 °C for 1 hour with 2 $\mu$M Glu-Plg containing varying concentrations of $\mu$-plasmin-$KD1_{L17R}$-$K_T$ or $\mu$-plasmin-$KD1_{L17R}$-$V_T$. The cells were washed and resuspended in 200 $\mu$l of HBS/BSA, pH 7.5, and the cell-bound Glu-Plg was activated with 10 nM uPA for 20 min at 37 °C. Each reaction mixture was diluted 10-fold and the plasmin activity (reflective of bound Glu-Plg) measured by S-2251 synthetic substrate hydrolysis using a Molecular Devices kinetic microplate reader.[58,78] The $IC_{50}$ ($\mu$l-plasmin-$KD1_{L17R}$-$K_T$ concentration required for 50% decrease in Glu-Plg binding to the cell surface receptors) was determined by fitting the data to the following $IC_{50}$ four-parameter logistic equation from Halfman[79] given below

$$y = \frac{a}{1+(x/\text{IC}_{50})^{\text{s}}} \qquad \text{Equation 3}$$

where y is the rate of S-2251 hydrolysis by plasmin generated from the Glu-Plg bound to the U937 cell receptors in the presence of a given concentration of $KD1_{L17R}$-$K_T$ or $KD1_{L17R}$-$V_T$ represented by *x*, a is the maximum rate of S-2251 hydrolysis in the absence of $KD1_{L17R}$-$K_T$ or $KD1_{L17R}$-$V_T$, and s is the slope factor. Each point was weighted equally, and the data were fitted to equation 3 using the nonlinear regression analysis program GraFit™ from Erithcus Software. To obtain a $K_d$ value for the interaction of $KD1_{L17R}$-$K_T$ with Glu-Plg, we used the following equation as described by Cheng and Prusoff[80] and further elaborated by Craig.[81]

$$K_{d\,(\text{KD1}_{\text{L17R}}\text{K}_{\text{T}})} = \frac{\text{IC}_{50}}{1+\left([\text{Glu}-\text{Plg}]/K_{d\,(\text{Glu}-\text{Plg/cell surface receptors})}\right)} \qquad \text{Equation 4}$$

where [Glu-Plg] concentration employed was 2 $\mu$M (-physiologic concentration).[9] To obtain $K_{d(\text{KD1}_{\text{L17R}}\text{K}_\text{T})}$ for Glu-Plg, we used a value of 1$\mu$M for $K_{d(\text{Glu-Plg/cell surface receptors})}$.[9,78,82]

**Molecular Modeling.** The crystal structures of $\mu$-plasmin,[83] plasminogen kringle domain1[84] and wild-type KD1[69] were used as templates to model the binary complex of $KD1_{L17R}$-$K_T$ with plasmin kringle domain1, and the ternary complex of $KD1_{L17R}$-$K_T$ with the plasmin protease domain and kringle domain1. The protocols for modeling these complexes have been described earlier.[66,85] Since the C-terminus residues are disordered in the wild-type KD1 crystal structure, we used the MODELLER program[86] to build this part of the KD1 molecule. The built models were further refined by energy minimization using the program CHARMM with CHARMM19 force field[87] consisting of 50 steps of the Steepest Descent, followed by 500 steps of Adopted Basis Newton-Raphson. Harmonic restraints of 10 kcal/mol/Å$^2$ were applied on the C$^\alpha$ atoms of the protein during the entire minimization.

RESULTS

**Proteolysis of KD1$_{L17R}$-V$_T$ by IIa.** In addition to the 58 residues corresponding to the BPTI-Kunitz domain, the 73-residue KD1$_{L17R}$-V$_T$ construct contains an additional nine residues at the N-terminus. The C-terminus tail residues (59EKVPKV64, BPTI numbering, SEQ ID NO: 44) in KD1$_{L17R}$-V$_T$ contain two lysine residues.[66] Experiments were conducted to determine whether incubation of KD1$_{L17R}$-V$_T$ with higher concentrations of IIa, as compared to those used during the removal of HiS$_6$-tag, could cleave between K60-V61 and/or between K63-V64 residues (59EK↓VPK↓V64). If so, the resulting molecule(s), in addition to inhibiting the active site of plasmin, also according to non-limiting theory, is believed to serve as a decoy receptor to attenuate pathological plasminogen activation. Incubation of KD1$_{L17R}$-V$_T$ (1 mg/ml, in TBS pH 7.5) with IIa at a 1:50 enzyme/substrate molar ratio for 24 hours at 37 °C resulted in the formation of a smaller molecular weight species referred to as KD1$_{L17R}$-K$_T$. SDS-PAGE data of KD$_{1L17}$-V$_T$ and KD1$_{L17R}$-K$_T$ are given in Figure 7. KD1$_{L17R}$-K$_T$ was separated from IIa using Superdex™-200 gel filtration chromatography for further studies.

The NH$_2$-terminal sequence(s) of KD1$_{L17R}$-K$_T$ is presented in Table 1 and the MALDI-TOF-ESI data are presented in Figure 8A. There were two readable sequences, a predominant sequence (KD1$_{L17R}$-K$_T$ (~80%), SEQ ID NO: 47) at -80 pmol level and a minority sequence (KD1$_{L17R}$-K$_T$ (~20%), SEQ ID NO: 48) at -20 pmol level. The minor sequence (SEQ ID NO: 48) was internal to the major sequence (SEQ ID NO: 47) starting at cycle 3.

**Table 1. NH$_2$-Terminal sequence analysis of IIa cleaved KD1$_{L17R}$-V$_T$.**

| Cycle Number | Amino acid (pmol) | Amino acid (pmol) |
|:---:|:---:|:---:|
| 1 | Gly (82.2) | Asn (19.2) |
| 2 | Asn (82.6) | Ala (21.5) |
| 3 | Asn (68.5) | Glu (16.5) |
| 4 | Ala (53.3) | Ile (14.7) |
| 5 | Glu (44.6) | X (Cys) |
| 6 | Ile (42.5) | Leu (12.8) |
| 7 | X (Cys) | Leu (11.9) |
| 8 | Leu (35.2) | Pro (10.8) |

Combined analysis of the sequence(s) and the mass spectrometry data revealed that -80% of KD1$_{L17R}$-K$_T$ had the N-terminal sequence GNNAEI (SEQ ID NO: 49) and -20% had the N-terminal sequence NAEI (SEQ ID NO: 50); both species had the same C-terminal sequence EKVPK (SEQ ID NO: 51). Amino acid sequence alignment of BPTI, KD1$_{L17R}$-V$_T$ and KD1$_{L17R}$-K$_T$ is presented in Figure 8B. It is believed according to non-limiting theory that IIa cleaved KD1$_{L17R}$-V$_T$ between VPK↓V at the C-terminus; however, the hydrolysis at PT↓GN and GN↓NA at the N-terminus could be by IIa or conceivably by a contaminating protease. No cleavage was observed between EK↓VP at the C-terminus. KD1$_{L17R}$-K$_T$ was thus homogeneous at the C-terminus with ~20% of the molecules lacking the first two amino acids at the N-terminus (Figures 8A and 8B).

IIa cleaves Arg/Lys(P1 subsite)-xxx peptide bonds and has a strong preference for proline or a small hydrophobic residue at the P2 position.[88] Further, a bulky or an acidic residue at the P2 position is strongly disfavored by IIa.[88,89] Accordingly, under the conditions of higher concentration of IIa used, cleavage between 62Pro-Lys↓Val64 (Figure 9) of KD1$_{L17R}$-V$_T$ is expected, whereas cleavage between 59Glu-Lys↓Val-Pro62 is disfavored (Figure 8B). Although threonine or asparagine at the P1 subsite is not preferred by IIa, its S1 pocket residue Asp189 can interact with Thr or Asn through a water molecule (Figure 9). The proteolysis between Pro-Thr↓Gly-Asn or Gly-Asn↓Asn-Ala by IIa is also, according to non-limiting theory, believed to be facilitated by the presence of Pro or Gly at the P2 position (Figure 9). As a result, a stable KD1$_{L17R}$-K$_T$ protein was obtained that had an intact Kunitz domain and contained a C-terminal lysine.

**KD1$_{L17R}$-K$_T$ Binding to tPA and Glu-Plg.** Surface plasmon resonance (SPR) was used to study the binding of KD1$_{L17R}$-K$_T$ to immobilized tPA (Figure 10A) and Glu-Plg (Figure 10B). The $k_{on}$ for binding of tPA to KD1$_{L17R}$-K$_T$was 0.91 ± 0.2 x 10$^3$ M$^{-1}$s$^{-1}$; $k_{off}$ was 1.9 ± 0.3 x 10$^{-4}$ s$^{-1}$, and the $K_d$ was 210 ± 20 nM. The $k_{on}$ for binding of Glu-Plg to KD1$_{L17R}$-K$_T$was 1.1 ± 0.4 x 10$^3$ M$^{-1}$s$^{-1}$; $k_{off}$ was 3.1 ± 0.8 x 10$^{-4}$ s$^{-1}$, and the $K_d$ was 280 ± 30 nM. KD1$_{L17R}$-V$_T$ showed negligible binding to tPA or Glu-Plg in SPR experiments, suggesting according to non-limiting theory that the C-terminal lysine in KD1$_{L17R}$-K$_T$ is important for this interaction.

Next, the complex of KD1$_{L17R}$-K$_T$ with the kringle domain1 of plasminogen was modeled. The C-terminal lysine bound in an analogous manner as $\varepsilon$ACA (Figure 10C). In addition, two other interactions were noted: (1) carboxylate of Glu59 of the inhibitor KD1$_{L17R}$-K$_T$ formed a salt bridge with the guanidino group of Arg32 of plasminogen; and (2) the carbonyl group of Lys60 of the inhibitor formed a hydrogen bond with the side chain of Arg70 of kringle domain1 of plasminogen. These additional interactions reflected the higher affinity of KD1$_{L17R}$-K$_T$ for kringle domain1 of plasminogen as compared to its affinity for $\varepsilon$ACA.[84,90]

**Inhibition Profile of KD1$_{L17R}$-K$_T$.** The KD1$_{L17R}$-K$_T$ inhibited plasmin (Figure 11) with high affinity ($K_i$ =1 $\pm$ 0.2 nM) very similar to KD1$_{L17R}$-V$_T$. Further, both KD1$_{L17R}$-V$_T$[66] and KD1$_{L17R}$-K$_T$ inhibited FXIa and pKLK with $K_i$ > 10 $\mu$M (Figure 11). Moreover, KD1$_{L17R}$-V$_T$[66] or KD1$_{L17R}$-K$_T$ (data not shown) did not inhibit IIa, activated protein C, tPA, uPA or tissue kallikreins. Collectively, the data indicate that the Kuntiz domain in KD1$_{L17R}$-K$_T$ was fully functional.

**Fibrinolysis Assay.** The abilities of each of KD1$_{L17R}$-K$_T$, KD1$_{L17R}$-V$_T$ and $\varepsilon$ACA to inhibit tPA-induced plasma clot fibrinolysis were compared. Addition of IIa to NPP caused fibrin formation reflected by the increase in OD$_{405}$ (Figure 12A-C, curve 1). Simultaneous addition of tPA caused initial clot formation followed by dissolution of fibrin induced by tPA mediated conversion of plasminogen to plasmin (curve 2, Figure 12A-C); the midpoint of fibrinolysis was between 6 to 7 minutes in each case. All three antifibrinolytic agents inhibited fibrinolysis in a dose-dependent manner. KD1$_{L17R}$-V$_T$ increased the fibrinolysis midpoint to 22 min at 2 $\mu$M, to 29 min at 3 $\mu$M and to 43 min at 5 $\mu$M, respectively (Figure 12A). In contrast, KD1$_{L17R}$-K$_T$ increased the fibrinolysis midpoint to 27 min at 2 $\mu$M, to 37 min at 3 $\mu$M and to >60 min at 5 $\mu$M, respectively (Figure 12A). Thus, at each concentration tested, KD1$_{L17R}$-K$_T$ was more effective as an antifibrinolytic agent as compared to KD1$_{L17R}$-V$_T$. Since active site inhibition of plasmin by both KD1$_{L17R}$-V$_T$[66] and KD1$_{L17R}$-K$_T$ was similar (Figure 11), the additional increased potency of KD1$_{L17R}$-K$_T$ could be due to its binding to the kringle domains of tPA and/or plasminogen (Figure 10A and 10B). As a result, it is expected that localized plasminogen activation at the fibrin clot would also be attenuated.

Higher concentrations of KD1$_{L17R}$-V$_T$ or KD1$_{L17R}$-K$_T$ were needed to inhibit fibrinolysis than those predicted from the $K_i$ values for plasmin inhibition. Without wishing to be bound by theory an explanation for these observations may be that the antifibrinolytic assay was a kinetic based assay, whereas the equilibrium inhibition constant $K_i$ was obtained upon prolonged incubation of the inhibitor with plasmin.

The antifibrinolytic activity of $\varepsilon$ACA is attributed to its binding to the kringle domains of tPA and plasminogen.[84,90] Therefore, $\varepsilon$ACA was tested for its ability to augment the fibrinolytic inhibition observed for KD1$_{L17R}$-V$_T$. These data are presented in Figure 12B. Equimolar additions of $\varepsilon$ACA to the KD1$_{L17R}$-V$_T$ at 2, 3, or 5 $\mu$M had no observable effect on the extent of inhibition of plasma clot fibrinolysis. The most likely non-limiting explanation for this observation was the low affinity ($K_d$~10 $\mu$M)[84,90] of $\varepsilon$ACA for tPA or plasminogen as compared to the KD1$_{L17R}$-K$_T$ ($K_d$ ~0.2-0.3 $\mu$M, Figure 10). Consistent with these data, much higher concentrations of $\varepsilon$ACA were needed to inhibit plasma clot fibrinolysis in the absence of KD1$_{L17R}$-V$_T$ (Figure 12C). Addition of $\varepsilon$ACA increased the clot lysis midpoint to 10 min at 0.1 mM, 19 min at 0.3 mM, and > 60 min at 0.5 mM, respectively. Essentially no fibrinolysis was observed when concentrations of 1 or 5 mM $\varepsilon$ACA were used.

**Mouse Liver Laceration Model.** Because augmented fibrinolysis is an important cause of bleeding from small vessels in the mouse liver laceration model,[91] the efficacy of KD1$_{L17R}$-K$_T$ in decreasing blood loss was evaluated, and compared to the efficacy of $\varepsilon$ACA and KD1$_{L17R}$-V$_T$ in this animal model. The doses of $\varepsilon$ACA, KD1$_{L17R}$-K$_T$ or KD1$_{L17R}$-V$_T$ that were used were comparable to the doses typically used in a clinical setting. Although, the half-life of each of these two antifibrinolytic agents was short (~2 hours), a total duration of bleeding for 30 min should retain a sufficient level of each inhibitor for efficacy studies. The effects of the three antifibrinolytic agents on the quantity of blood loss are depicted in Figure 13. As compared to saline, the blood loss was reduced by -74% by KD1$_{L17R}$-K$_T$ (*p 0.001*), ~50% by KD1$_{L17R}$-V$_T$ (*p 0.002*), and by -49 % by $\varepsilon$ACA (*p 0.002*). There was essentially no difference in the amount of blood loss between KD1$_{L17R}$-V$_T$ and $\varepsilon$ACA-treated animals. KD1$_{L17R}$-K$_T$ was more effective than KD1$_{L17R}$-V$_T$ (*p* < 0.05) or $\varepsilon$ACA (*p* < 0.05) at inhibiting plasmin-induced fibrinolysis and blood loss in this animal model.

In the mouse liver laceration model, there was no statistically significant difference between KD1$_{L17R}$-K$_T$ (SEQ ID NOS: 47: 48, ~80%:~20%) and the engineered KD1 polypeptide preparation previously described as "KD1-L17R", which was presumed to have Val at the C-terminus.[66] KD1-L17R was therefore characterized by SDS-PAGE, mass spectrometry and N-terminal sequence analysis (Figure 14). SDS-PAGE analysis indicated that the protein was not noticeably degraded. N-terminal sequencing revealed Gly-Ser-His-Met-Asp-Ala-Ala-Gln-Glu-Pro (SEQ ID NO: 53) as the first 10 residues and mass spectrometric data revealed that the sample had a molecular mass of 8792.1 Da, which was within standard error of the theoretical mass of 8787 Da for SEQ ID NO: 52 (76 amino acids) but which was -100 Da less than the theoretical mass of 8888 Da for KD1-L17R having C- terminal valine (SEQ ID NO: 46, 77 amino acids). KD1-L17R[66] was thus shown to have the 76 amino acid sequence set forth herein as SEQ ID NO: 52, which lacked C-terminal valine but which had C-terminal lysine, as was also the case for KD1$_{L17R}$-K$_T$. KD1-L17R having C-terminal lysine (SEQ ID NO: 52), and KD1$_{L17R}$-K$_T$ (SEQ ID NOS: 47: 48, ~80%:~20%) which also has C-terminal lysine both exhibited greater efficacy at preventing blood loss in the mouse liver laceration model than did KD1$_{L17R}$-V$_T$ (SEQ ID NO: 46), which has C-terminal valine.

It was also observed that four of the sixteen animals (one out of four each day) treated with $\varepsilon$ACA depicted

generalized seizures shortly after injection of the drug. During surgery, these animals had unilateral or bilateral clonic movements on 3-4.5% isoflurane. Abnormal movements were not observed during the induction of anesthesia, but rather 15-20 min after the onset of surgery. Seizures appeared as repetitive clonic movements of one or more extremities while the animals remained unresponsive under anesthesia. Animals were not observed to have any tonic movements. Convulsion and seizures have been previously observed in animals given $\varepsilon$ACA or TE and represent a major side effect of these drugs as antifibrinolytic agents.[92,93] Other recent studies have reported that $\varepsilon$ACA[39,40,94] or TE[39,94,95] causes seizures and convulsion in a significant number of patients. No seizures were observed in animals treated with either $KD1_{L17R}-V_T$ or $KD1_{L17R}-K_T$.

Furthermore, the use of $\varepsilon$ACA, TE and aprotinin is associated with renal dysfunction/failure,[36-39,94] whereas two KD1 molecules (with Arg15$\rightarrow$Lys or Leu17$\rightarrow$Arg) of TFPI-2 did not cause renal toxicity in mouse[66] or rat.[96] Thus, in accordance with some embodiments disclosed herein, $KD1_{L17R}-K_T$ is a preferred agent in attenuating fibrinolysis *in vivo.*

**Effects of $\mu$-plasmin-$KD1_{L17R}-K_T$ on Glu-Plg Binding to PMA-stimulated nonadherent U937 cells.** A majority of the cell surface receptors for plasminogen have lysine as their C-terminal residue.[82] Experiments were designed to determine whether $KD1_{L17R}-K_T$ (and not $KD1_{L17R}-V_T$) bound to the kringle domain(s) of Glu-Plg and impaired its binding to the U937 cell surface receptors. $\mu$-plasmin-$KD1_{L17R}-V_T$ and $\mu$-plasmin-$KD1_{L17R}-K_T$ complexes were prepared to block the Kunitz domain function. The SDS-PAGE of the Superdex™-200 gel purified complexes of $\mu$-plasmin-$KD1_{L17R}-V_T$ and $\mu$-plasmin-$KD1_{L17R}-K_T$ are shown in the inset of Figure 15A. PMA-stimulated U937 cells were incubated with Glu-Plg containing varying concentrations of $\mu$-plasmin-$KD1_{L17R}-V_T$ complex or $\mu$-plasmin-$KD1_{L17R}-K_T$ complex (Figure 15A). The increasing concentrations of $\mu$-plasmin-$KD1_{L17R}-K_T$ (but not of $\mu$-plasmin-$KD1_{L17R}-V_T$) inhibited Glu-Plg binding to the cell surface receptors, as deduced from the S-2251 hydrolysis data shown in Figure 15A. Thepercent reduction in plasmin generation by the purified $\mu$-plasmin-$KD1_{L17R}-K_T$ or $\mu$-plasmin-$KD1_{L17R}-V_T$ is presented in Figure 15B. $\mu$-plasmin-$KD1_{L17R}-K_T$ complex interfered with Glu-Plg binding to the PMA-stimulated U937 cells. Using equations 3 and 4, a $K_d$ value for the interaction of $KD1_{L17R}-K_T$ with Glu-Plg was calculated to be ~300 nM, a value consistent with the value (~280 nM) obtained from the SPR data (Figure 10B).

The data presented in Figure 15 were consistent with a model by which a single molecule of $KD1_{L17R}-K_T$ was bound simultaneously to the kringle domain of plasminogen/plasmin as well as to the active site of plasmin. A molecularly modeled ternary complex of $KD1_{L17R}-K_T$ with the plasmin protease domain and the plasminogen/plasmin kringle domain1 was generated and is depicted in Figure 16. As shown in Fig. 16, the $KD1_{L17R}-K_T$ interacts via its P5-P5' residues with the active site of plasmin, a typical mode of Kunitz domain binding to a serine protease domain. Similarly, the interactions of the C-terminal lysine of $KD1_{L17R}-K_T$ with the kringle domain1 of plasminogen/plasmin are analogous to interactions between the kringle domain1 and the lysine analogs $\varepsilon$ACA or TE. However, as compared to the lysine analogs, $KD1_{L17R}-K_T$ features additional points of contact with the kringle domain1 of plasminogen/plasmin that strengthen the binding interaction (Figure 16).

Accordingly and by way of non-limiting theory, a dual function as an antifibrinolytic agent is contemplated for the $KD1_{L17R}-K_T$ polypeptides described herein. First, $KD1_{L17R}-K_T$ attenuates tPA and plasminogen binding to the exposed C-terminal lysine residues in fibrin, which reduces plasmin formation. Secondly, $KD1_{L17R}-K_T$ inhibits the active site of the generated plasmin.

REFERENCES CITED IN EXAMPLE 5:

(1) Petersen, T. E., Martzen, M. R., Ichinose, A., and Davie, E. W. (1990) Characterization of the gene for human plasminogen, a key proenzyme in the fibrinolytic system, J. Biol. Chem. 265, 6104-6111.

(2) Zhang, L., Seiffert, D., Fowler, B. J., Jenkins, G. R., Thinnes, T. C., Loskutoff, D. J., Parmer, R. J., and Miles, L. A. (2002) Plasminogen has a broad extrahepatic distributions, Thromb. Haemostasis 87, 493-501.

(3) Bohmfalk, J. F., and Fuller, G. M. (1980) Plasminogen is synthesized by primary cultures of rat hepatocytes, Science 209, 408-410.

(4) Saito, H., Hamilton, S. M., Tavill, A. S., Louis, L., and Ratnoff, O. D. (1980) Production and release of plasminogen by isolated perfused rat-liver, Proc. Natl. Acad. Sci. U. S. A. 77, 6837-6840.

(5) Miles, L. A., Castellino, F. J., and Gong, Y. (2003) Critical role for conversion of glu-plasminogen to Lys-plasminogen for optimal stimulation of plasminogen activation on cell surfaces, Trends Cardiovasc. Med. 13, 21-30.

(6) Cockell, C. S., Marshall, J. M., Dawson, K. M., Cederholm-Williams, S. A., and Ponting, C. P. (1998) Evidence that the conformation of unliganded human plasminogen is maintained via an intramolecular interaction between the lysine-binding site of kringle 5 and the N-terminal peptide, Biochem. J. 333, 99-105.

(7) Law, R. H. P., Caradoc-Davies, T., Cowieson, N., Horvath, A. J., Quek, A. J., Encarnacao, J. A., Steer, D., Cowan, A., Zhang, Q. W., Lu, B. G. C., Pike, R. N., Smith, A. I., Coughlin, P. B., and Whisstock, J. C. (2012) The x-ray crystal structure of full-length human plasminogen, Cell Rep. 1, 185-190.

(8) Violand, B. N., and Castellino, F. J. (1976) Mechanism of urokinase-catalyzed activation of human plasminogen, J. Biol. Chem. 251, 3906-3912.

(9) Castellino, F. J., and Ploplis, V. A. (2005) Structure and function of the plasminogen/plasmin system, Thromb. Haemostasis 93, 647-654.

(10) Ellis, V., Behrendt, N., and Dano, K. (1991) Plasminogen activation by receptor-bound urokinase - a kinetic-study with both cell-associated and isolated receptor, J. Biol. Chem. 266, 12752-12758.

(11) Urano, T., Deserrano, V. S., Gaffney, P. J., and Castellino, F. J. (1988) Effectors of the activation of human [glu]plasminogen by human-tissue plasminogen-activator, Biochemistry 27, 6522-6528.

(12) Violand, B. N., Sodetz, J. M., and Castellino, F. J. (1975) Effect of $\varepsilon$-amino caproic acid on gross conformation of plasminogen and plasmin, Arch. Biochem. Biophys. 170, 300-305.

(13) Markus, G., Evers, J. L., and Hobika, G. H. (1978) Comparison of some properties of native (glu) and modified (lys) human plasminogen, J. Biol. Chem. 253, 733-739.

(14) Claeys, H., and Vermylen, J. (1974) Physicochemical and proenzyme properties of NH2-terminal glutamic-acid and NH2-terminal lysine human plasminogen - influence of 6-aminohexanoic acid, Biochim. Biophys. Acta 342, 351-359.

(15) Fredenburgh, J. C., and Nesheim, M. E. (1992) Lys-plasminogen is a significant intermediate in the activation of glu-plasminogen during fibrinolysis invitro, J. Biol. Chem. 267, 26150-26156.

(16) Kruithof, E. K. O., Tranthang, C., Ransijn, A., and Bachmann, F. (1984) Demonstration of a fast-acting inhibitor of plasminogen activators in human-plasma, Blood 64, 907-913.

(17) Åstedt, B., Lecander, I., and Ny, T. (1987) The placental type plasminogen activator inhibitor, PAI 2, Fibrinolysis 1, 203-208.

(18) Sprengers, E. D., and Kluft, C. (1987) Plasminogen-activator inhibitors, Blood 69, 381-387.

(19) Collen, D., and Wiman, B. (1978) Fast-acting plasmin inhibitor in human-plasma, Blood 51, 563-569.

(20) Hall, S. W., Humphries, J. E., and Gonias, S. L. (1991) Inhibition of cell-surface receptor-bound plasmin by alpha-2-antiplasmin and alpha-2-macroglobulin, J. Biol. Chem. 266, 12329-12336.

(21) Collen, D. (2001) Ham-Wasserman lecture: role of the plasminogen system in fibrin-homeostasis and tissue remodeling, Hematology Am. Soc. Hematol. Educ. Program, 1-9.

(22) Camiolo, S. M., Thorsen, S., and Astrup, T. (1971) Fibrinogenolysis and fibrinolysis with tissue plasminogen activator, urokinase, streptokinase-activated human globulin, and plasmin, P. Soc. Exp. Biol. Med. 138, 277-280.

(23) Pennica, D., Holmes, W. E., Kohr, W. J., Harkins, R. N., Vehar, G. A., Ward, C. A., Bennett, W. F., Yelverton, E., Seeburg, P. H., Heyneker, H. L., Goeddel, D. V., and Collen, D. (1983) Cloning and expression of human tissue-type plasminogen-activator cDNA in escherichia-coli, Nature 301, 214-221.

(24) Banyai, L., Varadi, A., and Patthy, L. (1983) Common evolutionary origin of the fibrin-binding structures of fibronectin and tissue-type plasminogen-activator, Febs. Lett. 163, 37-41.

(25) Devries, C., Veerman, H., and Pannekoek, H. (1989) Identification of the domains of tissue-type plasminogen-activator involved in the augmented binding to fibrin after limited digestion with plasmin, J. Biol. Chem. 264, 12604-12610.

(26) Vanzonneveld, A. J., Veerman, H., and Pannekoek, H. (1986) On the interaction of the finger and the kringle-2 domain of tissue-type plasminogen-activator with fibrin - inhibition of kringle-2 binding to fibrin by epsilon-amino caproic acid, J. Biol. Chem. 261, 14214-14218.

(27) Devries, C., Veerman, H., Koornneef, E., and Pannekoek, H. (1990) Tissue-type plasminogen-activator and its substrate glu-plasminogen share common binding-sites in limited plasmin-digested fibrin, J. Biol. Chem. 265, 13547-13552.

(28) Weisel, J. W., and Dempfle, C. H. (2012) Fibrinogen structure and function, In Hemostasis and Thrombosis (Marder, V. J., Aird, W. C., Bennett, J. S., Schulman, S., and White, G. C., Eds.) 6th Edition ed., pp 254-271, Lippincott Williams & Wilkins, Philadelphia, PA.

(29) Satkurunath, G., and Royston, D. (2008) Hemostatic drugs in trauma and orthopaedic practice, International Trauma Care 18, 24-28.

(30) Hartmann, M., Sucker, C., Boehm, O., Koch, A., Loer, S., and Zacharowski, K. (2006) Effects of cardiac surgery on hemostasis, Transfus. Med. Rev. 20, 230-241.

(31) Dhir, A. (2013) Antifibrinolytics in cardiac surgery, Ann. Card. Anaesth. 16, 117-125.

(32) Sauaia, A., Moore, F. A., Moore, E. E., Moser, K. S., Brennan, R., Read, R. A., and Pons, P. T. (1995) Epidemiology of trauma deaths - a reassessment, J. Trauma 38, 185-193.

(33) Levy, J. H. (2006) Aprotinin is useful as a hemostatic agent in cardiopulmonary surgery: yes, J. Thromb. Haemostasis 4, 1875-1878.

(34) Levi, M., Cromheecke, M. E., de Jonge, E., Prins, M. H., de Mol, B. J. M., Briet, E., and Buller, H. R. (1999) Pharmacological strategies to decrease excessive blood loss in cardiac surgery: a meta-analysis of clinically relevant endpoints, Lancet 354, 1940-1947.

(35) Royston, D., Van Haaften, N., and De Vooght, P. (2007) Aprotinin; friend or foe? A review of recent medical literature, Eur. J. Anaesth. 24, 6-14.

(36) Mangano, D. T., Tudor, I. C., Dietzel, C., Is, M. S. P., and Fdn, I. R. E. (2006) The risk associated with aprotinin in cardiac surgery, New Engl. J. Med. 354, 353-365.

(37) Immer, F. F., Jent, P., Englberger, L., Stalder, M., Gygax, E., Carrel, T. P., and Tevaearai, H. T. (2008) Aprotinin in cardiac surgery: A different point of view, Heart Surg. Forum 11, E9-E12.

(38) Beierlein, W., Scheule, A. M., Dietrich, W., and Ziemer, G. (2005) Forty years of clinical aprotinin use: A review of 124 hypersensitivity reactions, Ann. Thorac. Surg. 79, 741-748.

(39) Martin, K., Knorr, J., Breuer, T., Gertler, R., MacGuill, M., Lange, R., Tassani, P., and Wiesner, G. (2011) Seizures after open heart surgery: comparison of epsilon-aminocaproic acid and tranexamic acid, J. Cardiothor. Vasc. An. 25, 20-25.

(40) Rabinovici, R., Heyman, A., Kluger, Y., and Shinar, E. (1989) Convulsions induced by aminocaproic acid infusion, Dicp. Ann. Pharmac. 23, 780-781.

(41) Salonen, E. M., Zitting, A., and Vaheri, A. (1984) Laminin interacts with plasminogen and its tissue-type activator, FEBS Lett. 172, 29-32.

(42) Silverstein, R. L., Leung, L. L. K., Harpel, P. C., and Nachman, R. L. (1984) Complex-formation of platelet thrombospondin with plasminogen - modulation of activation by tissue activator, J. Clin. Invest. 74, 1625-1633.

(43) Salonen, E. M., Saksela, O., Vartio, T., Vaheri, A., Nielsen, L. S., and Zeuthen, J. (1985) Plasminogen and tissue-type plasminogen-activator bind to immobilized fibronectin, J. Biol. Chem. 260, 2302-2307.

(44) Lijnen, H. R., Van Hoef, B., Lupu, F., Moons, L., Carmeliet, P., and Collen, D. (1998) Function of the plasminogen/plasmin and matrix metalloproteinase systems after vascular injury in mice with targeted inactivation of fibrinolytic system genes, Arterioscl. Throm. Vas. 18, 1035-1045.

(45) Bonnefoy, A., and Legrand, C. (2000) Proteolysis of subendothelial adhesive glycoproteins (fibronectin, thrombospondin, and von Willebrand factor) by plasmin, leukocyte cathepsin G, and elastase, Thromb. Res. 98, 323-332.

(46) Lijnen, H. R. (2000) Molecular interactions between the plasminogen/plasmin and matrix metalloproteinase systems, Fibrinolysis Proteol. 14, 175-181.

(47) Plow, E. F., Herren, T., Redlitz, A., Miles, L. A., and Hooverplow, J. L. (1995) The cell biology of the plasminogen system, FASEB J. 9, 939-945.

(48) He, C. S., Wilhelm, S. M., Pentland, A. P., Marmer, B. L., Grant, G. A., Eisen, A. Z., and Goldberg, G. I. (1989) Tissue cooperation in a proteolytic cascade activating human interstitial collagenase, Proc. Natl. Acad. Sci. U. S. A. 86, 2632-2636.

(49) Okada, Y., Gonoji, Y., Naka, K., Tomita, K., Nakanishi, I., Iwata, K., Yamashita, K., and Hayakawa, T. (1992) Matrix metalloproteinase-9 (92-kda gelatinase type-iv collagenase) from ht-1080 human fibrosarcoma cells - purification and activation of the precursor and enzymatic-properties, J. Biol. Chem. 267, 21712-21719.

(50) Holliday, L. S., Welgus, H. G., Fliszar, C. J., Veith, G. M., Jeffrey, J. J., and Gluck, S. L. (1997) Initiation of osteoclast bone resorption by interstitial collagenase, J. Biol. Chem. 272, 22053-22058.

(51) Gong, Y. Q., Hart, E., Shchurin, A., and Hoover-Plow, J. (2008) Inflammatory macrophage migration requires MMP-9 activation by plasminogen in mice, J. Clin. Invest. 118, 3012-3024.

(52) Lee, S., Jilani, S. M., Nikolova, G. V., Carpizo, D., and Iruela-Arispe, M. L. (2005) Processing of VEGF-A by matrix metalloproteinases regulates bioavailability and vascular patterning in tumors, J. Cell. Biol. 169, 681-691.

(53) McColl, B. K., Baldwin, M. E., Roufail, S., Freeman, C., Moritz, R. L., Simpson, R. J., Alitalo, K., Stacker, S. A., and Achen, M. G. (2003) Plasmin activates the lymphangiogenic growth factors VEGF-C and VEGF-D, J. Exp. Med. 198, 863-868.

(54) Falcone, D. J., Mccaffrey, T. A., Haimovitzfriedman, A., Vergilio, J. A., and Nicholson, A. C. (1993) Macrophage and foam cell release of matrix-bound growth-factors - role of plasminogen activation, J. Biol. Chem. 268, 11951-11958.

(55) Syrovets, T., Lunov, O., and Simmet, T. (2012) Plasmin as a proinflammatory cell activator, J. Leukocyte Biol. 92, 509-519.

(56) Miles, L. A., Dahlberg, C. M., and Plow, E. F. (1988) The cell-binding domains of plasminogen and their function in plasma, J. Biol. Chem. 263, 11928-11934.

(57) Miles, L. A., Dahlberg, C. M., Plescia, J., Felez, J., Kato, K., and Plow, E. F. (1991) Role of cell-surface lysines in plasminogen binding to cells - identification of alpha-enolase as a candidate plasminogen receptor, Biochemistry 30, 1682-1691.

(58) Andronicos, N. M., Chen, E. I., Baik, N., Bai, H., Parmer, C. M., Kiosses, W. B., Kamps, M. P., Yates, J. R., Parmer, R. J., and Miles, L. A. (2010) Proteomics-based discovery of a novel, structurally unique, and developmentally regulated plasminogen receptor, Plg-R-KT, a major regulator of cell surface plasminogen activation, Blood 115, 1319-1330.

(59) Liotta, L. A., Goldfarb, R. H., Brundage, R., Siegal, G. P., Terranova, V., and Garbisa, S. (1981) Effect of plasminogen-activator (urokinase), plasmin, and thrombin on glycoprotein and collagenous components of basement-membrane, Cancer Res. 41, 4629-4636.

(60) Festuccia, C., Dolo, V., Guerra, F., Violini, S., Muzi, P., Pavan, A., and Bologna, M. (1998) Plasminogen activator system modulates invasive capacity and proliferation in prostatic tumor cells, Clin. Exp. Metastas. 16, 513-528.

(61) Sprecher, C. A., Kisiel, W., Mathewes, S., and Foster, D. C. (1994) Molecular-cloning, expression, and partial characterization of a second human tissue-factor-pathway inhibitor, Proc. Natl. Acad. Sci. U. S. A. 91, 3353-3357.

(62) Miyagi, Y., Koshikawa, N., Yasumitsu, H., Miyagi, E., Hirahara, F., Aoki, I., Misugi, K., Umeda, M., and Miyazaki, K. (1994) cDNA cloning and messenger-rna expression of a serine proteinase-inhibitor secreted by cancer-cells - identification as placental protein-5 and tissue factor pathway inhibitor-2, J. Biochem-Tokyo 116, 939-942.

(63) Rao, C. N., Reddy, P., Liu, Y. Y., OToole, E., Reeder, D., Foster, D. C., Kisiel, W., and Woodley, D. T. (1996) Extracellular matrix-associated serine protease inhibitors (M(r) 33,000, 31,000, and 27,000) are single-gene products with differential glycosylation: cDNA cloning of the 33-kDa inhibitor reveals its identity to tissue factor pathway inhibitor-2, Arch. Biochem. Biophys. 335, 82-92.

(64) Petersen, L. C., Sprecher, C. A., Foster, D. C., Blumberg, H., Hamamoto, T., and Kisiel, W. (1996) Inhibitory properties of a novel human Kunitz-type protease inhibitor homologous to tissue factor pathway inhibitor, Biochemistry 35, 266-272.

(65) Schechte.I, and Berger, A. (1967) On size of active site in proteases .I. papain, Biochem. Biophy. Res. Com. 27, 157-162.

(66) Bajaj, M. S., Ogueli, G. I., Kumar, Y., Vadivel, K., Lawson, G., Shanker, S., Schmidt, A. E., and Bajaj, S. P. (2011) Engineering kunitz domain 1 (KD1) of human tissue factor pathway inhibitor-2 to selectively inhibit fibrinolysis properties of KD1-L17R variant, J. Biol. Chem. 286, 4329-4340.

(67) Sambrook, J. and Russel, D. W. (2001) Molecular cloning: a laboratory manual, Third Edition, Cold Spring Harbor Laboratory Press, Cold Spring Harbor, ME.

(68) Chand, H. S., Schmidt, A. E., Bajaj, S. P., and Kisiel, W. (2004) Structure-function analysis of the reactive site in the first Kunitz-type domain of human tissue factor pathway inhibitor-2, J. Biol. Chem. 279, 17500-17507.

(69) Schmidt, A. E., Chand, H. S., Cascio, D., Kisiel, W., and Bajaj, S. P. (2005) Crystal structure of Kunitz domain 1 (KD1) of tissue factor pathway inhibitor-2 in complex with trypsin - implications for KD1 specificity of inhibition, J. Biol. Chem 280, 27832-27838.

(70) Stone, M. J., Ruf, W., Miles, D. J., Edgington, T. S., and Wright, P. E. (1995) Recombinant soluble human tissue factor secreted by saccharomyces-cerevisiae and refolded from escherichia-coli inclusion-bodies - glycosylation of mutants, activity and physical characterization, Biochem. J. 310, 605-614.

(71) Laemmli, U. K. (1970) Cleavage of structural proteins during assembly of head of bacteriophage-t4, Nature 227, 680-685.

(72) Bieth, J. G. (1984) In vivo significance of kinetic constants of protein proteinase-inhibitors, Biochem. Med. Metab. B 32, 387-397.

(73) Morrison, J. F., and Walsh, C. T. (1988) The behavior and significance of slow-binding enzyme-inhibitors, Adv. Enzymol. Ramb. 61, 201-301.

(74) Sperzel, M., and Huetter, J. (2007) Evaluation of aprotinin and tranexamic acid in different in vitro and in vivo models of fibrinolysis, coagulation and thrombus formation, J. Thromb. Haemostasis 5, 2113-2118.

(75) Henry, D. A., Carless, P. A., Moxey, A. J., O'Connell, D., Stokes, B. J., McClelland, B., Laupacis, A., and Fergusson, D. (2007) Anti-fibrinolytic use for minimising perioperative allogeneic blood transfusion, Cochrane Db. Syst. Rev.

(76) Medynski, D., Tuan, M., Liu, W., Wu, S. L., and Lin, X. L. (2007) Refolding, purification, and activation of miniplasminogen and microplasminogen isolated from E-coli inclusion bodies, Protein. Expres. Purif. 52, 395-402.

(77) Hudig, D., and Bajaj, S. P. (1982) Tissue factor-like activity of the human monocytic tumor-cell line-u937, Thromb. Res. 27, 321-332.

(78) Felez, J., Miles, L. A., Plescia, J., and Plow, E. F. (1990) Regulation of plasminogen receptor expression on human monocytes and monocytoid cell-lines, J. Cell. Biol. 111, 1673-1683.

(79) Halfman, C. J. (1981) Concentrations of binding protein and labeled analyte that are appropriate for measuring at any analyte concentration range in radioimmunoassays, Methods Enzymol. 74, 481-497.

(80) Cheng, Y., and Prusoff, W. H. (1973) Relationship between inhibition constant (KI) and concentration of inhibitor which causes 50 per cent inhibition (I50) of an enzymatic-reaction, Biochem. Pharmacol. 22, 3099-3108.

(81) Craig, D. A. (1993) The Cheng-Prusoff relationship - something lost in the translation, Trends Pharmacol. Sci. 14, 89-91.

(82) Plow, E. F., Doeuvre, L., and Das, R. (2012) So many plasminogen receptors: why?, J. Biomed. Biotechnol. 2012:141806

(83) Parry, M. A. A., Fernandez-Catalan, C., Bergner, A., Huber, R., Hopfner, K. P., Schlott, B., Guhrs, K. H., and Bode, W. (1998) The ternary microplasmin-staphylokinase-microplasmin complex is a proteinase-cofactor-substrate complex in action, Nat. Struct. Biol. 5, 917-923.

(84) Mathews, I. I., VanderhoffHanaver, P., Castellino, F. J., and Tulinsky, A. (1996) Crystal structures of the recombinant kringle 1 domain of human plasminogen in complexes with the ligands epsilon-aminocaproic acid and trans-4-(aminomethyl)cyclohexane-1-carboxylic acid, Biochemistry 35, 2567-2576.

(85) Bajaj, M. S., Birktoft, J. J., Steer, S. A., and Bajaj, S. P. (2001) Structure and biology of tissue factor pathway inhibitor, Thromb. Haemostasis 86, 959-972.

(86) Eswar, N., Marti-Renom, M.A., Webb, B., Madhusudhan, M.S., Eramian, D., Shen, M., Pieper, U. and Sali, A. (2006) Comparative protein structure modeling with MODELLER. Current Protocols in Bioinformatics, John Wiley & Sons, Inc., Supplement 15, 5.6.1-5.6.30.

(87) Brooks, B. R., Bruccoleri, R. E., Olafson, B. D., States, D. J., Swaminathan, S., and Karplus, M. (1983) CHARMM - a program for macromolecular energy, minimization, and dynamics calculations, J. Comp. Chem. 4, 187-217.

(88) Bode, W., Turk, D., and Karshikov, A. (1992) The refined 1.9-Å x-ray crystal-structure of d-phe-pro-arg chloromethylketone-inhibited human $\alpha$-thrombin - structure-analysis, overall structure, electrostatic properties, detailed active-site geometry, and structure-function-relationships, Protein Sci. 1, 426-471.

(89) Chang, J. Y. (1985) Thrombin specificity - requirement for apolar amino-acids adjacent to the thrombin cleavage site of polypeptide substrate, Eur. J. Biochem. 151, 217-224.

(90) Markus, G., Depasquale, J. L., and Wissler, F. C. (1978) Quantitative-determination of binding of epsilon-aminocaproic acid to native plasminogen, J. Biol. Chem. 253, 727-732.

(91) Groh, J., Welte, M., Azad, S. C., and Kratzer, M. A. A. (1993) Monitoring of hemostasis during liver surgery, Infusionstherapie 20, 173-179.

(92) Schlag, M.G., Hopf, R. and Redl, H. (2000) Convulsive seizures following subdural application of fibrin sealant containing tranexamic acid in a rat model. Neurosurgery 47, 1463-1467.

(93) Lecker, I., Wang, D. S., Romaschin, A. D., Peterson, M., Mazer, C. D., and Orser, B. A. (2012) Tranexamic acid concentrations associated with human seizures inhibit glycine receptors, J. Clin. Invest. 122, 4654-4666.

(94) Makhija, N., Sarupria, A., Choudhary, S.K., Das, S., Lakshmy, R. and Kiran, U. (2013) Comparison of Epsilon Aminocaproic Acid and Tranexamic Acid in Thoracic Aortic Surgery: Clinical Efficacy and Safety. J. Cardiothorac. Vasc. Anesth. 27, 1201-1207.

(95) Manji, R.A., Grocott, H.P., Leake, J., Ariano, R.E., Manji, J.S., Menkis, A.H. and Jacobsohn, E. (2012) Seizures following cardiac surgery: the impact of tranexamic acid and other risk factors. Can. J. Anaesth. 59, 6-13.

(96) Kempaiah, P., Danielson, L. A., Barry, M., and Kisiel, W. (2009) Comparative effects of aprotinin and human recombinant R24K KD1 on temporal renal function in long-evans rats, J. Pharmacol. Exp. Ther. 331, 940-945.

SEQUENCE LISTING

<110> The Regents of the University of California
Bajaj, S. Paul

<120> DUAL REACTIVITY POTENT KUNITZ INHIBITOR OF FIBRINOLYSIS

<130> 720156.403WO

<140> US
<141> 2014-03-14

<150> US 61/922,026
<151> 2013-12-30

<150> US 61/793,131
<151> 2013-03-15

<160> 53

<170> FastSEQ for Windows Version 4.0

<210> 1
<211> 57

<212> PRT
<213> Artificial Sequence

<220>
<223> Engineered polypeptide

<220>
<221> VARIANT
<222> (11)...(11)
<223> Xaa = Tyr, Ser, Thr, Asn, or Asp

<220>
<221> VARIANT
<222> (17)...(17)
<223> Xaa = Lys, Arg, Ser or Thr

<400> 1

```
Asn Ala Glu Ile Cys Leu Leu Pro Leu Asp Xaa Gly Pro Cys Arg Ala
 1               5                  10                  15
Xaa Leu Leu Arg Tyr Tyr Tyr Asp Arg Tyr Thr Gln Ser Cys Arg Gln
              20                  25                  30
Phe Leu Tyr Gly Gly Cys Glu Gly Asn Ala Asn Asn Phe Tyr Thr Trp
              35                  40                  45
Glu Ala Cys Asp Asp Ala Cys Trp Arg
    50                  55
```

<210> 2
<211> 6
<212> PRT
<213> Artificial Sequence

<220>
<223> Engineered polypeptide

<220>
<221> VARIANT
<222> (2)...(2)
<223> Xaa = Ala, Asp, Glu, Gly, His, Ile, Lys, Leu, Met, Asn, Pro, Gln, Ser, Thr, Val or Tyr

<220>
<221> VARIANT
<222> (5)...(5)
<223> Xaa = Ala, Asp, Glu, Gly, His, Ile, Lys, Leu, Met, Asn, Pro, Gln, Ser, Thr, Val or Tyr

<400> 2

```
Ile Xaa Lys Val Xaa Lys
 1               5
```

<210> 3
<211> 60
<212> PRT
<213> Artificial Sequence

<220>
<223> Engineered polypeptide

44

<400> 3

```
Asn Ala Glu Ile Cys Leu Leu Pro Leu Asp Thr Gly Pro Cys Arg Ala
1               5                   10                  15
Arg Leu Leu Arg Tyr Tyr Tyr Asp Arg Tyr Thr Gln Ser Cys Arg Gln
            20                  25                  30
Phe Leu Tyr Gly Gly Cys Glu Gly Asn Ala Asn Asn Phe Tyr Thr Trp
            35                  40                  45
Glu Ala Cys Asp Asp Ala Cys Trp Arg Ile Glu Lys
        50                  55                  60
```

<210> 4
<211> 63
<212> PRT
<213> Artificial Sequence

<220>
<223> Engineered polypeptide

<400> 4

```
Asn Ala Glu Ile Cys Leu Leu Pro Leu Asp Thr Gly Pro Cys Arg Ala
1               5                   10                  15
Arg Leu Leu Arg Tyr Tyr Tyr Asp Arg Tyr Thr Gln Ser Cys Arg Gln
            20                  25                  30
Phe Leu Tyr Gly Gly Cys Glu Gly Asn Ala Asn Asn Phe Tyr Thr Trp
            35                  40                  45
Glu Ala Cys Asp Asp Ala Cys Trp Arg Ile Glu Lys Val Pro Lys
        50                  55                  60
```

<210> 5
<211> 60
<212> PRT
<213> Artificial Sequence

<220>
<223> Engineered polypeptide

<400> 5

```
Asn Ala Glu Ile Cys Leu Leu Pro Leu Asp Tyr Gly Pro Cys Arg Ala
1               5                   10                  15
Arg Leu Leu Arg Tyr Tyr Tyr Asp Arg Tyr Thr Gln Ser Cys Arg Gln
            20                  25                  30
Phe Leu Tyr Gly Gly Cys Glu Gly Asn Ala Asn Asn Phe Tyr Thr Trp
                35                  40                  45
Glu Ala Cys Asp Asp Ala Cys Trp Arg Ile Glu Lys
        50                  55                  60
```

<210> 6
<211> 63
<212> PRT
<213> Artificial Sequence

<220>
<223> Engineered polypeptide

<400> 6

```
Asn Ala Glu Ile Cys Leu Leu Pro Leu Asp Tyr Gly Pro Cys Arg Ala
1               5                   10                  15
Arg Leu Leu Arg Tyr Tyr Tyr Asp Arg Tyr Thr Gln Ser Cys Arg Gln
            20                  25                  30
Phe Leu Tyr Gly Gly Cys Glu Gly Asn Ala Asn Asn Phe Tyr Thr Trp
            35                  40                  45
Glu Ala Cys Asp Asp Ala Cys Trp Arg Ile Glu Lys Val Pro Lys
        50                  55                  60
```

<210> 7
<211> 61
<212> PRT
<213> Artificial Sequence

<220>
<223> Engineered polypeptide

<400> 7

```
Met Asn Ala Glu Ile Cys Leu Leu Pro Leu Asp Thr Gly Pro Cys Arg
1               5                   10                  15
Ala Arg Leu Leu Arg Tyr Tyr Tyr Asp Arg Tyr Thr Gln Ser Cys Arg
            20                  25                  30
Gln Phe Leu Tyr Gly Gly Cys Glu Gly Asn Ala Asn Asn Phe Tyr Thr
            35                  40                  45
Trp Glu Ala Cys Asp Asp Ala Cys Trp Arg Ile Glu Lys
        50                  55                  60
```

<210> 8
<211> 64
<212> PRT
<213> Artificial Sequence

<220>
<223> Engineered polypeptide

<400> 8

```
Met Asn Ala Glu Ile Cys Leu Leu Pro Leu Asp Thr Gly Pro Cys Arg
1               5                   10                  15
Ala Arg Leu Leu Arg Tyr Tyr Tyr Asp Arg Tyr Thr Gln Ser Cys Arg
            20                  25                  30
Gln Phe Leu Tyr Gly Gly Cys Glu Gly Asn Ala Asn Asn Phe Tyr Thr
            35                  40                  45
Trp Glu Ala Cys Asp Asp Ala Cys Trp Arg Ile Glu Lys Val Pro Lys
        50                  55                  60
```

<210> 9
<211> 61
<212> PRT
<213> Artificial Sequence

<220>
<223> Engineered polypeptide

<400> 9

```
Met Asn Ala Glu Ile Cys Leu Leu Pro Leu Asp Tyr Gly Pro Cys Arg
1               5                   10                  15
Ala Arg Leu Leu Arg Tyr Tyr Tyr Asp Arg Tyr Thr Gln Ser Cys Arg
            20                  25                  30
Gln Phe Leu Tyr Gly Gly Cys Glu Gly Asn Ala Asn Asn Phe Tyr Thr
            35                  40                  45
Trp Glu Ala Cys Asp Asp Ala Cys Trp Arg Ile Glu Lys
    50                  55                  60
```

<210> 10
<211> 64
<212> PRT
<213> Artificial Sequence

<220>
<223> Engineered polypeptide

<400> 10

```
Met Asn Ala Glu Ile Cys Leu Leu Pro Leu Asp Tyr Gly Pro Cys Arg
1               5                   10                  15
Ala Arg Leu Leu Arg Tyr Tyr Tyr Asp Arg Tyr Thr Gln Ser Cys Arg
            20                  25                  30
Gln Phe Leu Tyr Gly Gly Cys Glu Gly Asn Ala Asn Asn Phe Tyr Thr
            35                  40                  45
Trp Glu Ala Cys Asp Asp Ala Cys Trp Arg Ile Glu Lys Val Pro Lys
    50                  55                  60
```

<210> 11
<211> 63
<212> PRT
<213> Artificial Sequence

<220>
<223> Engineered polypeptide

<220>
<221> VARIANT
<222> (59)...(59)
<223> Xaa = Ala, Asp, Glu, Gly, His, Ile, Lys, Leu, Met, Asn, Pro, Gln, Ser, Thr, Val or Tyr

<220>
<221> VARIANT
<222> (62)...(62)
<223> Xaa = Ala, Asp, Glu, Gly, His, Ile, Lys, Leu, Met, Asn, Pro, Gln, Ser, Thr, Val or Tyr

<400> 11

```
Asn Ala Glu Ile Cys Leu Leu Pro Leu Asp Thr Gly Pro Cys Arg Ala
1               5                   10                  15
Arg Leu Leu Arg Tyr Tyr Tyr Asp Arg Tyr Thr Gln Ser Cys Arg Gln
            20                  25                  30
Phe Leu Tyr Gly Gly Cys Glu Gly Asn Ala Asn Asn Phe Tyr Thr Trp
            35                  40                  45
Glu Ala Cys Asp Asp Ala Cys Trp Arg Ile Xaa Lys Val Xaa Lys
    50                  55                  60
```

<210> 12
```

<211> 63
<212> PRT
<213> Artificial Sequence

<220>
<223> Engineered polypeptide

<220>
<221> VARIANT
<222> (59)...(59)
<223> Xaa = Ala, Asp, Glu, Gly, His, Ile, Lys, Leu, Met, Asn, Pro, Gln, Ser, Thr, Val or Tyr

<220>
<221> VARIANT
<222> (62)...(62)
<223> Xaa = Ala, Asp, Glu, Gly, His, Ile, Lys, Leu, Met, Asn, Pro, Gln, Ser, Thr, Val or Tyr

<400> 12

```
Asn Ala Glu Ile Cys Leu Leu Pro Leu Asp Tyr Gly Pro Cys Arg Ala
1               5                   10                  15
Arg Leu Leu Arg Tyr Tyr Tyr Asp Arg Tyr Thr Gln Ser Cys Arg Gln
            20                  25                  30
Phe Leu Tyr Gly Gly Cys Glu Gly Asn Ala Asn Asn Phe Tyr Thr Trp
            35                  40                  45
Glu Ala Cys Asp Asp Ala Cys Trp Arg Ile Xaa Lys Val Xaa Lys
    50                  55                  60
```

<210> 13
<211> 64
<212> PRT
<213> Artificial Sequence

<220>
<223> Engineered polypeptide

<220>
<221> VARIANT
<222> (60)...(60)
<223> Xaa = Ala, Asp, Glu, Gly, His, Ile, Lys, Leu, Met, Asn, Pro, Gln, Ser, Thr, Val or Tyr

<220>
<221> VARIANT
<222> (63)...(63)
<223> Xaa = Ala, Asp, Glu, Gly, His, Ile, Lys, Leu, Met, Asn, Pro, Gln, Ser, Thr, Val or Tyr

<400> 13

```
Met Asn Ala Glu Ile Cys Leu Leu Pro Leu Asp Thr Gly Pro Cys Arg
1               5                   10                  15
Ala Arg Leu Leu Arg Tyr Tyr Tyr Asp Arg Tyr Thr Gln Ser Cys Arg
            20                  25                  30
Gln Phe Leu Tyr Gly Gly Cys Glu Gly Asn Ala Asn Asn Phe Tyr Thr
            35                  40                  45
Trp Glu Ala Cys Asp Asp Ala Cys Trp Arg Ile Xaa Lys Val Xaa Lys
    50                  55                  60
```

<210> 14

<211> 64
<212> PRT
<213> Artificial Sequence

<220>
<223> Engineered polypeptide

<220>
<221> VARIANT
<222> (60)...(60)
<223> Xaa = Ala, Asp, Glu, Gly, His, Ile, Lys, Leu, Met, Asn, Pro, Gln, Ser, Thr, Val or Tyr

<220>
<221> VARIANT
<222> (63)...(63)
<223> Xaa = Ala, Asp, Glu, Gly, His, Ile, Lys, Leu, Met, Asn, Pro, Gln, Ser, Thr, Val or Tyr

<400> 14

```
Met Asn Ala Glu Ile Cys Leu Leu Pro Leu Asp Tyr Gly Pro Cys Arg
1               5               10              15
Ala Arg Leu Leu Arg Tyr Tyr Tyr Asp Arg Tyr Thr Gln Ser Cys Arg
            20              25              30
Gln Phe Leu Tyr Gly Gly Cys Glu Gly Asn Ala Asn Asn Phe Tyr Thr
            35              40              45
Trp Glu Ala Cys Asp Asp Ala Cys Trp Arg Ile Xaa Lys Val Xaa Lys
    50              55              60
```

<210> 15
<211> 60
<212> PRT
<213> Artificial Sequence

<220>
<223> Engineered polypeptide

<220>
<221> VARIANT
<222> (59)...(59)
<223> Xaa = Ala, Asp, Glu, Gly, His, Ile, Lys, Leu, Met, Asn, Pro, Gln, Ser, Thr, Val or Tyr

<400> 15

```
Asn Ala Glu Ile Cys Leu Leu Pro Leu Asp Thr Gly Pro Cys Arg Ala
1               5               10              15
Arg Leu Leu Arg Tyr Tyr Tyr Asp Arg Tyr Thr Gln Ser Cys Arg Gln
            20              25              30
Phe Leu Tyr Gly Gly Cys Glu Gly Asn Ala Asn Asn Phe Tyr Thr Trp
            35              40              45
Glu Ala Cys Asp Asp Ala Cys Trp Arg Ile Xaa Lys
    50              55              60
```

<210> 16
<211> 60
<212> PRT
<213> Artificial Sequence

<220>

<223> Engineered polypeptide

<220>
<221> VARIANT
<222> (59)...(59)
<223> Xaa = Ala, Asp, Glu, Gly, His, Ile, Lys, Leu, Met, Asn, Pro, Gln, Ser, Thr, Val or Tyr

<400> 16

```
Asn Ala Glu Ile Cys Leu Leu Pro Leu Asp Tyr Gly Pro Cys Arg Ala
 1               5                  10                  15
Arg Leu Leu Arg Tyr Tyr Tyr Asp Arg Tyr Thr Gln Ser Cys Arg Gln
            20                  25                  30
Phe Leu Tyr Gly Gly Cys Glu Gly Asn Ala Asn Asn Phe Tyr Thr Trp
        35                  40                  45
Glu Ala Cys Asp Asp Ala Cys Trp Arg Ile Xaa Lys
    50                  55                  60
```

<210> 17
<211> 61
<212> PRT
<213> Artificial Sequence

<220>
<223> Engineered polypeptide

<220>
<221> VARIANT
<222> (60)...(60)
<223> Xaa = Ala, Asp, Glu, Gly, His, Ile, Lys, Leu, Met, Asn, Pro, Gln, Ser, Thr, Val or Tyr

<400> 17

```
Met Asn Ala Glu Ile Cys Leu Leu Pro Leu Asp Thr Gly Pro Cys Arg
 1               5                  10                  15
Ala Arg Leu Leu Arg Tyr Tyr Tyr Asp Arg Tyr Thr Gln Ser Cys Arg
            20                  25                  30
Gln Phe Leu Tyr Gly Gly Cys Glu Gly Asn Ala Asn Asn Phe Tyr Thr
        35                  40                  45
Trp Glu Ala Cys Asp Asp Ala Cys Trp Arg Ile Xaa Lys
    50                  55                  60
```

<210> 18
<211> 61
<212> PRT
<213> Artificial Sequence

<220>
<223> Engineered polypeptide

<220>
<221> VARIANT
<222> (60)...(60)
<223> Xaa = Ala, Asp, Glu, Gly, His, Ile, Lys, Leu, Met, Asn, Pro, Gln, Ser, Thr, Val or Tyr

<400> 18

```
Met Asn Ala Glu Ile Cys Leu Leu Pro Leu Asp Tyr Gly Pro Cys Arg
1               5               10              15
Ala Arg Leu Leu Arg Tyr Tyr Tyr Asp Arg Tyr Thr Gln Ser Cys Arg
        20              25              30
Gln Phe Leu Tyr Gly Gly Cys Glu Gly Asn Ala Asn Asn Phe Tyr Thr
        35              40              45
Trp Glu Ala Cys Asp Asp Ala Cys Trp Arg Ile Xaa Lys
        50              55              60
```

<210> 19
<211> 58
<212> PRT
<213> Artificial Sequence

<220>
<223> Engineered polypeptide

<400> 19

```
Asn Ala Glu Ile Cys Leu Leu Pro Leu Asp Tyr Gly Pro Cys Arg Ala
1               5               10              15
Leu Leu Leu Arg Tyr Tyr Tyr Asp Arg Tyr Thr Gln Ser Cys Arg Gln
        20              25              30
Phe Leu Tyr Gly Gly Cys Glu Gly Asn Ala Asn Asn Phe Tyr Thr Trp
        35              40              45
Glu Ala Cys Asp Asp Ala Cys Trp Arg Ile
        50              55
```

<210> 20
<211> 5
<212> PRT
<213> Artificial Sequence

<220>
<223> Engineered polypeptide

<220>
<221> VARIANT
<222> (2)...(2)
<223> Xaa = Ala, Asp, Glu, Gly, His, Ile, Lys, Leu, Met, Asn, Pro, Gln, Ser, Thr, Val or Tyr

<220>
<221> VARIANT
<222> (5)...(5)
<223> Xaa = Ala, Asp, Glu, Gly, His, Ile, Lys, Leu, Met, Asn, Pro, Gln, Ser, Thr, Val or Tyr

<400> 20

```
Ile Xaa Lys Val Xaa
1               5
```

<210> 21
<211> 4
<212> PRT
<213> Artificial Sequence

<220>
<223> Engineered polypeptide

<220>
<221> VARIANT
<222> (2)...(2)
<223> Xaa = Ala, Asp, Glu, Gly, His, Ile, Lys, Leu, Met, Asn, Pro, Gln, Ser, Thr, Val or Tyr

<400> 21

```
                    Ile Xaa Lys Val
                     1
```

<210> 22
<211> 61
<212> PRT
<213> Artificial Sequence

<220>
<223> Engineered polypeptide

<220>
<221> VARIANT
<222> (59)...(59)
<223> Xaa = Ala, Asp, Glu, Gly, His, Ile, Lys, Leu, Met, Asn, Pro, Gln, Ser, Thr, Val or Tyr

<400> 22

```
        Asn Ala Glu Ile Cys Leu Leu Pro Leu Asp Tyr Gly Pro Cys Arg Ala
         1               5                   10                  15
        Arg Leu Leu Arg Tyr Tyr Tyr Asp Arg Tyr Thr Gln Ser Cys Arg Gln
                     20                  25                  30
        Phe Leu Tyr Gly Gly Cys Glu Gly Asn Ala Asn Asn Phe Tyr Thr Trp
                     35                  40                  45
        Glu Ala Cys Asp Asp Ala Cys Trp Arg Ile Xaa Lys Val
             50                  55                  60
```

<210> 23
<211> 62
<212> PRT
<213> Artificial Sequence

<220>
<223> Engineered polypeptide

<220>
<221> VARIANT
<222> (59)...(59)
<223> Xaa = Ala, Asp, Glu, Gly, His, Ile, Lys, Leu, Met, Asn, Pro, Gln, Ser, Thr, Val or Tyr

<220>
<221> VARIANT
<222> (62)...(62)
<223> Xaa = Ala, Asp, Glu, Gly, His, Ile, Lys, Leu, Met, Asn, Pro, Gln, Ser, Thr, Val or Tyr

<400> 23

```
Asn Ala Glu Ile Cys Leu Leu Pro Leu Asp Tyr Gly Pro Cys Arg Ala
 1               5                   10                  15
Arg Leu Leu Arg Tyr Tyr Tyr Asp Arg Tyr Thr Gln Ser Cys Arg Gln
            20                  25                  30
Phe Leu Tyr Gly Gly Cys Glu Gly Asn Ala Asn Asn Phe Tyr Thr Trp
            35                  40                  45
Glu Ala Cys Asp Asp Ala Cys Trp Arg Ile Xaa Lys Val Xaa
        50                  55                  60
```

<210> 24
<211> 61
<212> PRT
<213> Artificial Sequence

<220>
<223> Engineered polypeptide

<220>
<221> VARIANT
<222> (59)...(59)
<223> Xaa = Ala, Asp, Glu, Gly, His, Ile, Lys, Leu, Met, Asn, Pro, Gln, Ser, Thr, Val or Tyr

<400> 24

```
Asn Ala Glu Ile Cys Leu Leu Pro Leu Asp Thr Gly Pro Cys Arg Ala
 1               5                   10                  15

Arg Leu Leu Arg Tyr Tyr Tyr Asp Arg Tyr Thr Gln Ser Cys Arg Gln
            20                  25                  30
Phe Leu Tyr Gly Gly Cys Glu Gly Asn Ala Asn Asn Phe Tyr Thr Trp
            35                  40                  45
Glu Ala Cys Asp Asp Ala Cys Trp Arg Ile Xaa Lys Val
        50                  55                  60
```

<210> 25
<211> 62
<212> PRT
<213> Artificial Sequence

<220>
<223> Engineered polypeptide

<220>
<221> VARIANT
<222> (59)...(59)
<223> Xaa = Ala, Asp, Glu, Gly, His, Ile, Lys, Leu, Met, Asn, Pro, Gln, Ser, Thr, Val or Tyr

<220>
<221> VARIANT
<222> (62)...(62)
<223> Xaa = Ala, Asp, Glu, Gly, His, Ile, Lys, Leu, Met, Asn, Pro, Gln, Ser, Thr, Val or Tyr

<400> 25

```
        Asn Ala Glu Ile Cys Leu Leu Pro Leu Asp Thr Gly Pro Cys Arg Ala
        1               5                   10                  15
        Arg Leu Leu Arg Tyr Tyr Tyr Asp Arg Tyr Thr Gln Ser Cys Arg Gln
                    20                  25                  30
        Phe Leu Tyr Gly Gly Cys Glu Gly Asn Ala Asn Asn Phe Tyr Thr Trp
                    35                  40                  45
        Glu Ala Cys Asp Asp Ala Cys Trp Arg Ile Xaa Lys Val Xaa
            50                  55                  60
```

<210> 26
<211> 62
<212> PRT
<213> Artificial Sequence

<220>
<223> Engineered polypeptide

<220>
<221> VARIANT
<222> (60)...(60)
<223> Xaa = Ala, Asp, Glu, Gly, His, Ile, Lys, Leu, Met, Asn, Pro, Gln, Ser, Thr, Val or Tyr

<400> 26

```
        Met Asn Ala Glu Ile Cys Leu Leu Pro Leu Asp Tyr Gly Pro Cys Arg
        1               5                   10                  15
        Ala Arg Leu Leu Arg Tyr Tyr Tyr Asp Arg Tyr Thr Gln Ser Cys Arg
                    20                  25                  30
        Gln Phe Leu Tyr Gly Gly Cys Glu Gly Asn Ala Asn Asn Phe Tyr Thr
                    35                  40                  45
        Trp Glu Ala Cys Asp Asp Ala Cys Trp Arg Ile Xaa Lys Val
            50                  55                  60
```

<210> 27
<211> 63
<212> PRT
<213> Artificial Sequence

<220>
<223> Engineered polypeptide

<220>
<221> VARIANT
<222> (60)...(60)
<223> Xaa = Ala, Asp, Glu, Gly, His, Ile, Lys, Leu, Met, Asn, Pro, Gln, Ser, Thr, Val or Tyr

<220>
<221> VARIANT
<222> (63)...(63)
<223> Xaa = Ala, Asp, Glu, Gly, His, Ile, Lys, Leu, Met, Asn, Pro, Gln, Ser, Thr, Val or Tyr

<400> 27

```
Met Asn Ala Glu Ile Cys Leu Leu Pro Leu Asp Tyr Gly Pro Cys Arg
1               5                   10                  15
Ala Arg Leu Leu Arg Tyr Tyr Tyr Asp Arg Tyr Thr Gln Ser Cys Arg
            20              25                  30
Gln Phe Leu Tyr Gly Gly Cys Glu Gly Asn Ala Asn Asn Phe Tyr Thr
            35              40                  45
Trp Glu Ala Cys Asp Asp Ala Cys Trp Arg Ile Xaa Lys Val Xaa
        50              55                  60
```

<210> 28
<211> 62
<212> PRT
<213> Artificial Sequence

<220>
<223> Engineered polypeptide

<220>
<221> VARIANT
<222> (60)...(60)
<223> Xaa = Ala, Asp, Glu, Gly, His, Ile, Lys, Leu, Met, Asn, Pro, Gln, Ser, Thr, Val or Tyr

<400> 28

```
Met Asn Ala Glu Ile Cys Leu Leu Pro Leu Asp Thr Gly Pro Cys Arg
1               5                   10                  15
Ala Arg Leu Leu Arg Tyr Tyr Tyr Asp Arg Tyr Thr Gln Ser Cys Arg
            20              25                  30
Gln Phe Leu Tyr Gly Gly Cys Glu Gly Asn Ala Asn Asn Phe Tyr Thr
            35              40                  45
Trp Glu Ala Cys Asp Asp Ala Cys Trp Arg Ile Xaa Lys Val
        50              55                  60
```

<210> 29
<211> 63
<212> PRT
<213> Artificial Sequence

<220>
<223> Engineered polypeptide

<220>
<221> VARIANT
<222> (60)...(60)
<223> Xaa = Ala, Asp, Glu, Gly, His, Ile, Lys, Leu, Met, Asn, Pro, Gln, Ser, Thr, Val or Tyr

<220>
<221> VARIANT
<222> (63)...(63)
<223> Xaa = Ala, Asp, Glu, Gly, His, Ile, Lys, Leu, Met, Asn, Pro, Gln, Ser, Thr, Val or Tyr

<400> 29

```
Met Asn Ala Glu Ile Cys Leu Leu Pro Leu Asp Thr Gly Pro Cys Arg
 1               5                   10                  15
Ala Arg Leu Leu Arg Tyr Tyr Tyr Asp Arg Tyr Thr Gln Ser Cys Arg
         20              25                  30
Gln Phe Leu Tyr Gly Gly Cys Glu Gly Asn Ala Asn Asn Phe Tyr Thr
         35                  40                  45
Trp Glu Ala Cys Asp Asp Ala Cys Trp Arg Ile Xaa Lys Val Xaa
    50                  55                  60
```

<210> 30
<211> 4
<212> PRT
<213> Artificial Sequence

<220>
<223> Engineered polypeptide

<220>
<221> VARIANT
<222> (2)...(2)
<223> Xaa = Ala, Asp, Glu, Gly, His, Ile, Lys, Leu, Met, Asn, Pro, Gln, Ser, Thr, Val or Tyr

<220>
<221> VARIANT
<222> (4)...(4)
<223> Xaa = any amino acid

<400> 30

```
Ile Xaa Lys Xaa
 1
```

<210> 31
<211> 5
<212> PRT
<213> Artificial Sequence

<220>
<223> Engineered polypeptide

<220>
<221> VARIANT
<222> (2)...(2)
<223> Xaa = Ala, Asp, Glu, Gly, His, Ile, Lys, Leu, Met, Asn, Pro, Gln, Ser, Thr, Val or Tyr

<220>
<221> VARIANT
<222> (5)...(5)
<223> Xaa = any amino acid

<220>
<221> VARIANT
<222> (6)...(6)
<223> Xaa = any amino acid

<400> 31

```
Ile Xaa Lys Xaa Xaa
1               5
```

<210> 32
<211> 61
<212> PRT
<213> Artificial Sequence

<220>
<223> Engineered polypeptide

<220>
<221> VARIANT
<222> (11)...(11)
<223> Xaa = Tyr, Ser, Thr, Asn, or Asp

<220>
<221> VARIANT
<222> (17)...(17)
<223> Xaa = Lys, Arg, Ser or Thr

<220>
<221> VARIANT
<222> (59)...(59)
<223> Xaa = Ala, Asp, Glu, Gly, His, Ile, Lys, Leu, Met, Asn, Pro, Gln, Ser, Thr, Val or Tyr

<220>
<221> VARIANT
<222> (61)...(61)
<223> Xaa = any amino acid

<400> 32

```
Asn Ala Glu Ile Cys Leu Leu Pro Leu Asp Xaa Gly Pro Cys Arg Ala
1               5                   10                  15
Xaa Leu Leu Arg Tyr Tyr Tyr Asp Arg Tyr Thr Gln Ser Cys Arg Gln
            20                  25                  30
Phe Leu Tyr Gly Gly Cys Glu Gly Asn Ala Asn Asn Phe Tyr Thr Trp
            35                  40                  45
Glu Ala Cys Asp Asp Ala Cys Trp Arg Ile Xaa Lys Xaa
            50                  55                  60
```

<210> 33
<211> 62
<212> PRT
<213> Artificial Sequence

<220>
<223> Engineered polypeptide

<220>
<221> VARIANT
<222> (11)...(11)
<223> Xaa = Tyr, Ser, Thr, Asn, or Asp

<220>
<221> VARIANT
<222> (17)...(17)

<223> Xaa = Lys, Arg, Ser or Thr

<220>
<221> VARIANT
<222> (59)...(59)
<223> Xaa = Ala, Asp, Glu, Gly, His, Ile, Lys, Leu, Met, Asn, Pro, Gln, Ser, Thr, Val or Tyr

<220>
<221> VARIANT
<222> (61)...(61)
<223> Xaa = Any amino acid

<220>
<221> VARIANT
<222> (62)...(62)
<223> Xaa = any amino acid

<400> 33

```
Asn Ala Glu Ile Cys Leu Leu Pro Leu Asp Xaa Gly Pro Cys Arg Ala
 1               5               10                  15
Xaa Leu Leu Arg Tyr Tyr Tyr Asp Arg Tyr Thr Gln Ser Cys Arg Gln
         20              25              30
Phe Leu Tyr Gly Gly Cys Glu Gly Asn Ala Asn Asn Phe Tyr Thr Trp
         35              40              45
Glu Ala Cys Asp Asp Ala Cys Trp Arg Ile Xaa Lys Xaa Xaa
    50              55              60
```

<210> 34
<211> 62
<212> PRT
<213> Artificial Sequence

<220>
<223> Engineered polypeptide

<220>
<221> VARIANT
<222> (12)...(12)
<223> Xaa = Tyr, Ser, Thr, Asn, or Asp

<220>
<221> VARIANT
<222> (18)...(18)
<223> Xaa = Lys, Arg, Ser or Thr

<220>
<221> VARIANT
<222> (60)...(60)
<223> Xaa = Ala, Asp, Glu, Gly, His, Ile, Lys, Leu, Met, Asn, Pro, Gln, Ser, Thr, Val or Tyr

<220>
<221> VARIANT
<222> (62)...(62)
<223> Xaa = any amino acid

<400> 34

```
        Met Asn Ala Glu Ile Cys Leu Leu Pro Leu Asp Xaa Gly Pro Cys Arg
        1               5                   10                  15
        Ala Xaa Leu Leu Arg Tyr Tyr Tyr Asp Arg Tyr Thr Gln Ser Cys Arg
                20                  25                  30
        Gln Phe Leu Tyr Gly Gly Cys Glu Gly Asn Ala Asn Asn Phe Tyr Thr
                35                  40                  45


        Trp Glu Ala Cys Asp Asp Ala Cys Trp Arg Ile Xaa Lys Xaa
            50                  55                  60
```

<210> 35
<211> 63
<212> PRT
<213> Artificial Sequence

<220>
<223> Engineered polypeptide

<220>
<221> VARIANT
<222> (12)...(12)
<223> Xaa = Tyr, Ser, Thr, Asn or Asp

<220>
<221> VARIANT
<222> (18)...(18)
<223> Xaa = Lys, Arg, Ser or Thr

<220>
<221> VARIANT
<222> (60)...(60)
<223> Xaa = Ala, Asp, Glu, Gly, His, Ile, Lys, Leu, Met, Asn, Pro, Gln, Ser, Thr, Val or Tyr

<220>
<221> VARIANT
<222> (62)...(62)
<223> Xaa = any amino acid

<220>
<221> VARIANT
<222> (63)...(63)
<223> Xaa = any amino acid

<400> 35

```
        Met Asn Ala Glu Ile Cys Leu Leu Pro Leu Asp Xaa Gly Pro Cys Arg
        1               5                   10                  15
        Ala Xaa Leu Leu Arg Tyr Tyr Tyr Asp Arg Tyr Thr Gln Ser Cys Arg
                20                  25                  30
        Gln Phe Leu Tyr Gly Gly Cys Glu Gly Asn Ala Asn Asn Phe Tyr Thr
                35                  40                  45
        Trp Glu Ala Cys Asp Asp Ala Cys Trp Arg Ile Xaa Lys Xaa Xaa
            50                  55                  60
```

<210> 36
<211> 14
<212> PRT
<213> Artificial Sequence

<220>
<223> Illustrative spacer

<400> 36

```
        Glu Gly Lys Ser Ser Gly Ser Gly Ser Glu Ser Lys Val Asp
         1               5                  10
```

<210> 37
<211> 18
<212> PRT
<213> Artificial Sequence

<220>
<223> Illustrative spacer

<400> 37

```
    Lys Glu Ser Gly Ser Val Ser Ser Glu Gln Leu Ala Gln Phe Arg Ser
     1               5                  10                  15
    Leu Asp
```

<210> 38
<211> 5
<212> PRT
<213> Artificial Sequence

<220>
<223> Flexible polylinker

<400> 38

```
                    Gly Gly Gly Gly Ser
                     1               5
```

<210> 39
<211> 6
<212> PRT
<213> Artificial Sequence

<220>
<223> Engineered polypeptide

<400> 39

```
                Ile Glu Lys Val Pro Lys
                 1               5
```

<210> 40
<211> 5
<212> PRT
<213> Artificial Sequence

<220>
<223> Engineered polypeptide

<400> 40

```
                         Ile Glu Lys Val Pro
                         1                 5
```

<210> 41
<211> 4
<212> PRT
<213> Artificial Sequence

<220>
<223> Engineered polypeptide

<400> 41

```
                            Ile Glu Lys Val
                            1
```

<210> 42
<211> 12
<212> PRT
<213> Artificial Sequence

<220>
<223> Engineered polypeptide

<400> 42

```
            Asp Ala Cys Trp Arg Ile Glu Lys Val Pro Lys Val
            1               5                 10
```

<210> 43
<211> 12
<212> PRT
<213> Artificial Sequence

<220>
<223> Engineered polypeptide

<400> 43

```
            Asp Ala Ala Gln Glu Pro Thr Gly Asn Asn Ala Glu
            1               5                 10
```

<210> 44
<211> 6
<212> PRT
<213> Artificial Sequence

<220>
<223> Engineered polypeptide

<400> 44

```
                      Glu Lys Val Pro Lys Val
                      1               5
```

<210> 45
<211> 58
<212> PRT

<213> Bovine sp.

<400> 45

```
        Arg Pro Asp Phe Cys Leu Glu Pro Pro Tyr Thr Gly Pro Cys Lys Ala
        1               5                   10                  15
        Arg Ile Ile Arg Tyr Phe Tyr Asn Ala Lys Ala Gly Leu Cys Gln Thr
                    20                  25                  30
        Phe Val Tyr Gly Gly Cys Arg Ala Lys Arg Asn Asn Phe Lys Ser Ala
                    35                  40                  45
        Glu Asp Cys Met Arg Thr Cys Gly Gly Ala
            50                  55
```

<210> 46
<211> 77
<212> PRT
<213> Artificial Sequence

<220>
<223> Engineered polypeptide

<400> 46

```
        Gly Ser His Met Asp Ala Ala Gln Glu Pro Thr Gly Asn Asn Ala Glu
        1               5                   10                  15
        Ile Cys Leu Leu Pro Leu Asp Tyr Gly Pro Cys Arg Ala Arg Leu Leu
                    20                  25                  30
        Arg Tyr Tyr Tyr Asp Arg Tyr Thr Gln Ser Cys Arg Gln Phe Leu Tyr
                    35                  40                  45
        Gly Gly Cys Glu Gly Asn Ala Asn Asn Phe Tyr Thr Trp Glu Ala Cys

                    50                  55                  60
        Asp Asp Ala Cys Trp Arg Ile Glu Lys Val Pro Lys Val
            65                  70                  75
```

<210> 47
<211> 65
<212> PRT
<213> Artificial Sequence

<220>
<223> Engineered polypeptide

<400> 47

```
        Gly Asn Asn Ala Glu Ile Cys Leu Leu Pro Leu Asp Tyr Gly Pro Cys
        1               5                   10                  15
        Arg Ala Arg Leu Leu Arg Tyr Tyr Tyr Asp Arg Tyr Thr Gln Ser Cys
                    20                  25                  30
        Arg Gln Phe Leu Tyr Gly Gly Cys Glu Gly Asn Ala Asn Asn Phe Tyr
                    35                  40                  45
        Thr Trp Glu Ala Cys Asp Asp Ala Cys Trp Arg Ile Glu Lys Val Pro
            50                  55                  60
        Lys
        65
```

<210> 48
<211> 63

<212> PRT
<213> Artificial Sequence

<220>
<223> Engineered polypeptide

<400> 48

```
Asn Ala Glu Ile Cys Leu Leu Pro Leu Asp Tyr Gly Pro Cys Arg Ala
1               5                   10                  15
Arg Leu Leu Arg Tyr Tyr Tyr Asp Arg Tyr Thr Gln Ser Cys Arg Gln
            20                  25                  30
Phe Leu Tyr Gly Gly Cys Glu Gly Asn Ala Asn Asn Phe Tyr Thr Trp
            35                  40                  45
Glu Ala Cys Asp Asp Ala Cys Trp Arg Ile Glu Lys Val Pro Lys
        50                  55                  60
```

<210> 49
<211> 6
<212> PRT
<213> Artificial Sequence

<220>
<223> Engineered polypeptide

<400> 49

```
Gly Asn Asn Ala Glu Ile
1               5
```

<210> 50
<211> 4
<212> PRT
<213> Artificial Sequence

<220>
<223> Engineered polypeptide

<400> 50

```
Asn Ala Glu Ile
1
```

<210> 51
<211> 5
<212> PRT
<213> Artificial Sequence

<220>
<223> Engineered polypeptide

<400> 51

```
Glu Lys Val Pro Lys
1               5
```

<210> 52
<211> 76

<212> PRT
<213> Artificial Sequence

<220>
<223> Engineered polypeptide

<400> 52

```
Gly Ser His Met Asp Ala Ala Gln Glu Pro Thr Gly Asn Asn Ala Glu
 1               5                  10                  15
Ile Cys Leu Leu Pro Leu Asp Tyr Gly Pro Cys Arg Ala Arg Leu Leu
             20                  25                  30
Arg Tyr Tyr Tyr Asp Arg Tyr Thr Gln Ser Cys Arg Gln Phe Leu Tyr
         35                  40                  45
Gly Gly Cys Glu Gly Asn Ala Asn Asn Phe Tyr Thr Trp Glu Ala Cys
     50                  55                  60
Asp Asp Ala Cys Trp Arg Ile Glu Lys Val Pro Lys
65                  70                  75
```

<210> 53
<211> 10
<212> PRT
<213> Artificial Sequence

<220>
<223> Engineered polypeptide

<400> 53

```
Gly Ser His Met Asp Ala Ala Gln Glu Pro
 1               5                  10
```

**Claims**

1. A plasmin-inhibiting polypeptide, comprising a polypeptide of general formula (I):

    N-Y-J-Z-C           (I)

    wherein:

    (a) N is an amino terminus of the plasmin-inhibiting polypeptide and consists of 0, 1, 2, 3, 4, 5, 6, 7, 8, 9, 10, 11, 12, 13, 14, 15, 16, 17, 18, 19, 20, 21, 22, 23, 24, 25-30, 31-40, 41-50, or 51-60 amino acids that are independently selected from natural and non-natural amino acids,
    (b) Y is either nothing or methionine or N-formylmethionine,
    (c) J is a Kunitz-type proteinase first inhibitor domain (KD1) polypeptide of 57 amino acids having the amino acid sequence set forth in SEQ ID NO:1,
    (d) Z is a KD1 carboxy-region polypeptide that is selected from:

        (i) a KD1 carboxy-region polypeptide of general formula (III):

            I-X3-K           (III)

        in which X3 is an amino acid selected from natural and non-natural amino acids and is not C, F, R or W,
        (ii) a KD1 carboxy-region polypeptide of general formula (II):

            I-X3-K-V-X4-K           (II) [SEQ ID NO:2]

in which X3 and X4 are amino acids independently selected from natural and non-natural amino acids and are not C, F, R or W,

(iii) a KD1 carboxy-region polypeptide of general formula (IV):

I-X3-K-V-X5          (IV) [SEQ ID NO:20]

in which X3 and X5 are independent, X3 is an amino acid selected from natural and non-natural amino acids and is not C, F, R or W, and X5 is an amino acid selected from natural and non-natural amino acids and is not C, F, R or W,

(iv) a KD1 carboxy-region polypeptide of general formula (V):

I-X3-K-V          (V) [SEQ ID NO:21]

in which X3 is an amino acid selected from natural and non-natural amino acids and is not C, F, R or W,

(v) a KD1 carboxy-region polypeptide of general formula (VI):

I-X3-K-X6          (VI) [SEQ ID NO:30]

in which X3 and X6 are amino acids independently selected from natural and non-natural amino acids, X3 is not C, F, R or W, and X6 is any natural or non-natural amino acid, and

(vi) a KD1 carboxy-region polypeptide of general formula (VII):

I-X3-K-X6-X7          (VII) [SEQ ID NO:31]

in which X3, X6 and X7 are amino acids independently selected from natural and non-natural amino acids, X3 is not C, F, R or W, X6 is any natural or non-natural amino acid, and X7 is any natural or non-natural amino acid,

(e) C is a carboxy terminus of the plasmin-inhibiting polypeptide and consists of 0 amino acids, and

(f) the plasmin-inhibiting polypeptide inhibits plasmin activity, and wherein the plasmin-inhibiting polypeptide has decreased anti-coagulation activity compared to a wild type human tissue factor pathway inhibitor-2 (TFPI-2) polypeptide first Kunitz-type proteinase inhibitor domain (KD1) having the amino acid sequence set forth in SEQ ID NO:19.

2.   The plasmin-inhibiting polypeptide of claim 1 that is selected from:

(a) the plasmin-inhibiting polypeptide in which N, Y and C are each nothing, J is the KD1 polypeptide having the amino acid sequence set forth in SEQ ID NO:1, and Z is the KD1 carboxy-region polypeptide of general formula (III) :

I-X3-K          (III)

in which X3 is an amino acid selected from natural and non-natural amino acids and is not C, F, R or W, and wherein optionally the polypeptide has the sequence IEK;

(b) the plasmin-inhibiting polypeptide in which N and C are each nothing, Y is methionine or N-formylmethionine, J is the KD1 polypeptide having the amino acid sequence set forth in SEQ ID NO:1, and Z is the KD1 carboxy-region polypeptide of general formula (III):

I-X3-K          (III)

in which X3 is an amino acid selected from natural and non-natural amino acids and is not C, F, R or W, and wherein optionally the polypeptide has the sequence IEK;

(c) the plasmin-inhibiting polypeptide in which N, Y and C are each nothing, J is the KD1 polypeptide having the amino acid sequence set forth in SEQ ID NO:1, and Z is the KD1 carboxy-region polypeptide of general formula (IV):

I-X3-K-V-X5          (IV) [SEQ ID NO:20]

in which X3 and X5 are independent, X3 is an amino acid selected from natural and non-natural amino acids and is not C, F, R or W, and X5 is an amino acid selected from natural and non-natural amino acids and is not C, F, R or W, and wherein optionally the polypeptide has the sequence IEKVP [SEQ ID NO:20];

(d) the plasmin-inhibiting polypeptide in which N, Y and C are each nothing, J is the KD1 polypeptide having the amino acid sequence set forth in SEQ ID NO:1, and Z is the KD1 carboxy-region polypeptide of general formula (V):

I-X3-K-V          (V) [SEQ ID NO:21]

in which X3 is an amino acid selected from natural and non-natural amino acids and is not C, F, R or W, and wherein optionally the polypeptide has the sequence IEKV [SEQ ID NO:21 in which X3 is E];

(e) the plasmin-inhibiting polypeptide in which N and C are each nothing, Y is methionine or N-formylmethionine, J is the KD1 polypeptide having the amino acid sequence set forth in SEQ ID NO:1, and Z is the KD1 carboxy-region polypeptide of general formula (II):

I-X3-K-V-X4-K          (II)

in which X3 and X4 are amino acids independently selected from natural and non-natural amino acids and are not C, F, R or W, and wherein optionally the polypeptide has the sequence IEKVPK [SEQ ID NO:2 in which X3 is E and X4 is P];

(f) the plasmin-inhibiting polypeptide in which N, Y and C are each nothing, J is the KD1 polypeptide having the amino acid sequence set forth in SEQ ID NO:1, and Z is the KD1 carboxy-region polypeptide of general formula (II):

I-X3-K-V-X4-K          (II)

in which X3 and X4 are amino acids independently selected from natural and non-natural amino acids and are not C, F, R or W, and wherein optionally the polypeptide has the sequence IEKVPK [SEQ ID NO:2 in which X3 is E and X4 is P];

(g) the plasmin-inhibiting polypeptide in which N and C are each nothing, Y is methionine or N-formylmethionine, J is the KD1 polypeptide having the amino acid sequence set forth in SEQ ID NO:1, and Z is the KD1 carboxy-region polypeptide of general formula (IV):

I-X3-K-V-X5          (IV) [SEQ ID NO:20]

in which X3 and X5 are independent, X3 is an amino acid selected from natural and non-natural amino acids and is not C, F, R or W, and X5 is an amino acid selected from natural and non-natural amino acids and is not C, F, R or W, and wherein optionally the polypeptide has the sequence IEKVP [SEQ ID NO:20 in which X3 is E and X5 is P]; and

(h) the plasmin-inhibiting polypeptide in which N and C are each nothing, Y is methionine or N-formylmethionine, J is the KD1 polypeptide having the amino acid sequence set forth in SEQ ID NO:1, and Z is the KD1 carboxy-region polypeptide of general formula (V):

I-X3-K-V          (V) [SEQ ID NO:21]

in which X3 is an amino acid selected from natural and non-natural amino acids and is not C, F, R or W and wherein optionally the polypeptide has the sequence IEKV [SEQ ID NO:21 in which X3 is E].

3.  A plasmin-inhibiting polypeptide of no more than 200, 190, 180, 170, 160, 150, 140, 130, 120, 110, 100, 90, 89, 88, 87, 86, 85, 84, 83, 82, 81, 80, 79, 78, 77, 76, 75, 74, 73, 72, 71, 70, 69, 68, 67, 66, 65, 64 or 63 amino acids, comprising the amino acid sequence set forth in any one of SEQ ID NOS:3-18, 22-29 and 32-35 at the carboxy terminus of said polypeptide, wherein the plasmin-inhibiting polypeptide inhibits plasmin activity, wherein the plasmin-inhibiting polypeptide has decreased anti-coagulation activity compared to a wild type human tissue factor pathway inhibitor-2 (TFPI-2) polypeptide first Kunitz-type proteinase inhibitor domain (KD1) having the amino acid sequence set forth in SEQ ID NO:19.

4.  A plasmin-inhibiting polypeptide of 60 or 61 amino acids that has an amino acid sequence that is at least 96, 97, 98 or 99% identical to the amino acid sequence of a plasmin-inhibiting polypeptide of general formula (I):

N-Y-J-Z-C                    (I)

in which:

(a) N is an amino terminus of the plasmin-inhibiting polypeptide,

(b) Y is either nothing or methionine or N-formylmethionine,

(c) J is a Kunitz-type proteinase first inhibitor domain (KD1) polypeptide of 57 amino acids having the amino acid sequence set forth in SEQ ID NO:1, in which amino acid X1 at position 11 in SEQ ID NO:1 is D, N or S, amino acid X2 at position 17 in SEQ ID NO:1 is R, and a cysteine residue is present at each of amino acid positions 14 and 38 in SEQ ID NO:1,

(d) Z is a KD1 carboxy-region polypeptide of general formula (III):

I-X3-K                    (III)

in which X3 is an amino acid selected from natural and non-natural amino acids and is not C, F, R or W,

(e) C is a carboxy terminus of the plasmin-inhibiting polypeptide and consists of 0 amino acids, and

(f) the plasmin-inhibiting polypeptide inhibits plasmin activity, and

wherein the plasmin-inhibiting polypeptide has decreased anti-coagulation activity compared to a wild type human tissue factor pathway inhibitor-2 (TFPI-2) polypeptide first Kunitz-type proteinase inhibitor domain (KD1) having the amino acid sequence set forth in SEQ ID NO:19.

5. A fusion protein comprising the plasmin-inhibiting polypeptide of any one of claims 1-4 fused at the amino terminus to a fusion polypeptide domain.

6. A composition comprising the plasmin-inhibiting polypeptide of any one of claims 1-4 to which a polyakylene glycol is attached to form a PAGylated polypeptide, wherein optionally polyethylene glycol is the polyakylene glycol and the PAGylated polypeptide is a PEGylated polypeptide.

7. A polynucleotide selected from an isolated polynucleotide comprising a nucleic acid sequence that encodes the plasmin-inhibiting polypeptide of any one of claims 1-4, and an expression vector comprising the polynucleotide of claim 13.

8. A host cell comprising the expression vector of claim 7.

9. A method of producing the plasmin-inhibiting polypeptide of any one of claims 1-4, said method comprising the steps of:

a) culturing the host cell of claim 8 under conditions and for a time sufficient to permit expression of the plasmin-inhibiting polypeptide; and

b) isolating the plasmin-inhibiting polypeptide from the cultured host cell; wherein optionally the plasmin-inhibiting polypeptide that is expressed by the host cell comprises N-terminal methionine or N-formylmethionine and wherein the host cell expresses a methionine aminopeptidase (MAP) under conditions and for a time sufficient for the MAP to remove the N-terminal methionine or N-formylmethionine from the plasmin-inhibiting polypeptide.

10. A pharmaceutical composition, comprising:

a) the plasmin-inhibiting polypeptide of any one of claims 1-4 or the fusion protein of claim 5; and

b) a physiologically acceptable carrier.

11. An *in vitro* method for inhibiting plasmin activity, comprising:
contacting, *in vitro,* (i) plasmin with (ii) the plasmin-inhibiting polypeptide of any one of claims 1-4, under conditions and for a time sufficient for the plasmin-inhibiting polypeptide to bind to the plasmin, and thereby inhibiting plasmin activity.

12. The plasmin-inhibiting polypeptide of any one of claims 1-4 or the isolated polynucleotide of claim 7 for use in medicine.

13. The plasmin-inhibiting polypeptide for use according to claim 12, wherein the subject is in need of inhibition of a plasmin activity, or has or is suspected of being at risk for having cancer, hemophilia, rheumatoid arthritis or systemic inflammatory response syndrome (SIRS), or wherein the subject is in need of or has undergone angiogenesis, bone remodeling or coronary artery bypass grafting (CABG), wherein optionally the subject is undergoing surgery or has recently undergone surgery, wherein optionally the surgery is cardiovascular surgery, oncological surgery, genitourinary surgery, orthopedic surgery, thoracic surgery, plastic surgery, trauma surgery, abdominal surgery, transplant surgery, neurologic surgery or otolaryngological surgery.

14. A method for isolating a plasmin-inhibiting polypeptide that comprises a Kunitz-type proteinase first inhibitor domain (KD1), comprising:

(a) contacting (i) a solid phase on which is immobilized plasmin that has been inactivated, with (ii) a sample that comprises a plasmin-inhibiting polypeptide which comprises a Kunitz-type proteinase first inhibitor domain (KD1) and impurities, under conditions and for a time sufficient for binding of the KD1 to the inactivated plasmin;
(b) washing the solid phase under conditions that do not disrupt KD1-plasmin binding, to remove impurities; and
(c) eluting the KD1 from the solid phase, and thereby isolating the polypeptide that comprises the KD1; wherein the plasmin-inhibiting polypeptide is a plasmin-inhibiting polypeptide according to any one of claims 1-4.

15. A method for protecting an isolated protein or a protein in an isolated biological sample from proteolytic degradation, comprising:
contacting the isolated protein or the isolated biological sample with a plasmin-inhibiting polypeptide according to any one of claims 1-4.

**Patentansprüche**

1. Plasmid-hemmendes Polypeptid, das ein Polypeptid der folgenden allgemeinen Formel (I) umfasst:

N-Y-J-Z-C (I)

worin:

(a) N ein Aminoterminus des Plasmid-hemmenden Polypeptids ist und aus 0, 1, 2, 3, 4, 5, 6, 7, 8, 9, 10, 11, 12, 13, 14, 15, 16, 17, 18, 19, 20, 21, 22, 23, 24, 25-30, 31-40, 41-50 oder 51-60 Aminosäuren besteht, die unabhängig aus natürlichen und nicht-natürlichen Aminosäuren ausgewählt sind,
(b) Y entweder Nichts oder Methionin oder N-Formylmethionin ist,
(c) J ein Kunitztyp-Proteinase-erste-Inhibitor-Domänen- (KD1-) Polypeptid aus 57 Aminosäuren mit der in SEQ ID NO: 1 dargelegten Aminosäuresequenz ist,
(d) Z ein KD1-Carboxy-Region-Polypeptid ist, das aus Folgendem ausgewählt ist:

(i) einem KD1-Carboxy-Region-Polypeptid der allgemeinen Formel (III):

I-X3-K (III)

worin X3 eine Aminosäure ist, die aus natürlichen und nicht-natürlichen Aminosäuren ausgewählt ist und nicht C, F, R oder W ist,
(ii) einem KD1-Carboxy-Region-Polypeptid der allgemeinen Formel (II):

I-X2-K-V-X4-K (II) [SEQ ID NO: 2]

worin X3 und X4 Aminosäuren sind, die unabhängig aus natürlichen und nicht-natürlichen Aminosäuren ausgewählt sind und nicht C, F, R, oder W sind,
(iii) einem KD1-Carboxy-Region-Polypeptid der allgemeinen Formel (IV):

I-X3-K-V-X5 (IV) [SEQ ID NO: 20]

worin X3 und X5 unabhängig sind, X3 eine Aminosäure ist, die aus natürlichen und nicht-natürlichen Aminosäuren ausgewählt ist und nicht C, F, R oder W ist, und X5 eine Aminosäure ist, die aus natürlichen und nicht-natürlichen Aminosäuren ausgewählt ist und nicht C, F, R oder W ist,
(iv) einem KD1-Carboxy-Region-Polypeptid der allgemeinen Formel (V):

I-X3-K-V                        (V) [SEQ ID NO: 21]

worin X3 eine Aminosäure ist, die aus natürlichen und nicht-natürlichen Aminosäuren ausgewählt ist und nicht C, F, R oder W ist,
(v) einem KD1-Carboxy-Region-Polypeptid der allgemeinen Formel (VI):

I-X3-K-X6                        (VI) [SEQ ID NO: 30]

worin X3 und X6 Aminosäuren sind, die unabhängig aus natürlichen und nicht-natürlichen Aminosäuren ausgewählt sind, X3 nicht C, F, R oder W ist und X6 irgendeine natürliche oder nicht-natürliche Aminosäure ist, und
(vi) einem KD1-Carboxy-Region-Polypeptid der allgemeinen Formel (VII):

I-X3-K-X6-X7                        (VII) [SEQ ID NO: 31]

worin X3, X6 und X7 Aminosäuren sind, die unabhängig aus natürlichen und nicht-natürlichen Aminosäuren ausgewählt sind, X3 nicht C, F, R oder W ist, X6 irgendeine natürliche oder nicht-natürliche Aminosäure ist und X7 irgendeine natürliche oder nicht-natürliche Aminosäure ist,

(e) C ein Carboxyterminus des Plasmin-hemmenden Polypeptids ist und aus 0 Aminosäuren besteht und
(f) das Plasmin-hemmende Polypeptid die Plasminaktivität hemmt und worin das Plasmin-hemmende Polypeptid eine verringerte gerinnungshemmende Aktivität im Vergleich zu einer Wildtyp-Human-Tissue-Factor-Pathway-Inhibitor-2-(TFPI-2-) Polypeptid-erste-Kunitztyp-Proteinase-Inhibitor-Domäne (KD1) mit der in SEQ ID NO: 19 dargelegten Aminosäuresequenz aufweist.

2.  Plasmin-hemmendes Polypeptid nach Anspruch 1, das aus Folgenden ausgewählt ist:

(a) Plasmin-hemmendem Polypeptid, in welchem N, Y und C jeweils Nichts sind, J das KD1-Polypeptid mit der in SEQ ID NO: 1 dargelegten Aminosäuresequenz ist und Z das KD1-Carboxy-Region-Polypeptid der allgemeinen Formel (III) ist:

I-X3-K                (III)

worin X3 eine Aminosäure ist, die aus natürlichen und nicht-natürlichen Aminosäuren ausgewählt ist und nicht C, F, R oder W ist, und worin gegebenenfalls das Polypeptid die Sequenz IEK aufweist,
(b) Plasmin-hemmendem Polypeptid, in welchem N und C jeweils Nichts sind, Y Methionin oder N-Formylmethionin ist, J das KD1-Polypeptid mit der in SEQ ID NO: 1 dargelegten Aminosäuresequenz ist und Z das KD1-Carboxy-Region-Polypeptid der allgemeinen Formel (III) ist:

I-X3-K                (III)

worin X3 eine Aminosäure ist, die aus natürlichen und nicht-natürlichen Aminosäuren ausgewählt ist und nicht C, F, R oder W ist, und worin gegebenenfalls das Polypeptid die Sequenz IEK aufweist;
(c) Plasmin-hemmendem Polypeptid, in welchen N, Y und C jeweils Nichts sind, J das KD1-Polypeptid mit der in SEQ ID NO: 1 dargelegten Aminosäuresequenz ist und Z das KD1-Carboxy-Region-Polypeptid der allgemeinen Formel (IV) ist:

I-X3-K-V-X5                (IV) [SEQ ID NO: 20]

worin X3 und X5 unabhängig sind, X3 eine Aminosäure ist, die aus natürlichen und nicht-natürlichen Aminosäuren ausgewählt ist und nicht C, F, R oder W ist, und X5 eine Aminosäure ist, die aus natürlichen und nicht-natürlichen Aminosäuren ausgewählt ist und nicht C, F, R oder W ist, und worin gegebenenfalls das Polypeptid die Sequenz IEKVP [SEQ ID NO: 20] aufweist;
(d) Plasmin-hemmendem Polypeptid, in welchem N, Y und C jeweils Nichts sind, J das KD1-Polypeptid mit der in SEQ ID NO: 1 dargelegten Aminosäuresequenz ist und Z das KD1-Carboxy-Region-Polypeptid der allge-

meinen Formel (V) ist:

$$\text{I-X3-K-V} \qquad \text{(V) [SEQ ID NO: 21]}$$

worin X3 eine Aminosäure ist, die aus natürlichen und nicht-natürlichen Aminosäuren ausgewählt ist und nicht C, F, R oder W ist, und worin gegebenenfalls das Polypeptid die Sequenz IEKV aufweist [SEQ ID NO: 21, worin X3 E ist];

(e) Plasmin-hemmendem Polypeptid, in welchem N und C jeweils Nichts sind, X Methionin oder N-Formylmethionin ist, J das KD1-Polypeptid mit der in SEQ ID NO: 1 dargelegten Aminosäuresequenz ist und Z das KD1-Carboxy-Region-Polypeptid der allgemeinen Formel (II) ist:

$$\text{I-X3-K-V-X4-K} \qquad \text{(II)}$$

worin X3 und X4 Aminosäuren sind, die unabhängig von natürlichen und nicht-natürlichen Aminosäuren ausgewählt sind und nicht C, F, R oder W sind, und worin gegebenenfalls das Polypeptid die Sequenz IEKVPK aufweist [SEQ ID NO: 2, worin X3 E ist und X4 P ist];

(f) Plasmin-hemmendem Polypeptid, in welchem N, Y und C jeweils Nichts sind, J das KD1-Polypeptid mit der in SEQ ID NO: 1 dargelegten Aminosäuresequenz ist und Z das KD1-Carboxy-Region-Polypeptid der allgemeinen Formel (II) ist:

$$\text{I-X3-K-V-X4-K} \qquad \text{(II)}$$

worin X3 und X4 Aminosäuren sind, die unabhängig aus natürlichen und nicht-natürlichen Aminosäuren ausgewählt sind und nicht C, F, R oder W sind, und worin gegebenenfalls das Polypeptid die Sequenz IEKVPK aufweist [SEQ ID NO: 2, worin X3 E ist und X4 P ist];

(g) Plasmin-hemmendem Polypeptid, worin N und C jeweils Nichts sind, Y Methionin oder N-Formylmethionin ist, J das KD1-Polypeptid mit der in SEQ ID NO: 1 dargestellten Aminosäuresequenz ist und Z das KD1-Carboxy-Region-Polypeptid der allgemeinen Formel (IV) ist:

$$\text{I-X3-K-V-X5} \qquad \text{(IV) [SEQ ID NO: 20]}$$

worin X3 und X5 unabhängig sind, X3 eine Aminosäure ist, die aus natürlichen und nicht-natürlichen Aminosäuren ausgewählt ist und nicht C, F, R oder W ist, und X5 eine Aminosäure ist, die aus natürlichen und nicht-natürlichen Aminosäuren ausgewählt ist und nicht C, F, R oder W ist, und worin gegebenenfalls das Polypeptid die Sequenz IEKVP aufweist [SEQ ID NO: 20, worin X3 E ist und X4 P ist]; und

(h) Plasmin-hemmendem Polypeptid, worin N und C jeweils Nichts sind, Y Methionin oder N-Formylmethionin ist, J das KD1-Polypeptid mit der in SEQ ID NO: 1 dargestellten Aminosäuresequenz ist und Z das KD1-Carboxy-Region-Polypeptid der allgemeinen Formel (V) ist:

$$\text{I-X3-K-V} \qquad \text{(V) [SEQ ID NO: 21]}$$

worin X3 eine Aminosäure ist, die aus natürlichen und nicht-natürlichen Aminosäuren ausgewählt ist und nicht C, F, R oder W ist, und worin gegebenenfalls das Polypeptid die Sequenz IEKV aufweist [SEQ ID NO: 21, worin X3 E ist].

3. Plasmin-hemmendes Polypeptid von nicht mehr als 200, 190, 180, 170, 160, 150, 140, 130, 120, 110, 100, 90, 89, 88, 87, 86, 85, 84, 83, 82, 81, 80, 79, 78, 77, 76, 75, 74, 73, 72, 71, 70, 69, 68, 67, 66, 65, 64 oder 63 Aminosäuren, umfassend die in einer der SEQ ID NOs: 3-18, 22-29 oder 32-35 dargestellten Aminosäuresequenz beim Carboxyterminus des Polypeptids, worin das Plasmin-hemmende Polypeptid die Plasminaktivität hemmt, worin das Plasmin-hemmende Polypeptid eine verringerte gerinnungshemmende Aktivität im Vergleich zu einer Wildtyp-Human-Tissue-Factor-Pathway-Inhibitor-2- (TFPI-2-) Polypeptid-erste-Kunitztyp-Proteinase-Inhibitor-Domäne (KD1) mit der in SEQ ID NO: 19 dargestellten Aminosäuresequenz aufweist.

4. Plasmin-inhibierendes Polypeptid aus 60 oder 61 Aminosäuren, das eine Aminosäuresequenz aufweist, die zu mindestens zu 96, 97, 98 oder 99 % identisch mit der Aminosäuresequenz eines Plasmin-hemmenden Polypeptids der allgemeinen Formel (I) ist:

$$\text{N-Y-J-Z-C} \qquad \text{(I),}$$

in welcher:

(a) N ein Aminoterminus des Plasmin-hemmenden Polypeptids ist,

(b) Y entweder Nichts oder Methionin oder N-Formylmethionin ist,

(c) J ein Kunitztyp-Proteinase-erster-Inhibitor-Domänen- (KD1-) Polypeptid aus 57 Aminosäuren mit der in SEQ ID NO: 1 dargestellten Aminosäuresequenz ist, worin die Aminosäure X1 an Position 11 in SEQ ID NO: 1 D, N oder S ist, die Aminosäure X2 an Position 17 in SEQ ID NO: 1 R ist und ein Cysteinrest an jeder der Aminosäurepositionen 14 und 38 in SEQ ID NO: 1 vorhanden ist,

(d) Z ein KD1-Carboxy-Region-Polypeptid der allgemeinen Formel (III) ist:

$$I\text{-}X3\text{-}K \qquad (III)$$

worin X3 eine Aminosäure ist, die aus natürlichen und nicht-natürlichen Aminosäuren ausgewählt ist und nicht C, F, R oder W ist,

(e) C ein Carboxyterminus des Plasmin-hemmenden Polypeptids ist und 0 Aminosäuren umfasst und

(f) das Plasmin-hemmende Polypeptid die Plasminaktivität hemmt und worin das Plasmin-hemmende Polypeptid eine verringerte gerinnungshemmende Aktivität im Vergleich zu einer Wildtyp-Human-Tissue-Factor-Pathway-Inhibitor-2-(TFPI-2-) Polypeptid-erste-Kunitztyp-Proteinase-Inhibitor-Domäne (KD1) mit der in SEQ ID NO: 19 dargelegten Aminosäuresequenz aufweist.

5. Fusionsprotein, umfassend das Plasmin-hemmende Polypeptid nach einem der Ansprüche 1-4, das am Aminoterminus an eine Fusionspolypeptiddomäne fusioniert ist.

6. Zusammensetzung, umfassend das Plasmin-hemmende Polypeptid nach einem der Ansprüche 1-4, an das Polyalkylenglykol gebunden ist, um ein PAGyliertes Polypeptid zu bilden, worin gegebenenfalls Polyethylenglykol das Polyalkylenglykol ist und das PAGylierte Polypeptid ein PEGyliertes Polypeptid ist.

7. Polynucleotid, das aus einem isolierten Polynucleotid, das eine Nucleinsäuresequenz umfasst, die für das Plasmin-hemmende Polypeptid nach einem der Ansprüche 1-4 kodiert, und einem Expressionsvektor, der das Polynucleotid nach Anspruch 13 umfasst, ausgewählt ist.

8. Wirtszelle, umfassend den Expressionsvektor nach Anspruch 7.

9. Verfahren zur Produktion des Plasmin-hemmenden Polypeptids nach einem der Ansprüche 1-4, wobei das Verfahren die folgenden Schritte umfasst:

(a) Kultivieren der Wirtszelle nach Anspruch 8 unter Bedingungen und für einen Zeitraum, die die Expression des Plasmin-hemmenden Polypeptids erlauben; und

(b) Isolation des Plasmin-hemmenden Polypeptids von der kultivierten Wirtszelle; wobei gegebenenfalls das Plasmin-hemmende Polypeptid, das durch die Wirtszelle exprimiert wird, N-terminales Methionin oder N-Formylmethionin umfasst und worin die Wirtszelle eine Methionin-Aminopeptidase (MAP) unter Bedingungen und für einen Zeitraum exprimiert, die der MAP die Entfernung des N-terminalen Methionins oder N-Formylmethionins vom Plasmin-hemmenden Polypeptid erlauben.

10. Pharmazeutische Zusammensetzung, umfassend:

(a) das Plasmin-hemmende Polypeptid nach einem der Ansprüche 1-4 oder das Fusionsprotein nach Anspruch 5; und

(b) einen physiologisch annehmbaren Träger.

11. In-vitro-Verfahren zur Hemmung von Plasminaktivität, umfassend:
Kontaktieren in vitro von (i) Plasmin mit (ii) dem Plasmin-hemmenden Polypeptid nach einem der Ansprüche 1-4 unter Bedingungen und für einen Zeitraum, die dem Plasmin-hemmenden Polypeptid erlauben, an das Plasmin zu binden, und dadurch Hemmen der Plasminaktivität.

12. Plasmin-hemmendes Polypeptid nach einem der Ansprüche 1-4 oder isoliertes Polynucleotid nach Anspruch 7 zur Verwendung in der Medizin.

**13.** Plasmin-hemmendes Polypeptid zur Verwendung nach Anspruch 12, worin das Individuum eine Hemmung der Plasminaktivität benötigt oder bei dem Individuum ein Risiko für Krebs, Hämophilie, rheumatoide Arthritis oder systemisches Entzündungsreaktionssyndrom (SIRS) bzw. der Verdacht darauf besteht oder worin das Individuum Angiogenese, Knochenumbau oder einen Koronararterienbypass (CABG) benötigt oder dieser unterzogen wurde, worin das Individuum gegebenenfalls einem chirurgischen Eingriff unterzogen wird oder kürzlich wurde, worin gegebenenfalls der chirurgische Eingriff ein kardiovaskulärer Eingriff, ein onkologischer Eingriff, ein urogenitaler Eingriff, ein orthopädischer Eingriff, ein thorakaler Eingriff, ein plastisch-chirurgischer Eingriff, ein unfallchirurgischer Eingriff, ein abdominaler Eingriff, ein Transplantationseingriff, ein neurologischer Eingriff oder ein otolaryngologischer Eingriff ist.

**14.** Verfahren zur Isolierung eines Plasmin-hemmenden Polypeptids, das eine Kunitztyp-Proteinase-erste-Inhibitor-Domäne (KD1) umfasst, umfassend:

(a) das Kontaktieren (i) einer festen Phase, auf welcher sich immobilisiertes Plasmin befindet, das inaktiviert worden ist, mit (ii) einer Probe, die ein Plasmin-hemmendes Polypeptid, das eine Kunitztyp-Proteinase-erste-Inhibitor-Domäne (KD1) umfasst, und Verunreinigungen umfasst, unter Bedingungen und für einen Zeitraum, die die Bindung von KD1 an das inaktivierte Plasmin erlauben;
(b) das Waschen der festen Phase unter Bedingungen, die die KD1-Plasmin-Bindung nicht unterbrechen, um Verunreinigungen zu entfernen; und
(c) die Elution des KD1 aus der festen Phase und dadurch Isolierung des Polypeptids, das das KD1 umfasst; worin das Plasmin-hemmende Polypeptid ein Plasmin-hemmendes Polypeptid nach einem der Ansprüche 1-4 ist.

**15.** Verfahren zum Schutz eines isolierten Proteins oder eines Proteins in einer isolierten biologischen Probe aus proteolytischem Abbau, umfassend:
das Kontaktieren des isolierten Proteins oder der isolierten biologischen Probe mit einem Plasmin-hemmenden Polypeptid nach einem der Ansprüche 1-4.

**Revendications**

**1.** Polypeptide inhibiteur de la plasmine, comprenant un polypeptide de formule générale (I) :

$$N\text{-}Y\text{-}J\text{-}Z\text{-}C \qquad (I)$$

dans laquelle :

(a) N est une extrémité N-terminale du polypeptide inhibiteur de la plasmine et consiste en 0, 1, 2, 3, 4, 5, 6, 7, 8, 9, 10, 11, 12, 13, 14, 15, 16, 17, 18, 19, 20, 21, 22, 23, 24, 25-30, 31-40, 41-50, ou 51-60 acides aminés qui sont indépendamment sélectionnés parmi des acides aminés naturels et non naturels,
(b) Y est rien ou la méthionine ou la N-formylméthionine,
(c) J est un polypeptide du premier domaine inhibiteur de protéinase de type Kunitz (KD1) de 57 acides aminés possédant la séquence d'acides aminés décrite dans SEQ ID NO: 1,
(d) Z est un polypeptide de la région carboxyle de KD1 qui est sélectionné parmi :

(i) un polypeptide de la région carboxyle de KD1 de formule générale (III) :

$$I\text{-}X3\text{-}K \qquad (III)$$

dans laquelle X3 est un acide aminé sélectionné parmi des acides aminés naturels et non naturels et n'est pas C, F, R ou W,
(ii) un polypeptide de la région carboxyle de KD1 de formule générale (II) :

$$I\text{-}X3\text{-}K\text{-}V\text{-}X4\text{-}K \qquad (II) \text{ [SEQ ID NO: 2]}$$

dans laquelle X3 et X4 sont des acides aminés indépendamment sélectionnés parmi des acides aminés naturels et non naturels et ne sont pas C, F, R ou W,
(iii) un polypeptide de la région carboxyle de KD1 de formule générale (IV) :

I-X3-K-V-X5        (IV) [SEQ ID NO: 20]

dans laquelle X3 et X5 sont indépendants, X3 est un acide aminé sélectionné parmi des acides aminés naturels et non naturels et n'est pas C, F, R ou W, et X5 est un acide aminé sélectionné parmi des acides aminés naturels et non naturels et n'est pas C, F, R ou W,
(iv) un polypeptide de la région carboxyle de KD1 de formule générale (V) :

I-X3-K-V        (V) [SEQ ID NO: 21]

dans laquelle X3 est un acide aminé sélectionné parmi des acides aminés naturels et non naturels et n'est pas C, F, R ou W,
(v) un polypeptide de la région carboxyle de KD1 de formule générale (VI) :

I-X3-K-X6        (VI) [SEQ ID NO: 30]

dans laquelle X3 et X6 sont des acides aminés indépendamment sélectionnés parmi des acides aminés naturels et non naturels, X3 n'est pas C, F, R ou W, et X6 est un quelconque acide aminé naturel ou non naturel, et
(vi) un polypeptide de la région carboxyle de KD1 de formule générale (VII) :

I-X3-K-X6-X7        (VII) [SEQ ID NO: 31]

dans laquelle X3, X6 et X7 sont des acides aminés indépendamment sélectionnés parmi des acides aminés naturels et non naturels, X3 n'est pas C, F, R ou W, X6 est un quelconque acide aminé naturel ou non naturel, et X7 est un quelconque acide aminé naturel ou non naturel,

(e) C est une extrémité C-terminale du polypeptide inhibiteur de la plasmine et consiste en 0 acides aminés, et
(f) le polypeptide inhibiteur de la plasmine inhibe l'activité de la plasmine, et où le polypeptide inhibiteur de la plasmine présente une activité anticoagulation réduite par comparaison à un premier domaine inhibiteur de protéinase de type Kunitz (KD1) d'un polypeptide inhibiteur de la voie du facteur tissulaire-2 humain de type sauvage (TFPI-2) possédant la séquence d'acides aminés décrite dans SEQ ID NO: 19.

2. Polypeptide inhibiteur de la plasmine selon la revendication 1 qui est sélectionné parmi :

(a) le polypeptide inhibiteur de la plasmine dans lequel N, Y et C sont chacun rien, J est le polypeptide KD1 possédant la séquence d'acides aminés décrite dans SEQ ID NO: 1, et Z est le polypeptide de la région carboxyle de KD1 de formule générale (III) :

I-X3-K        (III)

dans laquelle X3 est un acide aminé sélectionné parmi des acides aminés naturels et non naturels et n'est pas C, F, R ou W, et dans laquelle facultativement le polypeptide possède la séquence IEK ;
(b) le polypeptide inhibiteur de la plasmine dans lequel N et C sont chacun rien, Y est la méthionine ou la N-formylméthionine, J est le polypeptide KD1 possédant la séquence d'acides aminés décrite dans SEQ ID NO: 1, et Z est le polypeptide de la région carboxyle de KD1 de formule générale (III) :

I-X3-K        (III)

dans laquelle X3 est un acide aminé sélectionné parmi des acides aminés naturels et non naturels et n'est pas C, F, R ou W, et dans laquelle facultativement le polypeptide possède la séquence IEK ;
(c) le polypeptide inhibiteur de la plasmine dans lequel N, Y et C sont chacun rien, J est le polypeptide KD1 possédant la séquence d'acides aminés décrite dans SEQ ID NO: 1, et Z est le polypeptide de la région carboxyle de KD1 de formule générale (IV) :

I-X3-K-V-X5        (IV) [SEQ ID NO: 20]

dans laquelle X3 et X5 sont indépendants, X3 est un acide aminé sélectionné parmi des acides aminés naturels et non naturels et n'est pas C, F, R ou W, et X5 est un acide aminé sélectionné parmi des acides aminés naturels et non naturels et n'est pas C, F, R ou W, et dans laquelle facultativement le polypeptide possède la séquence IEKVP [SEQ ID NO: 20] ;

(d) le polypeptide inhibiteur de la plasmine dans lequel N, Y et C sont chacun rien, J est le polypeptide KD1 possédant la séquence d'acides aminés décrite dans SEQ ID NO: 1, et Z est le polypeptide de la région carboxyle de KD1 de formule générale (V) :

$$\text{I-X3-K-V} \qquad \text{(V) [SEQ ID NO: 21]}$$

dans laquelle X3 est un acide aminé sélectionné parmi des acides aminés naturels et non naturels et n'est pas C, F, R ou W, et dans laquelle facultativement le polypeptide possède la séquence IEKV [SEQ ID NO: 21 dans laquelle X3 est E] ;

(e) le polypeptide inhibiteur de la plasmine dans lequel N et C sont chacun rien, Y est la méthionine ou la N-formylméthionine, J est le polypeptide KD1 possédant la séquence d'acides aminés décrite dans SEQ ID NO: 1, et Z est le polypeptide de la région carboxyle de KD1 de formule générale (II) :

$$\text{I-X3-K-V-X4-K} \qquad \text{(II)}$$

dans laquelle X3 et X4 sont des acides aminés indépendamment sélectionnés parmi des acides aminés naturels et non naturels et ne sont pas C, F, R ou W, et dans laquelle facultativement le polypeptide possède la séquence IEKVPK [SEQ ID NO: 2 dans laquelle X3 est E et X4 est P] ;

(f) le polypeptide inhibiteur de la plasmine dans lequel N, Y et C sont chacun rien, J est le polypeptide KD1 possédant la séquence d'acides aminés décrite dans SEQ ID NO: 1, et Z est le polypeptide de la région carboxyle de KD1 de formule générale (II) :

$$\text{I-X3-K-V-X4-K} \qquad \text{(II)}$$

dans laquelle X3 et X4 sont des acides aminés indépendamment sélectionnés parmi des acides aminés naturels et non naturels et ne sont pas C, F, R ou W, et dans laquelle facultativement le polypeptide possède la séquence IEKVPK [SEQ ID NO: 2 dans laquelle X3 est E et X4 est P] ;

(g) le polypeptide inhibiteur de la plasmine dans lequel N et C sont chacun rien, Y est la méthionine ou la N-formylméthionine, J est le polypeptide KD1 possédant la séquence d'acides aminés décrite dans SEQ ID NO: 1, et Z est le polypeptide de la région carboxyle de KD1 de formule générale (IV) :

$$\text{I-X3-K-V-X5} \qquad \text{(IV) [SEQ ID NO: 20]}$$

dans laquelle X3 et X5 sont indépendants, X3 est un acide aminé sélectionné parmi des acides aminés naturels et non naturels et n'est pas C, F, R ou W, et X5 est un acide aminé sélectionné parmi des acides aminés naturels et non naturels et n'est pas C, F, R ou W, et dans laquelle facultativement le polypeptide possède la séquence IEKVP [SEQ ID NO: 20 dans laquelle X3 est E et X5 est P] ; et

(h) le polypeptide inhibiteur de la plasmine dans lequel N et C sont chacun rien, Y est la méthionine ou la N-formylméthionine, J est le polypeptide KD1 possédant la séquence d'acides aminés décrite dans SEQ ID NO: 1, et Z est le polypeptide de la région carboxyle de KD1 de formule générale (V) :

$$\text{I-X3-K-V} \qquad \text{(V) [SEQ ID NO: 21]}$$

dans laquelle X3 est un acide aminé sélectionné parmi des acides aminés naturels et non naturels et n'est pas C, F, R ou W, et dans laquelle facultativement le polypeptide possède la séquence IEKV [SEQ ID NO: 21 dans laquelle X3 est E] .

3. Polypeptide inhibiteur de la plasmine de 200, 190, 180, 170, 160, 150, 140, 130, 120, 110, 100, 90, 89, 88, 87, 86, 85, 84, 83, 82, 81, 80, 79, 78, 77, 76, 75, 74, 73, 72, 71, 70, 69, 68, 67, 66, 65, 64 ou 63 acides aminés au maximum, comprenant la séquence d'acides aminés décrite dans l'une quelconque de SEQ ID NO: 3-18, 22-29 et 32-35 à l'extrémité C-terminale dudit polypeptide, où le polypeptide inhibiteur de la plasmine inhibe l'activité de la plasmine, et où le polypeptide inhibiteur de la plasmine présente une activité anticoagulation réduite par comparaison à un premier domaine inhibiteur de protéinase de type Kunitz (KD1) d'un polypeptide inhibiteur de la voie du facteur tissulaire-2 humain de type sauvage (TFPI-2) possédant la séquence d'acides aminés décrite dans SEQ ID NO: 19.

**4.** Polypeptide inhibiteur de la plasmine de 60 ou 61 acides aminés qui possède une séquence d'acides aminés qui est au moins 96, 97, 98 ou 99 % identique à la séquence d'acides aminés d'un polypeptide inhibiteur de la plasmine de formule générale (I) :

$$N\text{-}Y\text{-}J\text{-}Z\text{-}C \qquad (I)$$

dans laquelle :

(a) N est une extrémité N-terminale du polypeptide inhibiteur de la plasmine,
(b) Y est rien ou la méthionine ou la N-formylméthionine ;
(c) J est un polypeptide du premier domaine inhibiteur de protéinase de type Kunitz (KD1) de 57 acides aminés possédant la séquence d'acides aminés décrite dans SEQ ID NO: 1, dans laquelle l'acide aminé X1 à la position 11 dans SEQ ID NO: 1 est D, N ou S, l'acide aminé X2 à la position 17 dans SEQ ID NO: 1 est R, et un résidu cystéine est présent à chacune des positions d'acides aminés 14 et 38 dans SEQ ID NO: 1,
(d) Z est un polypeptide de la région carboxyle de KD1 de formule générale (III) :

$$I\text{-}X3\text{-}K \qquad (III)$$

dans laquelle X3 est un acide aminé sélectionné parmi des acides aminés naturels et non naturels et n'est pas C, F, R ou W,
(e) C est une extrémité C-terminale du polypeptide inhibiteur de la plasmine et consiste en 0 acides aminés, et
(f) le polypeptide inhibiteur de la plasmine inhibe l'activité de la plasmine, et où le polypeptide inhibiteur de la plasmine présente une activité anticoagulation réduite par comparaison à un premier domaine inhibiteur de protéinase de type Kunitz (KD1) d'un polypeptide inhibiteur de la voie du facteur tissulaire-2 humain de type sauvage (TFPI-2) possédant la séquence d'acides aminés décrite dans SEQ ID NO: 19.

**5.** Protéine de fusion comprenant le polypeptide inhibiteur de la plasmine selon l'une quelconque des revendications 1-4 fusionné, au niveau de l'extrémité N-terminale, à un domaine de polypeptide de fusion.

**6.** Composition comprenant le polypeptide inhibiteur de la plasmine selon l'une quelconque des revendications 1-4 auquel un polyalkylène glycol est attaché pour former un polypeptide PAGylé, dans laquelle facultativement le polyéthylène glycol est le polyalkylène glycol et le polypeptide PAGylé est un polypeptide PEGylé.

**7.** Polynucléotide sélectionné parmi un polynucléotide isolé comprenant une séquence d'acide nucléique qui code pour le polypeptide inhibiteur de la plasmine selon l'une quelconque des revendications 1 à 4, et un vecteur d'expression comprenant le polynucléotide selon la revendication 13.

**8.** Cellule hôte comprenant le vecteur d'expression selon la revendication 7.

**9.** Procédé de production du polypeptide inhibiteur de la plasmine selon l'une quelconque des revendications 1 à 4, ledit procédé comprenant les étapes consistant à :

a) mettre en culture la cellule hôte selon la revendication 8 dans des conditions et pendant une durée suffisante pour permettre l'expression du polypeptide inhibiteur de la plasmine ; et
b) isoler le polypeptide inhibiteur de la plasmine à partir de la cellule hôte mise en culture ; où facultativement le polypeptide inhibiteur de la plasmine qui est exprimé par la cellule hôte comprend une méthionine ou une N-formylméthionine N-terminale et où la cellule hôte exprime une méthionine aminopeptidase (MAP) dans des conditions et pendant une durée suffisante pour que la MAP élimine la méthionine ou la N-formylméthionine N-terminale du polypeptide inhibiteur de la plasmine.

**10.** Composition pharmaceutique, comprenant :

a) le polypeptide inhibiteur de la plasmine selon l'une quelconque des revendications 1 à 4 ou la protéine de fusion selon la revendication 5 ; et
b) un véhicule physiologiquement acceptable.

**11.** Procédé *in vitro* pour inhiber l'activité de la plasmine, comprenant :
la mise en contact, *in vitro,* (i) de la plasmine avec (ii) le polypeptide inhibiteur de la plasmine selon l'une quelconque des revendications 1 à 4, dans des conditions et pendant une durée suffisante pour que le polypeptide inhibiteur de la plasmine se lie à la plasmine, et en inhibant ainsi l'activité de la plasmine.

12. Polypeptide inhibiteur de la plasmine selon l'une quelconque des revendications 1 à 4 ou polynucléotide isolé selon la revendication 7 destiné à être utilisé en médecine.

13. Polypeptide inhibiteur de la plasmine destiné à être utilisé selon la revendication 12, dans lequel le sujet nécessite une inhibition d'une activité de la plasmine, ou présente ou est suspecté d'être exposé à un risque de présenter un cancer, l'hémophilie, la polyarthrite rhumatoïde ou le syndrome de réponse inflammatoire systémique (SRIS), ou dans lequel le sujet nécessite ou a subi une angiogenèse, un remodelage osseux ou un pontage aorto-coronarien (PAC), dans lequel facultativement le sujet subit une chirurgie ou a récemment subi une chirurgie, dans lequel facultativement la chirurgie est une chirurgie cardiovasculaire, une chirurgie oncologique, une chirurgie génito-urinaire, une chirurgie orthopédique, une chirurgie thoracique, une chirurgie plastique, une chirurgie traumatologique, une chirurgie abdominale, une chirurgie de transplantation, une chirurgie neurologique ou une chirurgie d'otolaryngologie.

14. Procédé pour isoler un polypeptide inhibiteur de la plasmine qui comprend un premier domaine inhibiteur de protéinase de type Kunitz (KD1), comprenant :

(a) la mise en contact (i) d'une phase solide sur laquelle est immobilisée une plasmine qui a été inactivée, avec (ii) un échantillon qui comprend un polypeptide inhibiteur de la plasmine qui comprend un premier domaine inhibiteur de protéinase de type Kunitz (KD1) et des impuretés, dans des conditions et pendant une durée suffisante pour permettre la liaison du KD1 à la plasmine inactivée ;
(b) le lavage de la phase solide dans des conditions qui ne perturbent pas la liaison KD1-plasmine, pour éliminer les impuretés ; et
(c) l'élution du KD1 à partir de la phase solide, et ainsi l'isolement du polypeptide qui comprend le KD1 ; dans lequel le polypeptide inhibiteur de la plasmine est un polypeptide inhibiteur de la plasmine selon l'une quelconque des revendications 1-4.

15. Procédé pour protéger une protéine isolée, ou une protéine dans un échantillon biologique isolé, d'une dégradation protéolytique, comprenant :
la mise en contact de la protéine isolée ou de l'échantillon biologique isolé avec un polypeptide inhibiteur de la plasmine selon l'une quelconque des revendications 1 à 4.

FIG. 1

FIG. 2

*FIG. 3*

*FIG. 4*

SM = Single mutant L17R-60 aa
DM = Double Mutant-60 aa
BPTI = Aprotinin

FIG. 5

60A

60B

63-residue

*FIG. 6*

**FIG. 7**

A

(M+H)+

7663

-GN

7491

Relative Abundance

100

75

50

25

0

6500    7000    7500    8000    8500    9000

*m/z*

Plasmin

P1 P2*

B

|  |  |  |  | 1 | 12 |  | 22 |
|---|---|---|---|---|---|---|---|

SEQ ID NO: 45  BPTI (Kutitz Prototype) :   R PDFCLEPPYT GPCKARIIRY FYNAKAGLCQ

SEQ ID NO: 46  KD1$_{L17R}$-V$_T$ : GSHM DAAQEPTGNN AEICLLPLDY GPCRARLLRY YYDRYTQSCR

SEQ ID NO: 47  KD1$_{L17R}$-K$_T$ (~80%) :           GNN AEICLLPLDY GPCRARLLRY YYDRYTQSCR

SEQ ID NO: 48  KD1$_{L17R}$-K$_T$ (~20%) :             N AEICLLPLDY GPCRARLLRY YYDRYTQSCR

32        42        52      58    62

BPTI : TFVYGGCRAK RNNFKSAEDC MRTCGGA        (6518 Da)

KD1$_{L17R}$-V$_T$ : QFLYGGCEGN ANNFYTWEAC DDACWRIEKV PKV (8888 Da)

KD1$_{L17R}$-K$_T$ : QFLYGGCEGN ANNFYTWEAC DDACWRIEKV PK  (7662 Da)

KD1$_{L17R}$-K$_T$ : QFLYGGCEGN ANNFYTWEAC DDACWRIEKV PK  (7491 Da)

*FIG. 8*

*FIG. 9*

**FIG. 10**

Plasminogen-kringle1

*FIG. 11*

FIG. 12

FIG. 13

**(M+H)⁺**
8792.1

8380          8540          8700    **m/z**    8860          9020          9180

**BPTI numbering**
↓

SEQ ID NO: 46     GSHM DAAQEPTGNN₁ AEICLLPLDY GPCRARLLRY YYDRYTQSCR
QFLYGGCEGN ANNFYTWEAC DDACWRIEKV PK₆₃↓V₆₄

*FIG. 14*

FIG. 15

**Plasmin-protease**

**FIG. 16**

## REFERENCES CITED IN THE DESCRIPTION

**Patent documents cited in the description**

- US 20080026998 A
- US 20090018069 A
- US 4935233 A
- US 4751180 A
- US 7829659 B
- US 6623729 B
- US 6517824 B
- US 6515017 B
- US 6217869 B
- US 6191105 B
- US 5681811 A
- US 5455027 A
- US 20040018960 A
- US 20030229010 A
- US 20030229006 A
- US 20030186869 A
- US 20030026764 A
- US 20030017131 A
- US 6214966 B
- US 2003000447 A
- US 2001021763 A
- US 7786119 B
- US 5151510 A, Stec
- US 4237224 A
- US 5837458 A
- US 3845770 A
- US 4326525 A
- WO 9850016 A
- US 61922026 B
- US 61793131 B

**Non-patent literature cited in the description**

- **FURGUSSON et al.** *N Engl J Med.,* 2008, vol. 358, 2319
- **LASKOWSKI ; KATO.** *Annu. Rev. Biochem.,* 1980, vol. 49, 593
- **SPRECHER et al.** *Proc. Nat. Acad. Sci. USA,* 1994, vol. 91, 3353
- **CHAND et al.** *J. Biol. Chem.,* 2004, vol. 279, 17500
- **BAJAJ et al.** *J. Biol. Chem.,* 2011, vol. 286, 4329
- **CHAND et al.** *J. Biol. Chem.,* 2004, vol. 279, 17500-17505
- **NATARAJAN et al.** *PLoS ONE,* 2011, vol. 6 (5), e20176
- **SHEN et al.** *Proc. Natl. Acad. Sci. USA,* 1993, vol. 90, 8108
- **SHEN et al.** *Prot. Eng.,* 1997, vol. 10, 1085
- **TSUNASAWA et al.** *J. Biochem.,* 1997, vol. 122, 843
- **BRADSHAW et al.** *Trends Bioch. Sci.,* 1998, vol. 23, 263
- **BEN-BASSAT et al.** *J. Bacteriol.,* 1987, vol. 169, 751
- **REITER et al.** *Prot. Eng.,* 1994, vol. 7, 697
- **ZHU et al.** *Prot. Sci.,* 1997, vol. 6, 781
- **MABRY et al.** *Mabs,* 2010, vol. 2, 20
- **GAO et al.** *Proc. Nat. Acad. Sci. USA,* 1999, vol. 96, 6025
- **LIM et al.** *Biotechnol. Bioeng.,* 2010, vol. 106, 27
- **FAN et al.** *FASEB J.,* 2008, vol. 22, 3795
- **MARATEA et al.** *Gene,* 1985, vol. 40, 39-46
- **MURPHY et al.** *Proc. Natl. Acad. Sci. USA,* 1986, vol. 83, 8258-8262
- **CHAUDHARY et al.** *Proc. Natl. Acad. Sci. U.S.A.,* 1990, vol. 87, 1066-1070
- **BIRD et al.** *Science,* 1988, vol. 242, 423-426
- **PETERSEN et al.** *Biochem.,* 1996, vol. 35, 266
- **WHITE, J.S. ; WHITE, D.C.** Proteins, Peptides and Amino Acids Sourcebook. Humana Press, 2002
- **JONES, J.** Amino Acid and Peptide Synthesis. Oxford Univ. Press, 2002
- Unnatural Amino Acids. ChemFiles. Fluka Chemie GmbH; Sigma-Aldrich, 2001, vol. 1
- Unnatural Amino Acids II. ChemFiles. Fluka Chemie GmbH; Sigma-Aldrich, 2002, vol. 2
- **KOTHA.** *Acc. Chem. Res.,* 2003, vol. 36, 342
- **MARUOKA et al.** *Proc. Nat. Acad. Sci. USA,* 2004, vol. 101, 5824
- **LUNDQUIST et al.** *Org. Lett.,* 2001, vol. 3, 781
- **TANG et al.** *J. Org. Chem.,* 2002, vol. 67, 7819
- **ROTHMAN et al.** *J. Org. Chem.,* 2003, vol. 68, 6795
- **KREBS et al.** *Chemistry,* 2004, vol. 10, 544
- **GOODMAN et al.** *Biopolymers,* 2001, vol. 60, 229

- **SABAT et al.** *Org. Lett.,* 2000, vol. 2, 1089
- **FU et al.** *J. Org. Chem.,* 2001, vol. 66, 7118
- **HRUBY et al.** *Meths. Mol. Biol.,* 1994, vol. 35, 249
- **FAUCHERE.** *J. Adv. Drug Res.,* 1986, vol. 15, 29
- **EVANS et al.** *J. Med. Chem.,* 1987, vol. 30, 1229
- **HUBER et al.** *J Mol Biol.,* 1974, vol. 89, 73-101
- **SCHMIDT et al.** *J Biol Chem.,* 2005, vol. 280, 27832-27838
- **WANG et al.** *Science,* 1998, vol. 281, 1662-1665
- **BAJAJ et al.** *Thromb. Haemost.,* 2001, vol. 86, 959-972
- **DONATE et al.** *Prot. Sci.,* 1994, vol. 3, 2378
- **BRADLEY et al.** *Science,* 2005, vol. 309, 1868-1871
- **SCHUELER-FURMAN et al.** *Science,* 2005, vol. 310, 638
- **DIETZ et al.** *Proc. Nat. Acad. Sci. USA,* 2006, vol. 103, 1244
- **DODSON et al.** *Nature,* 2007, vol. 450, 176
- **QIAN et al.** *Nature,* 2007, vol. 450, 259
- **RAMAN et al.** *Science,* 2010, vol. 327, 1014-1018
- **BROOKS et al.** *J. Comput. Chem.,* 1983, vol. 4, 187-217
- **WEINER et al.** *J. Comput. Chem.,* 1981, vol. 106, 765
- **EISENFIELD et al.** *Am. J. Physiol.,* 1991, vol. 261, C376-386
- **LYBRAND.** *J. Pharm. Belg.,* 1991, vol. 46, 49-54
- **FROIMOWITZ.** *Biotechniques,* 1990, vol. 8, 640-644
- **BURBAM et al.** *Proteins,* 1990, vol. 7, 99-111
- **PEDERSEN.** *Environ. Health Perspect.,* 1985, vol. 61, 185-190
- **KINI et al.** *J. Biomol. Struct. Dyn.,* 1991, vol. 9, 475-488
- **GREENWALD et al.** *Adv. Drug Del. Rev.,* 2003, vol. 55, 217
- **MOLINEUX.** *Pharmacotherapy,* 2003, vol. 8, 3S-8S
- **ROBERTS et al.** *Adv. Drug Deliv. Rev.,* 2002, vol. 54, 459
- **BHADRA et al.** *Pharmazie,* 2002, vol. 57, 5
- **GREENWALD.** *Controlled Release,* 2001, vol. 74, 159
- **VERONESE et al.** *Farmaco.,* 1999, vol. 54, 497
- **ZALIPSKY.** *Adv. Drug Deliv. Rev.,* 1995, vol. 16, 157
- **GREENWALD et al.** *J. Med. Chem.,* 1996, vol. 39, 424
- **RICHARD C. LAROCK.** Comprehensive Organic Transformations: A Guide to Functional Group Preparations. Wiley-VCH, 1999
- **JERRY MARCH ; MICHAEL SMITH.** March's Advanced Organic Chemistry: Reactions, Mechanisms and Structure. John Wiley & Sons Inc, 2001
- **ALTSCHUL.** *J. Mol. Biol.,* 1991, vol. 219, 555-565
- **HENIKOFF ; HENIKOFF.** *PNAS USA,* 1992, vol. 89, 10915-10919
- **LAPLANCHE et al.** *Nucl. Acids Res.,* 1986, vol. 14, 9081
- **STEC et al.** *J. Am. Chem. Soc.,* 1984, vol. 106, 6077
- **STEIN et al.** *Nucl. Acids Res.,* 1988, vol. 16, 3209
- **ZON et al.** *Anti-Cancer Drug Design,* 1991, vol. 6, 539
- **ZON et al.** Oligonucleotides And Analogues: A Practical Approach. Oxford University Press, 1991, 87-108
- **UHLMANN ; PEYMAN.** *Chemical Reviews,* 1990, vol. 90, 543
- **CHAND et al.** *J. Biol. Chem.,* 2004, vol. 17, 17500
- A model of evolutionary change in proteins - Matrices for detecting distant relationships. **DAYHOFF, M.O.** Atlas of Protein Sequence and Structure. National Biomedical Research Foundation, 1978, vol. 5, 345-358
- **HEIN J.** *Unified Approach to Alignment and Phylogenes,* 1990, 626-645
- Methods in Enzymology. Academic Press, Inc, vol. 183
- **HIGGINS, D.G. ; SHARP, P.M.** *CABIOS,* 1989, vol. 5, 151-153
- **MYERS, E.W. ; MULLER W.** *CABIOS,* 1988, vol. 4, 11-17
- **ROBINSON, E.D.** *Comb. Theor,* 1971, vol. 11, 105
- **SANTOU, N. ; NES, M.** *Mol. Biol. Evol.,* 1987, vol. 4, 406-425
- **SNEATH, P.H.A. ; SOKAL, R.R.** Numerical Taxonomy - the Principles and Practice of Numerical Taxonomy. Freeman Press, 1973
- **WILBUR, W.J. ; LIPMAN, D.J.** *Proc. Natl. Acad., Sci. USA,* 1983, vol. 80, 726-730
- **SMITH ; WATERMAN.** *Add. APL. Math,* 1981, vol. 2, 482
- **NEEDLEMAN ; WUNSCH.** *J. Mol. Biol.,* 1970, vol. 48, 443
- **PEARSON ; LIPMAN.** *Proc. Natl. Acad. Sci. USA,* 1988, vol. 85, 2444
- **ALTSCHUL et al.** *Nucl. Acids Res.,* 1977, vol. 25, 3389-3402
- **ALTSCHUL et al.** *J. Mol. Biol.,* 1990, vol. 215, 403-410

- **HENIKOFF ; HENIKOFF.** *Proc. Natl. Acad. Sci. USA,* 1989, vol. 89, 10915
- **PLUCKTHUN, A.** *Bio/Technology,* 1991, vol. 9, 545-551
- *Curr. Opinion Biotech.,* 1993, vol. 4, 573-576
- **TRILL et al.** *Curr. Opinion Biotech,* 1995, vol. 6, 553-560
- **SAMBROOK et al.** Molecular Cloning: a Laboratory Manual. Cold Spring Harbor Laboratory Press, 1989
- Current Protocols in Molecular Biology. John Wiley & Sons, 1992
- **GRAHAM et al.** *Virology,* 1973, vol. 52, 456
- **SAMBROOK et al.** Molecular Cloning, A Laboratory Manual. Cold Spring Harbor Laboratories, 2001
- **DAVIS et al.** Basic Methods In Molecular Biology. Elsevier, 1986
- **CHU et al.** *Gene,* 1981, vol. 13, 197
- **HOGAN et al.** Manipulating the Mouse Embryo: A Laboratory Manual. Cold Spring Harbor Press, 1999
- **JACKSON et al.** Mouse Genetics and Transgenics: A Practical Approach. Oxford Univ. Press, 2000
- **PINKERT.** Transgenic Animal Technology: A Laboratory Handbook. Academic Press, 1999
- Molecular Diagnosis of Cancer: Methods and Protocols. Humana Press, 2004
- Cancer Imaging. Academic Press, 2007
- **SKARIN.** Atlas of Diagnostic Oncology. Mosby/Elsevier, 2002
- **NAKAMURA, R.M. et al.** Cancer Diagnostics. Humana Press, 2004
- **CHANG et al.** *CA Cancer J Clin,* 2008, vol. 58, 54-59
- TNM Classification of Malignant Tumours. UICC, 2002
- American Joint Committee on Cancer (AJCC). TNM Classification of Malignant Tumours. UICC, 2002
- The Science and Practice of Pharmacy. Philadelphia College of Pharmacy and Science, 2000
- Remington's Pharmaceutical Sciences. Mack Pub. Co,
- **ERDOGAN et al.** *J. Biomed. Mat. Res. B Appl. Biomat.,* 2008, vol. 85, 272
- **SPOTNITZ et al.** *Transfusion,* 2012, vol. 52, 2243
- **SCHUETZ et al.** *Ann. Thorac. Surg.,* 2004, vol. 78, 569
- **VAN DE WALLE et al.** *Acta. Biomater.,* 2012, vol. 8, 4080
- The Merck Manual. Merck and Co, 1992
- Goodman and Gilman's The Pharmacological Basis of Therapeutics. Pergamon Press, Inc, 2001
- Avery's Drug Treatment: Principles and Practice of Clinical Pharmacology and Therapeutics. ADIS Press, LTD., Williams and Wilkins, 1987
- **EBADI.** Pharmacology. Little, Brown and Co, 1985
- Remington's Pharmaceutical Sciences. Mack Publishing Co, 1990
- **KATZUNG.** Basic and Clinical Pharmacology. Appleton and Lange, 1992
- **P. J. KUZMA et al.** *Regional Anesthesia,* 1997, vol. 22 (6), 543-551
- **HUMAR et al.** Atlas of Organ Transplantation. Springer, 2006
- **KUO et al.** Comprehensive Atlas of Transplantation. Lippincott, Williams & Wilkins, 2004
- **GRUESSNER et al.** Living Donor Organ Transplantation. McGraw-Hill Professional, 2007
- **ANTIN et al.** Manual of Stem Cell and Bone Marrow Transplantation. Cambridge University Press, 2009
- Hematopoietic Stem Cell Transplantation: A Handbook for Clinicians. American Association of Blood Banks, 2009
- **SABISTON.** Textbook of Surgery. Saunders & Co, 2012
- **MULHOLLAND.** Greenfield's Surgery. Lippincott, Williams & Wilkins, 2010
- Schwartz's Principles of Surgery. McGraw-Hill, 2009
- **LAWRENCE.** Essentials of General Surgery. Lippincott, Williams & Wilkins, 2012
- Current Protocols in Molecular Biology or Current Protocols in Immunology. John Wiley & Sons, 2009
- **AUSUBEL et al.** Short Protocols in Molecular Biology. Wiley & Sons, 1995
- **SAMBROOK ; RUSSELL.** Molecular Cloning: A Laboratory Manual. 2001
- **MANIATIS et al.** Molecular Cloning: A Laboratory Manual. 1982
- DNA Cloning: A Practical Approach. vol. I, II
- Oligonucleotide Synthesis. 1984
- Nucleic Acid Hybridization. 1985
- Transcription and Translation. 1984
- Animal Cell Culture. 1986
- **PERBAL.** *A Practical Guide to Molecular Cloning,* 1984
- **PETERSEN, T. E. ; MARTZEN, M. R. ; ICHINOSE, A. ; DAVIE, E. W.** Characterization of the gene for human plasminogen, a key proenzyme in the fibrinolytic system. *J. Biol. Chem.,* 1990, vol. 265, 6104-6111
- **ZHANG, L. ; SEIFFERT, D. ; FOWLER, B. J. ; JENKINS, G. R. ; THINNES, T. C. ; LOSKUTOFF, D. J. ; PARMER, R. J. ; MILES, L. A.** Plasminogen has a broad extrahepatic distributions. *Thromb. Haemostasis,* 2002, vol. 87, 493-501

- **BOHMFALK, J. F. ; FULLER, G. M.** Plasminogen is synthesized by primary cultures of rat hepatocytes. *Science,* 1980, vol. 209, 408-410
- **SAITO, H. ; HAMILTON, S. M. ; TAVILL, A. S. ; LOUIS, L. ; RATNOFF, O. D.** Production and release of plasminogen by isolated perfused rat-liver. *Proc. Natl. Acad. Sci. U. S. A.,* 1980, vol. 77, 6837-6840
- **MILES, L. A. ; CASTELLINO, F. J. ; GONG, Y.** Critical role for conversion of glu-plasminogen to Lys-plasminogen for optimal stimulation of plasminogen activation on cell surfaces. *Trends Cardiovasc. Med.,* 2003, vol. 13, 21-30
- **COCKELL, C. S. ; MARSHALL, J. M. ; DAWSON, K. M. ; CEDERHOLM-WILLIAMS, S. A. ; PONTING, C. P.** Evidence that the conformation of unliganded human plasminogen is maintained via an intramolecular interaction between the lysine-binding site of kringle 5 and the N-terminal peptide. *Biochem. J.,* 1998, vol. 333, 99-105
- **LAW, R. H. P. ; CARADOC-DAVIES, T. ; COWIESON, N. ; HORVATH, A. J. ; QUEK, A. J. ; ENCARNACAO, J. A. ; STEER, D. ; COWAN, A. ; ZHANG, Q. W. ; LU, B. G. C.** The x-ray crystal structure of full-length human plasminogen. *Cell Rep.,* 2012, vol. 1, 185-190
- **VIOLAND, B. N. ; CASTELLINO, F. J.** Mechanism of urokinase-catalyzed activation of human plasminogen. *J. Biol. Chem.,* 1976, vol. 251, 3906-3912
- **CASTELLINO, F. J. ; PLOPLIS, V. A.** Structure and function of the plasminogen/plasmin system. *Thromb. Haemostasis,* 2005, vol. 93, 647-654
- **ELLIS, V. ; BEHRENDT, N. ; DANO, K.** Plasminogen activation by receptor-bound urokinase - a kinetic-study with both cell-associated and isolated receptor. *J. Biol. Chem.,* 1991, vol. 266, 12752-12758
- **URANO, T. ; DESERRANO, V. S. ; GAFFNEY, P. J. ; CASTELLINO, F. J.** Effectors of the activation of human [glu]plasminogen by human-tissue plasminogen-activator. *Biochemistry,* 1988, vol. 27, 6522-6528
- **VIOLAND, B. N. ; SODETZ, J. M. ; CASTELLINO, F. J.** Effect of ε-amino caproic acid on gross conformation of plasminogen and plasmin. *Arch. Biochem. Biophys.,* 1975, vol. 170, 300-305
- **MARKUS, G. ; EVERS, J. L. ; HOBIKA, G. H.** Comparison of some properties of native (glu) and modified (lys) human plasminogen. *J. Biol. Chem.,* 1978, vol. 253, 733-739
- **CLAEYS, H. ; VERMYLEN, J.** Physicochemical and proenzyme properties of NH2-terminal glutamic-acid and NH2-terminal lysine human plasminogen - influence of 6-aminohexanoic acid. *Biochim. Biophys. Acta,* 1974, vol. 342, 351-359
- **FREDENBURGH, J. C. ; NESHEIM, M. E.** Lys-plasminogen is a significant intermediate in the activation of glu-plasminogen during fibrinolysis invitro. *J. Biol. Chem.,* 1992, vol. 267, 26150-26156
- **KRUITHOF, E. K. O. ; TRANTHANG, C. ; RANSIJN, A. ; BACHMANN, F.** Demonstration of a fast-acting inhibitor of plasminogen activators in human-plasma. *Blood,* 1984, vol. 64, 907-913
- **ÅSTEDT, B. ; LECANDER, I. ; NY, T.** The placental type plasminogen activator inhibitor, PAI 2. *Fibrinolysis,* 1987, vol. 1, 203-208
- **SPRENGERS, E. D. ; KLUFT, C.** Plasminogen-activator inhibitors. *Blood,* 1987, vol. 69, 381-387
- **COLLEN, D. ; WIMAN, B.** Fast-acting plasmin inhibitor in human-plasma. *Blood,* 1978, vol. 51, 563-569
- **HALL, S. W. ; HUMPHRIES, J. E. ; GONIAS, S. L.** Inhibition of cell-surface receptor-bound plasmin by alpha-2-antiplasmin and alpha-2-macroglobulin. *J. Biol. Chem.,* 1991, vol. 266, 12329-12336
- **COLLEN, D.** Ham-Wasserman lecture: role of the plasminogen system in fibrin-homeostasis and tissue remodeling. *Hematology Am. Soc. Hematol. Educ. Program,* 2001, 1-9
- **CAMIOLO, S. M. ; THORSEN, S. ; ASTRUP, T.** Fibrinogenolysis and fibrinolysis with tissue plasminogen activator, urokinase, streptokinase-activated human globulin, and plasmin. *P. Soc. Exp. Biol. Med.,* 1971, vol. 138, 277-280
- **PENNICA, D. ; HOLMES, W. E. ; KOHR, W. J. ; HARKINS, R. N. ; VEHAR, G. A. ; WARD, C. A. ; BENNETT, W. F. ; YELVERTON, E. ; SEEBURG, P. H. ; HEYNEKER, H. L.** Cloning and expression of human tissue-type plasminogen-activator cDNA in escherichia-coli. *Nature,* 1983, vol. 301, 214-221
- **BANYAI, L. ; VARADI, A. ; PATTHY, L.** Common evolutionary origin of the fibrin-binding structures of fibronectin and tissue-type plasminogen-activator. *Febs. Lett.,* 1983, vol. 163, 37-41
- **DEVRIES, C. ; VEERMAN, H. ; PANNEKOEK, H.** Identification of the domains of tissue-type plasminogen-activator involved in the augmented binding to fibrin after limited digestion with plasmin. *J. Biol. Chem.,* 1989, vol. 264, 12604-12610
- **VANZONNEVELD, A. J. ; VEERMAN, H. ; PANNEKOEK, H.** On the interaction of the finger and the kringle-2 domain of tissue-type plasminogen-activator with fibrin - inhibition of kringle-2 binding to fibrin by epsilon-amino caproic acid. *J. Biol. Chem.,* 1986, vol. 261, 14214-14218
- **DEVRIES, C. ; VEERMAN, H. ; KOORNNEEF, E. ; PANNEKOEK, H.** Tissue-type plasminogen-activator and its substrate glu-plasminogen share common binding-sites in limited plasmin-digested fibrin. *J. Biol. Chem.,* 1990, vol. 265, 13547-13552

- Fibrinogen structure and function. **WEISEL, J. W. ; DEMPFLE, C. H.** Hemostasis and Thrombosis. Lippincott Williams & Wilkins, 2012, 254-271

- **SATKURUNATH, G. ; ROYSTON, D.** Hemostatic drugs in trauma and orthopaedic practice. *International Trauma Care,* 2008, vol. 18, 24-28

- **HARTMANN, M. ; SUCKER, C. ; BOEHM, O. ; KOCH, A. ; LOER, S. ; ZACHAROWSKI, K.** Effects of cardiac surgery on hemostasis. *Transfus. Med. Rev.,* 2006, vol. 20, 230-241

- **DHIR, A.** Antifibrinolytics in cardiac surgery. *Ann. Card. Anaesth.,* 2013, vol. 16, 117-125

- **SAUAIA, A. ; MOORE, F. A. ; MOORE, E. E. ; MOSER, K. S. ; BRENNAN, R. ; READ, R. A. ; PONS, P. T.** Epidemiology of trauma deaths - a reassessment. *J. Trauma,* 1995, vol. 38, 185-193

- **LEVY, J. H.** Aprotinin is useful as a hemostatic agent in cardiopulmonary surgery: yes. *J. Thromb. Haemostasis,* 2006, vol. 4, 1875-1878

- **LEVI, M. ; CROMHEECKE, M. E. ; DE JONGE, E. ; PRINS, M. H. ; DE MOL, B. J. M. ; BRIET, E. ; BULLER, H. R.** Pharmacological strategies to decrease excessive blood loss in cardiac surgery: a meta-analysis of clinically relevant endpoints. *Lancet,* 1999, vol. 354, 1940-1947

- **ROYSTON, D. ; VAN HAAFTEN, N. ; DE VOOGHT, P.** Aprotinin; friend or foe? A review of recent medical literature. *Eur. J. Anaesth.,* 2007, vol. 24, 6-14

- **MANGANO, D. T. ; TUDOR, I. C. ; DIETZEL, C. ; IS, M. S. P. ; FDN, I. R. E.** The risk associated with aprotinin in cardiac surgery. *New Engl. J. Med.,* 2006, vol. 354, 353-365

- **IMMER, F. F. ; JENT, P. ; ENGLBERGER, L. ; STALDER, M. ; GYGAX, E. ; CARREL, T. P. ; TEVAEARAI, H. T.** Aprotinin in cardiac surgery: A different point of view. *Heart Surg. Forum,* 2008, vol. 11, E9-E12

- **BEIERLEIN, W. ; SCHEULE, A. M. ; DIETRICH, W. ; ZIEMER, G.** Forty years of clinical aprotinin use: A review of 124 hypersensitivity reactions. *Ann. Thorac. Surg.,* 2005, vol. 79, 741-748

- **MARTIN, K. ; KNORR, J. ; BREUER, T. ; GERTLER, R. ; MACGUILL, M. ; LANGE, R. ; TASSANI, P. ; WIESNER, G.** Seizures after open heart surgery: comparison of epsilon-aminocaproic acid and tranexamic acid. *J. Cardiothor. Vasc. An.,* 2011, vol. 25, 20-25

- **RABINOVICI, R. ; HEYMAN, A. ; KLUGER, Y. ; SHINAR, E.** Convulsions induced by aminocaproic acid infusion. *Dicp. Ann. Pharmac.,* 1989, vol. 23, 780-781

- **SALONEN, E. M. ; ZITTING, A. ; VAHERI, A.** Laminin interacts with plasminogen and its tissue-type activator. *FEBS Lett.,* 1984, vol. 172, 29-32

- **SILVERSTEIN, R. L. ; LEUNG, L. L. K. ; HARPEL, P. C. ; NACHMAN, R. L.** Complex-formation of platelet thrombospondin with plasminogen - modulation of activation by tissue activator. *J. Clin. Invest.,* 1984, vol. 74, 1625-1633

- **SALONEN, E. M. ; SAKSELA, O. ; VARTIO, T. ; VAHERI, A. ; NIELSEN, L. S. ; ZEUTHEN, J.** Plasminogen and tissue-type plasminogen-activator bind to immobilized fibronectin. *J. Biol. Chem.,* 1985, vol. 260, 2302-2307

- **LIJNEN, H. R. ; VAN HOEF, B. ; LUPU, F. ; MOONS, L. ; CARMELIET, P. ; COLLEN, D.** Function of the plasminogen/plasmin and matrix metalloproteinase systems after vascular injury in mice with targeted inactivation of fibrinolytic system genes. *Arterioscl. Throm. Vas.,* 1998, vol. 18, 1035-1045

- **BONNEFOY, A. ; LEGRAND, C.** Proteolysis of subendothelial adhesive glycoproteins (fibronectin, thrombospondin, and von Willebrand factor) by plasmin, leukocyte cathepsin G, and elastase. *Thromb. Res.,* 2000, vol. 98, 323-332

- **LIJNEN, H. R.** Molecular interactions between the plasminogen/plasmin and matrix metalloproteinase systems. *Fibrinolysis Proteol.,* 2000, vol. 14, 175-181

- **PLOW, E. F. ; HERREN, T. ; REDLITZ, A. ; MILES, L. A. ; HOOVERPLOW, J. L.** The cell biology of the plasminogen system. *FASEB J.,* 1995, vol. 9, 939-945

- **HE, C. S. ; WILHELM, S. M. ; PENTLAND, A. P. ; MARMER, B. L. ; GRANT, G. A. ; EISEN, A. Z. ; GOLDBERG, G. I.** Tissue cooperation in a proteolytic cascade activating human interstitial collagenase. *Proc. Natl. Acad. Sci. U. S. A.,* 1989, vol. 86, 2632-2636

- **OKADA, Y. ; GONOJI, Y. ; NAKA, K. ; TOMITA, K. ; NAKANISHI, I. ; IWATA, K. ; YAMASHITA, K. ; HAYAKAWA, T.** Matrix metalloproteinase-9 (92-kda gelatinase type-iv collagenase) from ht-1080 human fibrosarcoma cells - purification and activation of the precursor and enzymatic-properties. *J. Biol. Chem.,* 1992, vol. 267, 21712-21719

- **HOLLIDAY, L. S. ; WELGUS, H. G. ; FLISZAR, C. J. ; VEITH, G. M. ; JEFFREY, J. J. ; GLUCK, S. L.** Initiation of osteoclast bone resorption by interstitial collagenase. *J. Biol. Chem.,* 1997, vol. 272, 22053-22058

- **GONG, Y. Q. ; HART, E. ; SHCHURIN, A. ; HOOVER-PLOW, J.** Inflammatory macrophage migration requires MMP-9 activation by plasminogen in mice. *J. Clin. Invest.,* 2008, vol. 118, 3012-3024

- **LEE, S. ; JILANI, S. M. ; NIKOLOVA, G. V. ; CARPIZO, D. ; IRUELA-ARISPE, M. L.** Processing of VEGF-A by matrix metalloproteinases regulates bioavailability and vascular patterning in tumors. *J. Cell. Biol.,* 2005, vol. 169, 681-691

- **MCCOLL, B. K. ; BALDWIN, M. E. ; ROUFAIL, S. ; FREEMAN, C. ; MORITZ, R. L. ; SIMPSON, R. J. ; ALITALO, K. ; STACKER, S. A. ; ACHEN, M. G.** Plasmin activates the lymphangiogenic growth factors VEGF-C and VEGF-D. *J. Exp. Med.,* 2003, vol. 198, 863-868
- **FALCONE, D. J. ; MCCAFFREY, T. A. ; HAIMOV-ITZFRIEDMAN, A. ; VERGILIO, J. A. ; NICHOLSON, A. C.** Macrophage and foam cell release of matrix-bound growth-factors - role of plasminogen activation. *J. Biol. Chem.,* 1993, vol. 268, 11951-11958
- **SYROVETS, T. ; LUNOV, O. ; SIMMET, T.** Plasmin as a proinflammatory cell activator. *J. Leukocyte Biol.,* 2012, vol. 92, 509-519
- **MILES, L. A. ; DAHLBERG, C. M. ; PLOW, E. F.** The cell-binding domains of plasminogen and their function in plasma. *J. Biol. Chem.,* 1988, vol. 263, 11928-11934
- **MILES, L. A. ; DAHLBERG, C. M. ; PLESCIA, J. ; FELEZ, J. ; KATO, K. ; PLOW, E. F.** Role of cell-surface lysines in plasminogen binding to cells - identification of alpha-enolase as a candidate plasminogen receptor. *Biochemistry,* 1991, vol. 30, 1682-1691
- **ANDRONICOS, N. M. ; CHEN, E. I. ; BAIK, N. ; BAI, H. ; PARMER, C. M. ; KIOSSES, W. B. ; KAMPS, M. P. ; YATES, J. R. ; PARMER, R. J. ; MILES, L. A.** Proteomics-based discovery of a novel, structurally unique, and developmentally regulated plasminogen receptor, Plg-R-KT, a major regulator of cell surface plasminogen activation. *Blood,* 2010, vol. 115, 1319-1330
- **LIOTTA, L. A. ; GOLDFARB, R. H. ; BRUNDAGE, R. ; SIEGAL, G. P. ; TERRANOVA, V. ; GARBISA, S.** Effect of plasminogen-activator (urokinase), plasmin, and thrombin on glycoprotein and collagenous components of basement-membrane. *Cancer Res.,* 1981, vol. 41, 4629-4636
- **FESTUCCIA, C. ; DOLO, V. ; GUERRA, F. ; VIOLINI, S. ; MUZI, P. ; PAVAN, A. ; BOLOGNA, M.** Plasminogen activator system modulates invasive capacity and proliferation in prostatic tumor cells. *Clin. Exp. Metastas.,* 1998, vol. 16, 513-528
- **SPRECHER, C. A. ; KISIEL, W. ; MATHEWES, S. ; FOSTER, D. C.** Molecular-cloning, expression, and partial characterization of a second human tissue-factor-pathway inhibitor. *Proc. Natl. Acad. Sci. U. S. A.,* 1994, vol. 91, 3353-3357
- **MIYAGI, Y. ; KOSHIKAWA, N. ; YASUMITSU, H. ; MIYAGI, E. ; HIRAHARA, F. ; AOKI, I. ; MISUGI, K. ; UMEDA, M. ; MIYAZAKI, K.** cDNA cloning and messenger-rna expression of a serine proteinase-inhibitor secreted by cancer-cells - identification as placental protein-5 and tissue factor pathway inhibitor-2. *J. Biochem-Tokyo,* 1994, vol. 116, 939-942
- **RAO, C. N. ; REDDY, P. ; LIU, Y. Y. ; OTOOLE, E. ; REEDER, D. ; FOSTER, D. C. ; KISIEL, W. ; WOODLEY, D. T.** Extracellular matrix-associated serine protease inhibitors (M(r) 33,000, 31,000, and 27,000) are single-gene products with differential glycosylation: cDNA cloning of the 33-kDa inhibitor reveals its identity to tissue factor pathway inhibitor-2. *Arch. Biochem. Biophys.,* 1996, vol. 335, 82-92
- **PETERSEN, L. C. ; SPRECHER, C. A. ; FOSTER, D. C. ; BLUMBERG, H. ; HAMAMOTO, T. ; KISIEL, W.** Inhibitory properties of a novel human Kunitz-type protease inhibitor homologous to tissue factor pathway inhibitor. *Biochemistry,* 1996, vol. 35, 266-272
- **SCHECHTE.I ; BERGER, A.** On size of active site in proteases .I. papain. *Biochem. Biophy. Res. Com.,* 1967, vol. 27, 157-162
- **BAJAJ, M. S. ; OGUELI, G. I. ; KUMAR, Y. ; VADIVEL, K. ; LAWSON, G. ; SHANKER, S. ; SCHMIDT, A. E. ; BAJAJ, S. P.** Engineering kunitz domain 1 (KD1) of human tissue factor pathway inhibitor-2 to selectively inhibit fibrinolysis properties of KD1-L17R variant. *J. Biol. Chem.,* 2011, vol. 286, 4329-4340
- **SAMBROOK, J. ; RUSSEL, D. W.** Molecular cloning: a laboratory manual. Cold Spring Harbor Laboratory Press, 2001
- **CHAND, H. S. ; SCHMIDT, A. E. ; BAJAJ, S. P. ; KISIEL, W.** Structure-function analysis of the reactive site in the first Kunitz-type domain of human tissue factor pathway inhibitor-2. *J. Biol. Chem.,* 2004, vol. 279, 17500-17507
- **SCHMIDT, A. E. ; CHAND, H. S. ; CASCIO, D. ; KISIEL, W. ; BAJAJ, S. P.** Crystal structure of Kunitz domain 1 (KD1) of tissue factor pathway inhibitor-2 in complex with trypsin - implications for KD1 specificity of inhibition. *J. Biol. Chem,* 2005, vol. 280, 27832-27838
- **STONE, M. J. ; RUF, W. ; MILES, D. J. ; EDGINGTON, T. S. ; WRIGHT, P. E.** Recombinant soluble human tissue factor secreted by saccharomyces-cerevisiae and refolded from escherichia-coli inclusion-bodies - glycosylation of mutants, activity and physical characterization. *Biochem. J.,* 1995, vol. 310, 605-614
- **LAEMMLI, U. K.** Cleavage of structural proteins during assembly of head of bacteriophage-t4. *Nature,* 1970, vol. 227, 680-685
- **BIETH, J. G.** In vivo significance of kinetic constants of protein proteinase-inhibitors. *Biochem. Med. Metab. B,* 1984, vol. 32, 387-397
- **MORRISON, J. F. ; WALSH, C. T.** The behavior and significance of slow-binding enzyme-inhibitors. *Adv. Enzymol. Ramb.,* 1988, vol. 61, 201-301

- **SPERZEL, M. ; HUETTER, J.** Evaluation of aprotinin and tranexamic acid in different in vitro and in vivo models of fibrinolysis, coagulation and thrombus formation. *J. Thromb. Haemostasis,* 2007, vol. 5, 2113-2118
- **HENRY, D. A. ; CARLESS, P. A. ; MOXEY, A. J. ; O'CONNELL, D. ; STOKES, B. J. ; MCCLELLAND, B. ; LAUPACIS, A. ; FERGUSSON, D.** Anti-fibrinolytic use for minimising perioperative allogeneic blood transfusion. *Cochrane Db. Syst. Rev.,* 2007
- **MEDYNSKI, D. ; TUAN, M. ; LIU, W. ; WU, S. L. ; LIN, X. L.** Refolding, purification, and activation of miniplasminogen and microplasminogen isolated from E-coli inclusion bodies. *Protein. Expres. Purif.,* 2007, vol. 52, 395-402
- **HUDIG, D. ; BAJAJ, S. P.** Tissue factor-like activity of the human monocytic tumor-cell line-u937. *Thromb. Res.,* 1982, vol. 27, 321-332
- **FELEZ, J. ; MILES, L. A. ; PLESCIA, J. ; PLOW, E. F.** Regulation of plasminogen receptor expression on human monocytes and monocytoid cell-lines. *J. Cell. Biol.,* 1990, vol. 111, 1673-1683
- **HALFMAN, C. J.** Concentrations of binding protein and labeled analyte that are appropriate for measuring at any analyte concentration range in radioimmunoassays. *Methods Enzymol.,* 1981, vol. 74, 481-497
- **CHENG, Y. ; PRUSOFF, W. H.** Relationship between inhibition constant (KI) and concentration of inhibitor which causes 50 per cent inhibition (I50) of an enzymatic-reaction. *Biochem. Pharmacol.,* 1973, vol. 22, 3099-3108
- **CRAIG, D. A.** The Cheng-Prusoff relationship - something lost in the translation. *Trends Pharmacol. Sci.,* 1993, vol. 14, 89-91
- **PLOW, E. F. ; DOEUVRE, L. ; DAS, R.** So many plasminogen receptors: why?. *J. Biomed. Biotechnol.,* 2012, vol. 2012, 141806
- **PARRY, M. A. A. ; FERNANDEZ-CATALAN, C. ; BERGNER, A. ; HUBER, R. ; HOPFNER, K. P. ; SCHLOTT, B. ; GUHRS, K. H. ; BODE, W.** The ternary microplasmin-staphylokinase-microplasmin complex is a proteinase-cofactor-substrate complex in action. *Nat. Struct. Biol.,* 1998, vol. 5, 917-923
- **MATHEWS, I. I. ; VANDERHOFFHANAVER, P. ; CASTELLINO, F. J. ; TULINSKY, A.** Crystal structures of the recombinant kringle 1 domain of human plasminogen in complexes with the ligands epsilon-aminocaproic acid and trans-4-(aminomethyl)cyclohexane-1-carboxylic acid. *Biochemistry,* 1996, vol. 35, 2567-2576
- **BAJAJ, M. S. ; BIRKTOFT, J. J. ; STEER, S. A. ; BAJAJ, S. P.** Structure and biology of tissue factor pathway inhibitor. *Thromb. Haemostasis,* 2001, vol. 86, 959-972
- Comparative protein structure modeling with MODELLER. **ESWAR, N. ; MARTI-RENOM, M.A. ; WEBB, B. ; MADHUSUDHAN, M.S. ; ERAMIAN, D. ; SHEN, M. ; PIEPER, U. ; SALI, A.** Current Protocols in Bioinformatics. John Wiley & Sons, Inc, 2006
- **BROOKS, B. R. ; BRUCCOLERI, R. E. ; OLAFSON, B. D. ; STATES, D. J. ; SWAMINATHAN, S. ; KARPLUS, M.** CHARMM - a program for macromolecular energy, minimization, and dynamics calculations. *J. Comp. Chem.,* 1983, vol. 4, 187-217
- **BODE, W. ; TURK, D. ; KARSHIKOV, A.** The refined 1.9-Å x-ray crystal-structure of d-phe-pro-arg chloromethylketone-inhibited human α-thrombin - structure-analysis, overall structure, electrostatic properties, detailed active-site geometry, and structure-function-relationships. *Protein Sci.,* 1992, vol. 1, 426-471
- **CHANG, J. Y.** Thrombin specificity - requirement for apolar amino-acids adjacent to the thrombin cleavage site of polypeptide substrate. *Eur. J. Biochem.,* 1985, vol. 151, 217-224
- **MARKUS, G. ; DEPASQUALE, J. L. ; WISSLER, F. C.** Quantitative-determination of binding of epsilon-aminocaproic acid to native plasminogen. *J. Biol. Chem.,* 1978, vol. 253, 727-732
- **GROH, J. ; WELTE, M. ; AZAD, S. C. ; KRATZER, M. A. A.** Monitoring of hemostasis during liver surgery. *Infusionstherapie,* 1993, vol. 20, 173-179
- **SCHLAG, M.G. ; HOPF, R. ; REDL, H.** Convulsive seizures following subdural application of fibrin sealant containing tranexamic acid in a rat model. *Neurosurgery,* 2000, vol. 47, 1463-1467
- **LECKER, I. ; WANG, D. S. ; ROMASCHIN, A. D. ; PETERSON, M. ; MAZER, C. D. ; ORSER, B. A.** Tranexamic acid concentrations associated with human seizures inhibit glycine receptors. *J. Clin. Invest.,* 2012, vol. 122, 4654-4666
- **MAKHIJA, N. ; SARUPRIA, A. ; CHOUDHARY, S.K. ; DAS, S. ; LAKSHMY, R. ; KIRAN, U.** Comparison of Epsilon Aminocaproic Acid and Tranexamic Acid in Thoracic Aortic Surgery: Clinical Efficacy and Safety. *J. Cardiothorac. Vasc. Anesth.,* 2013, vol. 27, 1201-1207
- **MANJI, R.A. ; GROCOTT, H.P. ; LEAKE, J. ; ARIANO, R.E. ; MANJI, J.S. ; MENKIS, A.H. ; JACOBSOHN, E.** Seizures following cardiac surgery: the impact of tranexamic acid and other risk factors. *Can. J. Anaesth.,* 2012, vol. 59, 6-13
- **KEMPAIAH, P. ; DANIELSON, L. A. ; BARRY, M. ; KISIEL, W.** Comparative effects of aprotinin and human recombinant R24K KD1 on temporal renal function in long-evans rats. *J. Pharmacol. Exp. Ther.,* 2009, vol. 331, 940-945